# EUROPEAN PATENT APPLICATION

(11) **EP 4 633 333 A1**
(43) Date of publication of application: **15.10.2025**
(21) Application number: 23900422.9
(22) Date of filing: 16.11.2023
(51) Int. Cl.: H10K 30/60, C07D 333/54, C07D 495/04, H10K 30/30, H10K 39/32, H10K 85/60

(54) **PHOTOELECTRIC CONVERSION ELEMENT, IMAGING DEVICE, PHOTOSENSOR, METHOD FOR PRODUCING IMAGING DEVICE, AND COMPOUND**

(30) Priority: 09.12.2022 JP 2022196744; 23.10.2023 JP 2023181780
(71) Applicant: FUJIFILM Corporation, Tokyo 106-8620 (JP)
(72) Inventor: SASAKI, Kouitsu, Ashigarakami-gun, Kanagawa 258-8577 (JP); SUZUKI, Yuko, Ashigarakami-gun, Kanagawa 258-8577 (JP); YAMAMOTO, Yosuke, Ashigarakami-gun, Kanagawa 258-8577 (JP); SUGIURA, Hiroki, Ashigarakami-gun, Kanagawa 258-8577 (JP); IZUMI, Saika, Ashigarakami-gun, Kanagawa 258-8577 (JP)
(74) Representative: HGF
(86) International application number: PCT/JP2023/041261
(87) International publication number: WO 2024/122301

(57) **Abstract**

The present invention provides a photoelectric conversion element having excellent quantum efficiency in a case of receiving blue light. In addition, the present invention provides an imaging element, an optical sensor, and a compound, which are related to the photoelectric conversion element. The photoelectric conversion element of the present invention includes, in the following order, a conductive film, a photoelectric conversion film, and a transparent conductive film, in which the photoelectric conversion film contains a compound represented by Formula (1).

## Description

### BACKGROUND OF THE INVENTION

### 1. Field of the Invention

The present invention relates to a photoelectric conversion element, an imaging element, an optical sensor, a manufacturing method of an imaging element, and a compound.

### 2. Description of the Related Art

In recent years, development of an element (for example, an imaging element) having a photoelectric conversion film has progressed.

For example, WO2014/026224A discloses a photoelectric conversion element containing a specific compound as an electron accepting material.

### SUMMARY OF THE INVENTION

In recent years, further improvements are also required for various characteristics required for a photoelectric conversion element used in an imaging element and an optical sensor, along with demands for improving performance of the imaging element, the optical sensor, and the like.

For example, a high level of quantum efficiency in a case where the photoelectric conversion element receives blue light (particularly, at a wavelength of 460 nm) is required. Here, the above-described blue light refers to light in a wavelength range of 400 to 500 nm.

As a result of studying the photoelectric conversion element containing the compound, disclosed in WO2014/026224A, the present inventors have found that there is room for further improvement in quantum efficiency in a case of receiving the above-described blue light.

An object of the present invention is to provide a photoelectric conversion element having excellent quantum efficiency in a case of receiving blue light.

Another object of the present invention is to provide an imaging element, an optical sensor, a manufacturing method of an imaging element, and a compound, which are related to the above-described photoelectric conversion element.

The present inventors have completed the present invention as a result of intensive studies to solve the above-described problems. That is, the present inventors have found that the above-described objects can be achieved by the following configuration.
[1] A photoelectric conversion element comprising, in the following order:
   a conductive film;
   a photoelectric conversion film; and
   a transparent conductive film,
   in which the photoelectric conversion film contains a compound represented by Formula (1) described later.
[2] The photoelectric conversion element according to [1],
   in which R^{Y1} represents a hydrogen atom, an alkyl group which may have a substituent, an alkenyl group which may have a substituent, an alkynyl group which may have a substituent, an aryl group which may have a substituent, a heteroaryl group which may have a substituent, an alkoxy group which may have a substituent, an aryloxy group which may have a substituent, an amino group which may have a substituent, a silyl group which may have a substituent, a cyano group, or a halogen atom.
[3] The photoelectric conversion element according to [1] or [2],
   in which D represents a group represented by any of Formula (2-10), Formula (2-11), Formula (2-12), Formula (2-13), Formula (3-10), Formula (3-11), Formula (3-12), Formula (3-13), Formula (4-10), Formula (4-11), Formula (5-10), or Formula (5-11), which will be described later.
[4] The photoelectric conversion element according to any one of [1] to [3],
   in which Rs represents a linear alkyl group having 1 to 5 carbon atoms, which may have a substituent, a branched alkyl group having 3 to 7 carbon atoms, which may have a substituent, a cyclic alkyl group having 3 to 6 carbon atoms, which may have a substituent, an alkenyl group having 2 to 5 carbon atoms, which may have a substituent, an alkynyl group having 2 to 5 carbon atoms, which may have a substituent, an aryl group having 6 to 14 carbon atoms, which may have a substituent, a heteroaryl group having 2 to 14 carbon atoms, which may have a substituent, an alkoxy group having 1 to 5 carbon atoms, which may have a substituent, an aryloxy group having 6 to 14 carbon atoms, which may have a substituent, an amino group which may have a substituent, a silyl group which may have a substituent, a cyano group, or a halogen atom.
[5] The photoelectric conversion element according to [3],
   in which D represents the group represented by any of Formula (2-10), Formula (2-11), Formula (2-12), Formula (2-13), Formula (3-10), Formula (3-11), Formula (3-12), Formula (3-13), Formula (5-10), or Formula (5-11).
[6] The photoelectric conversion element according to any one of [1] to [5],
   in which W¹ is an oxygen atom or a sulfur atom.
[7] The photoelectric conversion element according to any one of [1] to [6],
   in which the group represented by Formula (A-1) is a group represented by Formula (A-2) described later.
[8] The photoelectric conversion element according to [7],
   in which the group represented by Formula (A-2) is a group represented by Formula (C-1) described later or a group represented by Formula (C-2) described later.
[9] The photoelectric conversion element according to [8],
   in which both X^{c1} and X^{c2} are oxygen atoms and both x^{c3} and x^{c4} are oxygen atoms.
[10] The photoelectric conversion element according to any one of [1] to [9],
   in which Rs represents a linear alkyl group having 1 to 5 carbon atoms, which may have a substituent, a branched alkyl group having 3 to 7 carbon atoms, which may have a substituent, a cyclic alkyl group having 3 to 6 carbon atoms, which may have a substituent, an aryl group having 6 to 14 carbon atoms, which may have a substituent, a heteroaryl group having 2 to 14 carbon atoms, which may have a substituent, an alkoxy group having 1 to 5 carbon atoms, which may have a substituent, an aryloxy group having 6 to 14 carbon atoms, which may have a substituent, a silyl group which may have a substituent, or a halogen atom.
[11] The photoelectric conversion element according to [3],
   in which D represents the group represented by Formula (4-10) or the group represented by Formula (4-11).
[12] The photoelectric conversion element according to [11],
   in which Rs represents a linear alkyl group having 1 to 5 carbon atoms, which may have a substituent, a branched alkyl group having 3 to 7 carbon atoms, which may have a substituent, a cyclic alkyl group having 3 to 6 carbon atoms, which may have a substituent, or a group represented by Formula (S1) described later.
[13] The photoelectric conversion element according to any one of [1] to [12],
   in which the photoelectric conversion film further contains an n-type organic semiconductor, and
   the photoelectric conversion film has a bulk hetero structure formed in a state in which the compound represented by Formula (1) and the n-type organic semiconductor are mixed with each other.
[14] The photoelectric conversion element according to [13],
   in which the n-type organic semiconductor includes fullerenes selected from the group consisting of a fullerene and derivatives of the fullerene.
[15] The photoelectric conversion element according to any one of [1] to [14],
   in which the photoelectric conversion film further contains a p-type organic semiconductor.
[16] The photoelectric conversion element according to any one of [1] to [15],
   in which the photoelectric conversion film further contains a coloring agent.
[17] The photoelectric conversion element according to any one of [1] to [16], further comprising:
   one or more interlayers between the conductive film and the transparent conductive film, in addition to the photoelectric conversion film.
[18] An imaging element comprising:
   the photoelectric conversion element according to any one of [1] to [17].
[19] An optical sensor comprising:
   the photoelectric conversion element according to any one of [1] to [17].
[20] A manufacturing method of an imaging element, comprising:
   a step of manufacturing the photoelectric conversion element according to any one of [1] to [17].
[21] A compound represented by Formula (1) described later.
[22] The compound according to [21],
   in which R^{Y1} represents a hydrogen atom, an alkyl group which may have a substituent, an alkenyl group which may have a substituent, an alkynyl group which may have a substituent, an aryl group which may have a substituent, a heteroaryl group which may have a substituent, an alkoxy group which may have a substituent, an aryloxy group which may have a substituent, an amino group which may have a substituent, a silyl group which may have a substituent, a cyano group, or a halogen atom.
[23] The compound according to [21] or [22],
   in which D represents a group represented by any of Formula (2-10), Formula (2-11), Formula (2-12), Formula (2-13), Formula (3-10), Formula (3-11), Formula (3-12), Formula (3-13), Formula (4-10), Formula (4-11), Formula (5-10), or Formula (5-11), which will be described later.
[24] The compound according to any one of [21] to [23],
   in which Rs represents a linear alkyl group having 1 to 5 carbon atoms, which may have a substituent, a branched alkyl group having 3 to 7 carbon atoms, which may have a substituent, a cyclic alkyl group having 3 to 6 carbon atoms, which may have a substituent, an alkenyl group having 2 to 5 carbon atoms, which may have a substituent, an alkynyl group having 2 to 5 carbon atoms, which may have a substituent, an aryl group having 6 to 14 carbon atoms, which may have a substituent, a heteroaryl group having 2 to 14 carbon atoms, which may have a substituent, an alkoxy group having 1 to 5 carbon atoms, which may have a substituent, an aryloxy group having 6 to 14 carbon atoms, which may have a substituent, an amino group which may have a substituent, a silyl group which may have a substituent, a cyano group, or a halogen atom.
[25] The compound according to [23],
   in which D represents the group represented by any of Formula (2-10), Formula (2-11), Formula (2-12), Formula (2-13), Formula (3-10), Formula (3-11), Formula (3-12), Formula (3-13), Formula (5-10), or Formula (5-11).
[26] The compound according to any one of [21] to [25],
   in which W¹ is an oxygen atom or a sulfur atom.
[27] The compound according to any one of [21] to [26],
   in which the group represented by Formula (A-1) is a group represented by Formula (A-2) described later.
[28] The compound according to [27],
   in which the group represented by Formula (A-2) is a group represented by Formula (C-1) described later or a group represented by Formula (C-2) described later.
[29] The compound according to [28],
   in which both X^{c1} and X^{c2} are oxygen atoms and both x^{c3} and x^{c4} are oxygen atoms.
[30] The compound according to any one of [21] to [29],
   in which Rs represents a linear alkyl group having 1 to 5 carbon atoms, which may have a substituent, a branched alkyl group having 3 to 7 carbon atoms, which may have a substituent, a cyclic alkyl group having 3 to 6 carbon atoms, which may have a substituent, an aryl group having 6 to 14 carbon atoms, which may have a substituent, a heteroaryl group having 2 to 14 carbon atoms, which may have a substituent, an alkoxy group having 1 to 5 carbon atoms, which may have a substituent, an aryloxy group having 6 to 14 carbon atoms, which may have a substituent, a silyl group which may have a substituent, or a halogen atom.
[31] The compound according to [23],
   in which D represents the group represented by Formula (4-10) or the group represented by Formula (4-11).
[32] The compound according to [31],
   in which Rs represents a linear alkyl group having 1 to 5 carbon atoms, which may have a substituent, a branched alkyl group having 3 to 7 carbon atoms, which may have a substituent, a cyclic alkyl group having 3 to 6 carbon atoms, which may have a substituent, or a group represented by Formula (S1) described later.

According to the present invention, it is possible to provide a photoelectric conversion element having excellent quantum efficiency in a case of receiving blue light.

In addition, according to the present invention, it is possible to provide an imaging element, an optical sensor, a manufacturing method of an imaging element, and a compound, which are related to the above-described photoelectric conversion element.

### BRIEF DESCRIPTION OF THE DRAWINGS

Fig. 1 is a schematic cross-sectional view showing an example of a configuration of a photoelectric conversion element.
Fig. 2 is a schematic cross-sectional view showing an example of a configuration of a photoelectric conversion element.

### DESCRIPTION OF THE PREFERRED EMBODIMENTS

Hereinafter, the present invention will be described in detail.

The description of the configuration requirements described below is made on the basis of representative embodiments of the present invention, but it should not be construed that the present invention is limited to those embodiments.

Hereinafter, meaning of each description in the present specification will be explained.

In the present specification, numerical ranges represented by "to" include numerical values before and after "to" as lower limit values and upper limit values.

In the present specification, a hydrogen atom may be a light hydrogen atom (normal hydrogen atom) or a heavy hydrogen atom (for example, a deuterium atom or the like).

In the present specification, regarding a compound which may have a geometric isomer (cis-trans isomer), a general formula or a structural formula representing the compound may be described only in the form of either a cis isomer or a trans isomer for convenience. Even in such a case, unless otherwise specified, the form of the compound is not limited to either the cis isomer or the trans isomer, and the compound may be the cis isomer or the trans isomer.

A bonding direction of a divalent group (for example, -CO-O-) described in the present specification is not limited unless otherwise specified. For example, in a case where Y in a compound represented by a formula "X-Y-Z" is -CO-O-, the compound may be any of "X-O-CO-Z" or "X-CO-O-Z".

A symbol "*" specified in a chemical formula represents a bonding position unless otherwise specified.

In the present specification, in a case of a plurality of substituents, linking groups, and the like (hereinafter, also referred to as "substituent and the like") represented by specific reference numeral, or in a case of simultaneously defining a plurality of the substituent and the like, it means that each of the substituent and the like may be the same as or different with each other. This also applies to a case of specifying the number of substituents and the like.

In the present specification, the "substituent" includes a group exemplified by a substituent W described later, unless otherwise specified.

### (Substituent W)

The substituent W in the present specification will be described below.

Examples of the substituent W include a halogen atom (for example, a fluorine atom, a chlorine atom, a bromine atom, an iodine atom, and the like), an alkyl group (including a cycloalkyl group, a bicycloalkyl group, and a tricycloalkyl group), an alkenyl group (including a cycloalkenyl group and a bicycloalkenyl group), an alkynyl group, an aryl group, a heterocyclic group (a heteroaryl group or an aliphatic heterocyclic group), a cyano group, a nitro group, an alkoxy group, an aryloxy group, a silyl group, a silyloxy group, a heterocyclic oxy group, an acyloxy group, a carbamoyloxy group, an alkoxycarbonyloxy group, an aryloxycarbonyloxy group, a primary, secondary, or tertiary amino group (including an anilino group), an alkylthio group, an arylthio group, a heterocyclic thio group, an alkyl or an arylsulfinyl group, an alkyl or an arylsulfonyl group, an acyl group, an aryloxycarbonyl group, an alkoxycarbonyl group, an aryl or a heterocyclic azo group, an imide group, a phosphino group, a phosphinyl group, a phosphinyloxy group, a phosphinylamino group, a phosphono group, a carboxy group, a phosphoric acid group, a sulfonic acid group, a hydroxy group, a thiol group, an acylamino group, a carbamoyl group, a ureido group, and a boronic acid group.

In addition, each of the above-described groups may further have a substituent (for example, one or more groups of each of the above-described groups), as possible. For example, an alkyl group which may have a substituent is also included as the form of the substituent W.

In addition, in a case where the substituent W has a carbon atom, the number of carbon atoms in the substituent W is, for example, 1 to 20.

The number of atoms other than a hydrogen atom in the substituent W is, for example, 1 to 30.

It is also preferable that a specific compound described later does not contain, as a substituent, a carboxy group, a salt of a carboxy group, a salt of a phosphoric acid group, a sulfonic acid group, a salt of a sulfonic acid group, a hydroxy group, a thiol group, an acylamino group, a carbamoyl group, a ureido group, or a boronic acid group (-B(OH)₂) and/or a primary amino group.

In the present specification, the aliphatic hydrocarbon group may be linear, branched, or cyclic.

Examples of the above-described aliphatic hydrocarbon group include an alkyl group, an alkenyl group, and an alkynyl group.

In addition, in the present specification, unless otherwise specified, the number of carbon atoms in the alkyl group is preferably 1 to 20, more preferably 1 to 10, and still more preferably 1 to 6.

The alkyl group may be linear, branched, or cyclic.

Examples of the alkyl group include a methyl group, an ethyl group, an n-propyl group, an isopropyl group, an n-butyl group, a t-butyl group, an n-hexyl group, and a cyclopentyl group.

In addition, the alkyl group may be any of a cycloalkyl group, a bicycloalkyl group, or a tricycloalkyl group, and may have a ring structure thereof as a partial structure.

In the alkyl group which may have a substituent, examples of the substituent which may be included in the alkyl group include the groups exemplified as the substituent W. Among these, an aryl group (preferably having 6 to 18 carbon atoms and more preferably having 6 carbon atoms), a heteroaryl group (preferably having 5 to 18 carbon atoms and more preferably having 5 or 6 carbon atoms), or a halogen atom (preferably a fluorine atom or a chlorine atom) is preferable.

In the present specification, unless otherwise specified, an alkyl group moiety in the alkoxy group is preferably the above-described alkyl group. An alkyl group moiety in the alkylthio group is preferably the above-described alkyl group.

In the alkoxy group which may have a substituent, examples of the substituent which may be included in the alkoxy group include the same examples as the substituent in the alkyl group which may have a substituent. In the alkylthio group which may have a substituent, examples of the substituent which may be included in the alkylthio group include the same examples as the substituent in the alkyl group which may have a substituent.

In the present specification, the alkenyl group may be any of linear, branched, or cyclic, unless otherwise specified. The number of carbon atoms in the above-described alkenyl group is preferably 2 to 20. In the alkenyl group which may have a substituent, examples of the substituent which may be included in the alkenyl group include the same examples as the substituent in the alkyl group which may have a substituent.

In the present specification, the alkynyl group may be any of linear, branched, or cyclic, unless otherwise specified. The number of carbon atoms in the above-described alkynyl group is preferably 2 to 20. In the alkynyl group which may have a substituent, examples of the substituent which may be included in the alkynyl group include the same examples as the substituent in the alkyl group which may have a substituent.

In the present specification, an aromatic ring or an aromatic ring constituting the aromatic ring group may be any of a monocyclic ring or a polycyclic ring (for example, 2 to 6 rings or the like), unless otherwise specified. The monocyclic aromatic ring is an aromatic ring having only one aromatic ring structure as a ring structure. The polycyclic (for example, 2 to 6 rings or the like) aromatic ring is an aromatic ring formed by a plurality of (for example, 2 to 6 or the like) fused aromatic ring structures, as a ring structure.

The number of ring member atoms in the above-described aromatic ring is preferably 4 to 15.

The above-described aromatic ring may be an aromatic hydrocarbon ring or an aromatic heterocyclic ring.

In a case where the above-described aromatic ring is an aromatic heterocyclic ring, the number of heteroatoms included as ring member atoms is, for example, 1 to 10. Examples of the above-described heteroatom include a nitrogen atom, a sulfur atom, an oxygen atom, a selenium atom, a tellurium atom, a phosphorus atom, a silicon atom, and a boron atom.

Examples of the above-described aromatic hydrocarbon ring include a benzene ring, a naphthalene ring, an anthracene ring, and a phenanthrene ring.

Examples of the above-described aromatic heterocyclic ring include a pyridine ring, a pyrimidine ring, a pyridazine ring, a pyrazine ring, a triazine ring (for example, 1,2,3-triazine ring, 1,2,4-triazine ring, 1,3,5-triazine ring, and the like), a tetrazine ring (for example, 1,2,4,5-tetrazine ring and the like), a quinoxaline ring, a pyrrole ring, a furan ring, a thiophene ring, an imidazole ring, an oxazole ring, a thiazole ring, a benzopyrrole ring, a benzofuran ring, a benzothiophene ring, a benzimidazole ring, a benzoxazole ring, a benzothiazole ring, a naphthopyrrole ring, a naphthofuran ring, a naphthothiophene ring, a naphthimidazole ring, a naphthoxazole ring, a pyrroloimidazole ring (for example, a 5H-pyrrolo[1,2-a]imidazole ring and the like), an imidazooxazole ring (for example, an imidazo[2,1-b]oxazole ring and the like), a thienothiazole ring (for example, a thieno[2,3-d]thiazole ring and the like), a benzothiadiazole ring, a benzodithiophene ring (for example, benzo[1,2-b:4,5-b']dithiophene ring and the like), a thienothiophene ring (for example, thieno[3,2-b]thiophene ring and the like), a thiazolothiazole ring (for example, thiazolo[5,4-d]thiazole ring and the like), a naphthodithiophene ring (for example, a naphtho[2,3-b:6,7-b']dithiophene ring, a naphtho[2,1-b:6,5-b']dithiophene ring, a naphtho[1,2-b:5,6-b']dithiophene ring, a 1,8-dithiadicyclopenta[b,g]naphthalene ring, and the like), a benzothienobenzothiophene ring, a dithieno[3,2-b:2',3'-d]thiophene ring, and a 3,4,7,8-tetrathiadicyclopenta[a,e]pentalene ring.

In the aromatic ring which may have a substituent, examples of the type of the substituent which may be included in the aromatic ring include the groups exemplified as the substituent W. In a case where the above-described aromatic ring has a substituent, the number of substituents may be 1 or more (for example, 1 to 4 or the like).

In the present specification, examples of the "aromatic ring group" include a group obtained by removing one or more hydrogen atoms (for example, 1 to 5 or the like) from the above-described aromatic ring.

In the present specification, examples of the "aryl group" include a group obtained by removing one hydrogen atom from a ring corresponding to the aromatic hydrocarbon ring among the above-described aromatic rings.

In the present specification, examples of the "heteroaryl group" include a group obtained by removing one hydrogen atom from a ring corresponding to the aromatic heterocyclic ring among the above-described aromatic rings.

In the present specification, examples of the "arylene group" include a group obtained by removing two hydrogen atoms from a ring corresponding to the aromatic hydrocarbon ring among the above-described aromatic rings.

In the present specification, examples of the "heteroarylene group" include a group obtained by removing two hydrogen atoms from a ring corresponding to the aromatic heterocyclic ring among the above-described aromatic rings.

In the aromatic ring group which may have a substituent, the aryl group which may have a substituent, the heteroaryl group which may have a substituent, the arylene group which may have a substituent, and the heteroarylene group which may have a substituent, examples of the type of the substituent which may be included in these groups include the groups exemplified as the substituent W. In a case where these groups each of which may have a substituent have a substituent, the number of substituents may be 1 or more (for example, 1 to 4 or the like).

In the present specification, the number of ring members in the aliphatic heterocyclic group is preferably 5 to 20, more preferably 5 to 12, and still more preferably 6 to 8.

Examples of a heteroatom which is included in the above-described aliphatic heterocyclic group include a sulfur atom, an oxygen atom, a nitrogen atom, a selenium atom, a tellurium atom, a phosphorus atom, a silicon atom, and a boron atom; and a sulfur atom, an oxygen atom, or a nitrogen atom is preferable.

Examples of an aliphatic heterocyclic ring constituting the above-described aliphatic heterocyclic group include a pyrrolidine ring, an oxolane ring, a thiolane ring, a piperidine ring, a tetrahydropyran ring, a thiacine ring, a piperazine ring, a morpholine ring, a quinocyclidine ring, an azetidine ring, an oxetane ring, an aziridine ring, a dioxane ring, and γ-butyrolactone.

### [Photoelectric conversion element]

The photoelectric conversion element according to the embodiment of the present invention includes a conductive film, a photoelectric conversion film, and a transparent conductive film in this order, in which the photoelectric conversion film contains a compound represented by Formula (1) described later (hereinafter, referred to as "specific compound").

The mechanism by which the photoelectric conversion element according to the embodiment of the present invention can achieve the above-described problems is not necessarily clear, but the present inventors assume as follows.

The mechanism by which the effect is obtained is not limited by the following supposition. That is, even in a case where the effect is obtained by a mechanism other than the following, it is included in the scope of the present invention.

It is generally considered that an A-D-A type coloring agent in which an acceptor is directly bonded to a donor having a two-fused ring structure, such as the specific compound according to the present invention, is not desirable for obtaining a high quantum efficiency because the conjugated system is short and thus the absorbance is generally low.

However, as a result of a comprehensive search on the A-D-A type coloring agent, the present inventors have found that, contrary to expectation, a high quantum efficiency can be obtained even in the A-D-A type coloring agent in which the acceptor is directly bonded to the donor having a two-fused ring structure, and efficient absorption is exhibited even in a short wavelength range (in a vicinity of 400 to 500 nm).

As described above, the present inventors presume that the reason why the high quantum efficiency is obtained is that the absorbance of the molecule itself is not so high, but the specific compound has a relatively low molecular weight, and thus, in a case where the specific compound is formed into a film as the photoelectric conversion film, the amount of the substance per unit volume in the film can be increased.

In addition, the present inventors presume that, in the donor structure of the two-fused ring structure described above, by introducing an appropriate substituent, aggregation of the A-D-A type coloring agents is suppressed, and the transfer of electrons and holes can be efficiently performed, and as a result, the higher quantum efficiency can be achieved as compared with the photoelectric conversion element disclosed in WO2014/026224A, which does not have a substituent on the donor site.

Hereinafter, the fact that the quantum efficiency in a case where the photoelectric conversion element receives blue light (light in a wavelength range of 400 to 500 nm) is more excellent is also referred to as the fact that the effect of the present invention is more excellent.

A configuration of the photoelectric conversion element according to the embodiment of the present invention will be described in detail below.

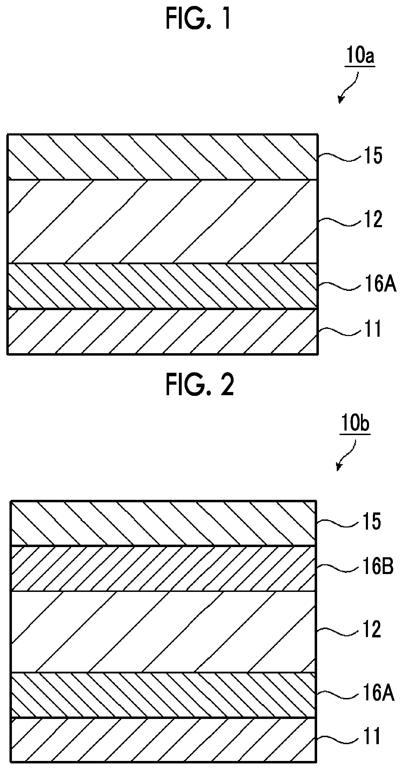

Fig. 1 shows a schematic cross-sectional view of one embodiment of the photoelectric conversion element according to the present invention.

A photoelectric conversion element 10a shown in Fig. 1 has a configuration in which a conductive film (hereinafter, also referred to as "lower electrode") 11 functioning as a lower electrode, an electron blocking film 16A, a photoelectric conversion film 12 containing the specific compound, and a transparent conductive film (hereinafter, also referred to as "upper electrode") 15 functioning as an upper electrode are laminated in this order.

Fig. 2 shows a configuration example of another photoelectric conversion element. A photoelectric conversion element 10b shown in Fig. 2 has a configuration in which the electron blocking film 16A, the photoelectric conversion film 12, a hole blocking film 16B, and the upper electrode 15 are laminated on the lower electrode 11 in this order. The lamination order of the electron blocking film 16A, the photoelectric conversion film 12, and the hole blocking film 16B in Figs. 1 and 2 may be appropriately changed according to the application and the characteristics.

In the photoelectric conversion element 10a (or 10b), it is preferable that light is incident to the photoelectric conversion film 12 through the upper electrode 15.

In a case where the photoelectric conversion element 10a (or 10b) is used, a voltage can be applied. In this case, it is preferable that the lower electrode 11 and the upper electrode 15 form a pair of electrodes, and a voltage of 1 × 10⁻⁵ to 1 × 10⁷ V/cm is applied between the pair of electrodes. From the viewpoint of performance and power consumption, the applied voltage is more preferably 1 × 10⁻⁴ to 1 × 10⁷ V/cm and still more preferably 1 × 10⁻³ to 5 × 10⁶ V/cm.

Regarding the voltage application method, in Figs. 1 and 2, it is preferable that the voltage is applied such that the electron blocking film 16A side is a cathode and the photoelectric conversion film 12 side is an anode. In a case where the photoelectric conversion element 10a (or 10b) is used as an optical sensor, or also in a case of being incorporated in an imaging element, the voltage can be applied by the same method.

As described in detail below, the photoelectric conversion element 10a (or 10b) can be suitably applied to applications of an imaging element.

Hereinafter, the form of each layer constituting the photoelectric conversion element according to the embodiment of the present invention will be described in detail.

### [Photoelectric conversion film]

The photoelectric conversion element according to the embodiment of the present invention includes a photoelectric conversion film.

### <Specific compound>

The photoelectric conversion film contains a compound represented by Formula (1) (specific compound).

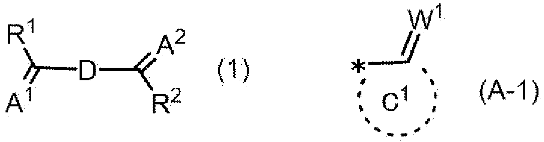

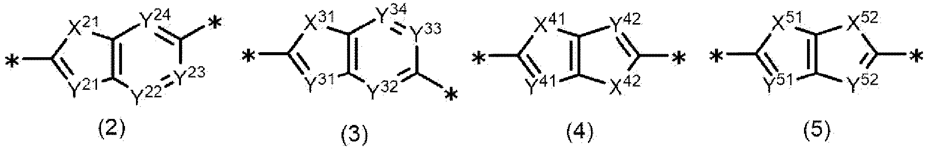

In Formula (1), D represents a group represented by any of Formula (2) to Formula (5).

In Formula (2) to Formula (5), X²¹, X³¹, X⁴¹, X⁴², X⁵¹, and X⁵² each independently represent a sulfur atom, an oxygen atom, NR^{X1}, or CR^{X2}R^{X3}.

R^{X1} to R^{X3} each independently represent a hydrogen atom, an aliphatic hydrocarbon group which may have a substituent, or an aromatic ring group which may have a substituent.

R^{X2} and R^{X3} may be linked to each other through a single bond or a divalent linking group. Examples of the divalent linking group include an alkylene group and -O-.

In Formula (1), from the viewpoint that the effect of the present invention is more excellent, D is preferably the group represented by Formula (2), Formula (3), or Formula (5), and more preferably the group represented by Formula (2).

In Formulae (2) to (5), X²¹, X³¹, X⁴¹, X⁴², X⁵¹, and X⁵² are each preferably a sulfur atom or an oxygen atom, and more preferably a sulfur atom.

Examples of the aliphatic hydrocarbon group which may have a substituent, represented by R^{X1} to R^{X3}, include an alkyl group, an alkenyl group, and an alkynyl group. The number of carbon atoms in the above-described aliphatic hydrocarbon group is preferably 1 to 10, more preferably 1 to 6, and still more preferably 1 or 2. Examples of the substituent which may be included in the aliphatic hydrocarbon group include the groups exemplified as the substituent W.

Among these, the aliphatic hydrocarbon group which may have a substituent is preferably a linear or branched alkyl group having 1 to 5 carbon atoms, and more preferably a methyl group, an ethyl group, or an isopropyl group.

In addition, a specific aspect of the aromatic ring group which may have a substituent, represented by R^{X1} to R^{X3}, is as described above; and examples thereof include the aryl group which may have a substituent and the heteroaryl group which may have a substituent. Examples of the substituent which may be included in the aromatic ring group include the groups exemplified as the substituent W. As the substituent which may be included in the aromatic ring group, a methyl group is preferable.

The aromatic ring group which may have a substituent, represented by R^{X1} to R^{X3}, is preferably an aryl group having 6 to 14 carbon atoms, which may have a substituent, or a heteroaryl group having 2 to 14 carbon atoms, which may have a substituent, and more preferably a phenyl group.

In Formula (2) to Formula (5), Y²¹, Y²², Y²³, Y²⁴, Y³¹, Y³², Y³³, Y³⁴, Y⁴¹, Y⁴², Y⁵¹, and Y⁵² each independently represent -CR^{Y1}= or a nitrogen atom. R^{Y1} represents a hydrogen atom or a substituent.

Here, in Formula (2), at least one of Y²¹, ..., or Y²⁴ represents -CRs=, in Formula (3), at least one of Y³¹, ..., or Y³⁴ represents -CRs=, in Formula (4), at least one of Y⁴¹ or Y⁴² represents -CRs=, and in Formula (5), at least one of Y⁵¹ or Y⁵² represents -CRs=.

The Rs represents an alkyl group which may have a substituent, an alkenyl group which may have a substituent, an alkynyl group which may have a substituent, an aryl group which may have a substituent, a heteroaryl group which may have a substituent, an alkoxy group which may have a substituent, an aryloxy group which may have a substituent, an amino group which may have a substituent, a silyl group which may have a substituent, a cyano group, or a halogen atom.

R^{Y1} is preferably a hydrogen atom.

The substituent represented by R^{Y1} is not particularly limited, and examples thereof include the groups exemplified as the substituent W. As the substituent represented by R^{Y1} an alkyl group which may have a substituent, an alkenyl group which may have a substituent, an alkynyl group which may have a substituent, an aryl group which may have a substituent, a heteroaryl group which may have a substituent, an alkoxy group which may have a substituent, an aryloxy group which may have a substituent, an amino group which may have a substituent, a silyl group which may have a substituent, a cyano group, or a halogen atom is preferable; and an alkyl group which may have a substituent, an aryl group which may have a substituent, a heteroaryl group which may have a substituent, an alkoxy group which may have a substituent, a silyl group which may have a substituent, or a halogen atom is more preferable.

A more preferred aspect of the substituent represented by R^{Y1} is the same as the preferred aspect of each group represented by Rs, which will be described later.

In Formula (2), it is preferable that at least one of Y²¹, ..., or Y²⁴ represents -CR^{Y1}=, it is more preferable that at least two thereof represent -CR^{Y1}=, it is still more preferable that at least three thereof represent -CR^{Y1}=, and it is particularly preferable that all of Y²¹ to Y²⁴ represent -CR^{Y1}=.

In Formula (3), it is preferable that at least one of Y³¹, ..., or Y³⁴ represents -CR^{Y1}=, it is more preferable that at least two thereof represent -CR^{Y1}=, it is still more preferable that at least three thereof represent -CR^{Y1}=, and it is particularly preferable that all of Y³¹ to Y³⁴ represent -CR^{Y1}=.

In Formula (4), it is preferable that at least one of Y⁴¹ or Y⁴² represents -CR^{Y1}=, and it is more preferable that both Y⁴¹ and Y⁴² represent -CR^{Y1}=.

In Formula (5), it is preferable that at least one of Y⁵¹ or Y⁵² represents -CR^{Y1}=, and it is more preferable that both Y⁵¹ and Y⁵² represent -CR^{Y1}=.

In Formula (2), it is preferable that one or two of Y²¹ to Y²⁴ represent -CRs=, and it is more preferable that one represents -CRs=.

In Formula (3), it is preferable that one or two of Y³¹ to Y³⁴ represent -CRs=, and it is more preferable that one represents -CRs=.

In Formula (4), it is preferable that at least one of Y⁴¹ or Y⁴² represents -CRs=, and it is more preferable that one of Y⁴¹ or Y⁴² represents -CRs=.

In Formula (5), it is preferable that at least one of Y⁵¹ or Y⁵² represents -CRs=, and it is more preferable that one of Y⁵¹ or Y⁵² represents -CRs=.

In a case where the group represented by Y²¹ to Y²⁴, Y³¹ to Y³⁴, Y⁴¹ and Y⁴², and Y⁵¹ and Y⁵² can correspond to both -CR^{Y1}= and CRs=, the group is counted as -CRs=. For example, in a case where two of Y⁴¹ and Y⁴² are -CCH₃=, -CCH₃= corresponds to both -CR^{Y1}= and CRs=, but the count is made such that there are two -CRs=. In other words, a method of counting such that one of two -CCH₃='s corresponds to -CR^{Y1}= and the other corresponds to -CRs=, and thus the number of -CRs= is one is not used.

As described above, Rs represents an alkyl group which may have a substituent, an alkenyl group which may have a substituent, an alkynyl group which may have a substituent, an aryl group which may have a substituent, a heteroaryl group which may have a substituent, an alkoxy group which may have a substituent, an aryloxy group which may have a substituent, an amino group which may have a substituent, a silyl group which may have a substituent, a cyano group, or a halogen atom.

The alkyl group which may have a substituent, represented by Rs, may be linear, branched, or cyclic.

In a case where the above-described alkyl group is a linear alkyl group, the number of carbon atoms is preferably 1 to 10, more preferably 1 to 5, and still more preferably 1 or 2.

In a case where the above-described alkyl group is a branched alkyl group, the number of carbon atoms is preferably 3 to 10 and more preferably 3 to 7.

In a case where the above-described alkyl group is a cyclic alkyl group, the number of carbon atoms is preferably 3 to 10 and more preferably 3 to 6.

In addition, examples of the substituent which may be included in the alkyl group include the groups exemplified as the substituent W. As the substituent which may be included in the alkyl group, a halogen atom is preferable, and a fluorine atom is more preferable. The alkyl group may be a perfluoroalkyl group such as a trifluoromethyl group.

The alkenyl group which may have a substituent, represented by Rs, may be linear, branched, or cyclic.

The number of carbon atoms in the alkenyl group is preferably 2 to 10, more preferably 2 to 6, and still more preferably 2 to 5.

In addition, examples of the substituent which may be included in the alkenyl group include the groups exemplified as the substituent W. As the substituent which may be included in the alkenyl group, an aryl group is preferable, an aryl group having 6 to 10 carbon atoms is more preferable, and a phenyl group is still more preferable.

Among these, as the alkenyl group, a vinyl group, a group obtained by removing a hydrogen atom at the 2-position of propylene, or a group obtained by removing a hydrogen atom at the 1-position of isobutene is preferable.

Examples of the substituent which may be included in the alkynyl group which may have a substituent, represented by Rs, include the groups exemplified as the substituent W. As the substituent which may be included in the alkynyl group, an aryl group having 6 to 10 carbon atoms is preferable, and a phenyl group is more preferable.

The number of carbon atoms in the alkynyl group is preferably 2 to 10, more preferably 2 to 6, and still more preferably 2 to 5.

Among these, the alkynyl group which may have a substituent is preferably a propargyl group, a 1-methylpropargyl group, or a phenylacetynyl group.

The aryl group which may have a substituent, represented by Rs, is not particularly limited, and may be monocyclic or polycyclic.

The number of carbon atoms in the above-described aryl group is preferably 6 to 20, more preferably 6 to 14, and still more preferably 6 to 10.

Examples of the substituent which may be included in the aryl group include the groups exemplified as the substituent W. Examples of the substituent which may be included in the aryl group include a halogen atom, an alkyl group, and an alkoxy group. Specific aspects of the above-described groups are as described above, but the substituent which may be included in the aryl group is preferably a methyl group, an isopropyl group, or a methoxy group.

The heteroaryl group which may have a substituent, represented by Rs, is not particularly limited, and may be monocyclic or polycyclic.

Specific examples of an aromatic heterocyclic ring constituting the above-described heteroaryl group are as described above.

The number of carbon atoms in the heteroaryl group is preferably 2 to 20, more preferably 2 to 14, and still more preferably 2 to 10. In addition, the number of heteroatoms included in the heteroaryl group is preferably 1 to 10, more preferably 1 to 3, and still more preferably 1 or 2.

Examples of the substituent which may be included in the heteroaryl group include the groups exemplified as the substituent W. Examples of the substituent which may be included in the heteroaryl group include a halogen atom, an alkyl group, and an alkoxy group. Specific aspects of the above-described groups are as described above, but the substituent which may be included in the heteroaryl group is preferably a methyl group, an isopropyl group, or a methoxy group.

In the alkoxy group which may have a substituent, represented by Rs, an alkyl group bonded to an oxygen atom may be linear, branched, or cyclic.

In a case where the above-described alkyl group is a linear alkyl group, the number of carbon atoms is preferably 1 to 10, more preferably 1 to 5, and still more preferably 1 or 2.

In a case where the above-described alkyl group is a branched alkyl group, the number of carbon atoms is preferably 3 to 10 and more preferably 3 to 7.

In a case where the above-described alkyl group is a cyclic alkyl group, the number of carbon atoms is preferably 3 to 10 and more preferably 3 to 6.

Examples of the substituent which may be included in the alkoxy group include the groups exemplified as the substituent W.

In the aryloxy group which may have a substituent, represented by Rs, an aryl group bonded to an oxygen atom is not particularly limited, and may be monocyclic or polycyclic.

The number of carbon atoms in the above-described aryl group is preferably 6 to 20, more preferably 6 to 14, and still more preferably 6 to 10.

Examples of the substituent which may be included in the aryl group include the groups exemplified as the substituent W. Examples of the substituent which may be included in the aryl group include a halogen atom, an alkyl group, and an alkoxy group. Specific aspects of the above-described groups are as described above, but the substituent which may be included in the aryl group is preferably a methyl group, an isopropyl group, or a methoxy group.

The amino group which may have a substituent, represented by Rs, is not particularly limited, and may be a primary amino group (-NH₂), a secondary amino group (-NR^{T}H), or a tertiary amino group (-NR^{T}₂).

R^{T} represents a substituent which may be included in the above-described amino group, and a plurality of R^{T}'s may be different from each other. R^{T} is preferably the above-described alkyl group which may have a substituent, represented by Rs, and more preferably an alkyl group having 1 to 3 carbon atoms.

Examples of the silyl group which may have a substituent, represented by Rs, include a group represented by -Si(R^{S1})(R^{S2})(R^{S3}). R^{S1}, R^{S2}, and R^{S3} each independently represent an alkyl group which may have a substituent, an alkoxy group which may have a substituent, an alkylthio group which may have a substituent, an aryl group which may have a substituent, or a heteroaryl group which may have a substituent; and an alkyl group which may have a substituent or an aryl group which may have a substituent is preferable.

Examples of the substituent which may be included in each of the groups represented by R^{S1}, R^{S2}, and R^{S3} include the groups exemplified as the substituent W.

In addition, suitable aspects of each of the groups represented by R^{S1}, R^{S2}, and R^{S3} are the same as the suitable aspects of the alkyl group which may have a substituent, the alkoxy group which may have a substituent, the alkylthio group which may have a substituent, the aryl group which may have a substituent, or the heteroaryl group which may have a substituent, which are represented by Rs.

As the halogen atom represented by Rs, a fluorine atom or a chlorine atom is preferable.

Among these, Rs preferably represents a linear alkyl group having 1 to 5 carbon atoms, which may have a substituent, a branched alkyl group having 3 to 7 carbon atoms, which may have a substituent, a cyclic alkyl group having 3 to 6 carbon atoms, which may have a substituent, an alkenyl group having 2 to 5 carbon atoms, which may have a substituent, an alkynyl group having 2 to 5 carbon atoms, which may have a substituent, an aryl group having 6 to 14 carbon atoms, which may have a substituent, a heteroaryl group having 2 to 14 carbon atoms, which may have a substituent, an alkoxy group having 1 to 5 carbon atoms, which may have a substituent, an aryloxy group having 6 to 14 carbon atoms, which may have a substituent, an amino group which may have a substituent, a silyl group which may have a substituent, a cyano group, or a halogen atom; and more preferably represents a linear alkyl group having 1 to 5 carbon atoms, which may have a substituent, a branched alkyl group having 3 to 7 carbon atoms, which may have a substituent, a cyclic alkyl group having 3 to 6 carbon atoms, which may have a substituent, an aryl group having 6 to 14 carbon atoms, which may have a substituent, a heteroaryl group having 2 to 14 carbon atoms, which may have a substituent, an alkoxy group having 1 to 5 carbon atoms, which may have a substituent, an aryloxy group having 6 to 14 carbon atoms, which may have a substituent, a silyl group which may have a substituent, or a halogen atom.

From the viewpoint that the effect of the present invention is more excellent, D in Formula (1) is preferably a group represented by any of Formula (2-10), Formula (2-11), Formula (2-12), Formula (2-13), Formula (3-10), Formula (3-11), Formula (3-12), Formula (3-13), Formula (4-10), Formula (4-11), Formula (5-10), or Formula (5-11); and more preferably a group represented by any of Formula (2-10), Formula (2-11), Formula (2-12), Formula (2-13), Formula (3-10), Formula (3-11), Formula (3-12), Formula (3-13), Formula (5-10), or Formula (5-11).

In addition, it is also preferable that D represents a group represented by Formula (4-10) or a group represented by Formula (4-11). In a case where D represents the group represented by Formula (4-10) or the group represented by Formula (4-11), electric field strength dependence of the quantum efficiency is more excellent.

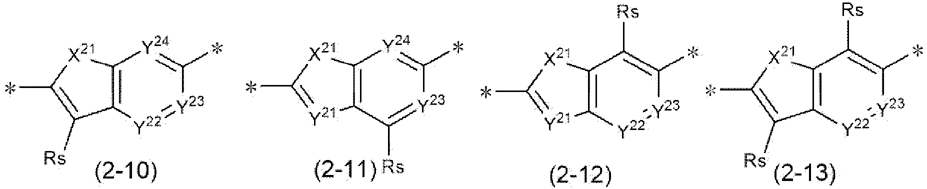

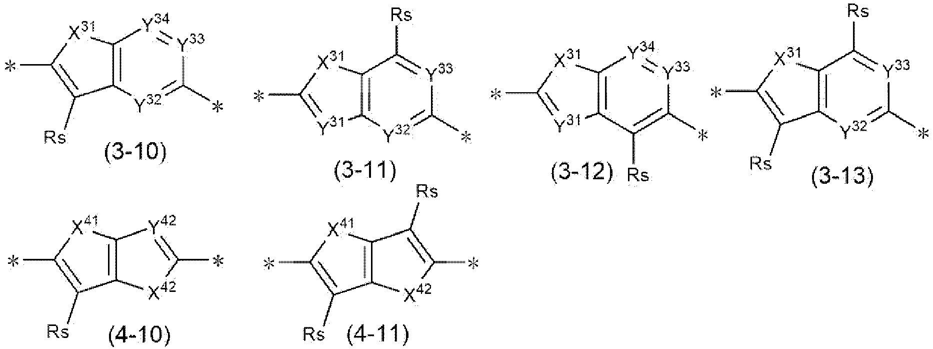

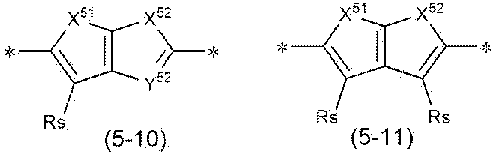

In Formula (2-10), Formula (2-11), Formula (2-12), Formula (2-13), Formula (3-10), Formula (3-11), Formula (3-12), Formula (3-13), Formula (4-10), Formula (4-11), Formula (5-10), and Formula (5-11), X²¹, X³¹, X⁴¹, X⁴², X⁵¹, and X⁵² each independently represent a sulfur atom, an oxygen atom, NR^{X1}, or CR^{X2}R^{X3}.

R^{X1} to R^{X3} each independently represent a hydrogen atom, an aliphatic hydrocarbon group which may have a substituent, or an aromatic ring group which may have a substituent.

Specific aspects and preferred aspects of R^{X1} to R^{X3} are as described above.

Specific aspects and preferred aspects of X²¹, X³¹, X⁴¹, X⁴², X⁵¹, and X⁵² are the same as the specific aspects and preferred aspects of X²¹, X³¹, X⁴¹, X⁴², X⁵¹, and X⁵² in Formula (2) to Formula (5).

In Formula (2-10), Formula (2-11), Formula (2-12), Formula (2-13), Formula (3-10), Formula (3-11), Formula (3-12), Formula (3-13), Formula (4-10), Formula (4-11), Formula (5-10), and Formula (5-11), Y²¹, Y²², Y²³, Y²⁴, Y³¹, Y³² , Y³³, Y³⁴, Y⁴², and Y⁵² each independently represent -CR^{Y1}= or a nitrogen atom. R^{Y1} represents a hydrogen atom or a substituent.

Specific aspects and suitable aspects of Y²¹, Y²², Y²³, Y²⁴, Y³¹, Y³², Y³³, Y³⁴, Y⁴², and Y⁵² are the same as the specific aspects and suitable aspects of Y²¹, Y²², Y²³, Y²⁴, Y³¹, Y³², Y³³, Y³⁴, Y⁴², and Y⁵² in Formula (2) to Formula (5).

In Formula (2-10), it is preferable that at least one of Y²², ..., or Y²⁴ represents -CR^{Y1}=, it is more preferable that at least two thereof represent -CR^{Y1}=, and it is still more preferable that all of Y²² to Y²⁴ represent -CR^{Y1}=.

In Formula (2-11), it is preferable that at least one of Y²¹, Y²³, or Y²⁴ represents -CR^{Y1}=, it is more preferable that at least two thereof represent -CR^{Y1}=, and it is still more preferable that all of Y²¹, Y²³, and Y²⁴ represent -CR^{Y1}=.

In Formula (2-12), it is preferable that at least one of Y²¹, ..., or Y²³ represents -CR^{Y1}=, it is more preferable that at least two thereof represent -CR^{Y1}=, and it is still more preferable that all of Y²¹ to Y²³ represent -CR^{Y1}=.

In Formula (2-13), it is preferable that at least one of Y²² or Y²³ represents -CR^{Y1}=, and it is more preferable that both Y²² and Y²³ represent -CR^{Y1}=.

In Formula (3-10), it is preferable that at least one of Y³², ..., or Y³⁴ represents -CR^{Y1}=, it is more preferable that at least two thereof represent -CR^{Y1}=, and it is still more preferable that all of Y³² to Y³⁴ represent -CR^{Y1}=.

In Formula (3-11), it is preferable that at least one of Y³¹, ..., or Y³³ represents -CR^{Y1}=, it is more preferable that at least two thereof represent -CR^{Y1}=, and it is still more preferable that all of Y³¹ to Y³³ represent -CR^{Y1}=.

In Formula (3-12), it is preferable that at least one of Y³¹, Y³³, or Y³⁴ represents -CR^{Y1}=, it is more preferable that at least two thereof represent -CR^{Y1}=, and it is still more preferable that all of Y³¹, Y³³, and Y³⁴ represent -CR^{Y1}=.

In Formula (3-13), it is preferable that at least one of Y³² or Y³³ represents -CR^{Y1}=, and it is more preferable that both Y³² and Y³³ represent -CR^{Y1}=.

In Formula (4-10), Y⁴² preferably represents -CR^{Y1}=.

In Formula (5-10), Y⁵² preferably represents -CR^{Y1}=.

In Formula (2-10), Formula (2-11), Formula (2-12), Formula (2-13), Formula (3-10), Formula (3-11), Formula (3-12), Formula (3-13), Formula (4-10), Formula (4-11), Formula (5-10), and Formula (5-11), Rs represents an alkyl group which may have a substituent, an alkenyl group which may have a substituent, an alkynyl group which may have a substituent, an aryl group which may have a substituent, a heteroaryl group which may have a substituent, an alkoxy group which may have a substituent, an aryloxy group which may have a substituent, an amino group which may have a substituent, a silyl group which may have a substituent, a cyano group, or a halogen atom.

Specific aspects and preferred aspects of Rs are the same as the specific aspects and preferred aspects of Rs in Formula (2) to Formula (5).

Among these, in a case where D in Formula (1) represents a group represented by any of Formula (2-10), Formula (2-11), Formula (2-12), Formula (2-13), Formula (3-10), Formula (3-11), Formula (3-12), Formula (3-13), Formula (5-10), or Formula (5-11), from the viewpoint that the effect of the present invention is more excellent, it is preferable that Rs represents a linear alkyl group having 1 to 5 carbon atoms, which may have a substituent, a branched alkyl group having 3 to 7 carbon atoms, which may have a substituent, a cyclic alkyl group having 3 to 6 carbon atoms, which may have a substituent, an alkenyl group having 2 to 5 carbon atoms, which may have a substituent, an alkynyl group having 2 to 5 carbon atoms, which may have a substituent, an aryl group having 6 to 14 carbon atoms, which may have a substituent, a heteroaryl group having 2 to 14 carbon atoms, which may have a substituent, an alkoxy group having 1 to 5 carbon atoms, which may have a substituent, an aryloxy group having 6 to 14 carbon atoms, which may have a substituent, an amino group which may have a substituent, a silyl group which may have a substituent, a cyano group, or a halogen atom.

In addition, in a case where D represents a group represented by any of Formula (4-10) or Formula (4-11), from the viewpoint that the electric field strength dependence of the quantum efficiency is more excellent, Rs is preferably a linear alkyl group having 1 to 5 carbon atoms, which may have a substituent, a branched alkyl group having 3 to 7 carbon atoms, which may have a substituent, a cyclic alkyl group having 3 to 6 carbon atoms, which may have a substituent, or a group represented by Formula (S1) shown below.

In addition, the group represented by Formula (S1) may be a group represented by Formula (S2).

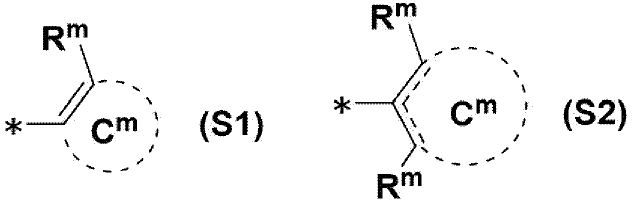

In Formula (S1), C^{m} represents an aromatic hydrocarbon having 6 to 14 carbon atoms, which may have a substituent.

R^{m} represents a substituent.

In Formula (S1), C^{m} represents an aromatic hydrocarbon having 6 to 14 carbon atoms, which may have a substituent. The aromatic hydrocarbon represented by C^{m} may be monocyclic or polycyclic, but is preferably monocyclic.

Specific examples of the aromatic hydrocarbon ring and the aromatic heterocyclic ring are as described above.

The number of substituents (the number including R^{m} specified in the formula) which may be included in the aromatic hydrocarbon represented by C^{m} is preferably 1 to 5, more preferably 1 to 4, and still more preferably 1 or 2.

Examples of the substituent which may be included in the aromatic hydrocarbon represented by C^{m} include the groups exemplified as the substituent W; and among these, a halogen atom, an alkyl group, an aryl group, an alkoxy group, or a silyl group is preferable, and a methyl group, an isopropyl group, or a methoxy group is more preferable.

Preferred aspects of each group (halogen atom, alkyl group, aryl group, alkoxy group, and silyl group) exemplified as the substituent are the same as the preferred aspects of each group exemplified as Rs.

Among these, C^{m} is preferably an aromatic hydrocarbon having 6 to 10 carbon atoms, which may have a substituent, and more preferably a benzene ring which may have a substituent.

In Formula (S1), R^{m} represents a substituent.

Examples of the substituent represented by R^{m} include each group exemplified as the substituent W; and among these, an alkyl group which may have a substituent, an alkenyl group which may have a substituent, an alkynyl group which may have a substituent, an aryl group which may have a substituent, a heteroaryl group which may have a substituent, an alkoxy group which may have a substituent, an aryloxy group which may have a substituent, an amino group which may have a substituent, a silyl group which may have a substituent, a cyano group, or a halogen atom is preferable, and an alkyl group which may have a substituent, an aryl group which may have a substituent, an alkoxy group which may have a substituent, or a halogen atom is more preferable.

Preferred aspects of each group exemplified above are the same as the preferred aspects of each group exemplified as Rs.

In Formula (S2), C^{m} represents an aromatic hydrocarbon having 6 to 14 carbon atoms, which may have a substituent.

R^{m}'s each independently represent a substituent.

Suitable aspects of C^{m} and R^{m} in Formula (S2) are the same as the suitable aspects of C^{m} and R^{m} in Formula (S1).

In Formula (1), R¹ and R² each independently represent a hydrogen atom or a substituent.

Examples of the substituent represented by R¹ and R² include the groups exemplified as the substituent W. Among these, from the viewpoint that the effect of the present invention is more excellent, R¹ and R² are each preferably a hydrogen atom.

In Formula (1), A¹ and A² each independently represent the above-described group represented by Formula (A-1).

In Formula (A-1), W¹ represents a sulfur atom, an oxygen atom, =NR^{W2}, or -CR^{W3}R^{W4}.

R^{W2} represents a hydrogen atom or a substituent. R^{W3} and R^{W4} each independently represent a cyano group, -SO₂R^{W5}, -COOR^{W6}, or -COR^{W7}.

From the viewpoint that the effect of the present invention is more excellent, W¹ preferably represents an oxygen atom or a sulfur atom.

Examples of the substituent represented by R^{W2} include the substituents exemplified as the substituent W.

In addition, R^{W5} to R^{W7} each independently represent an aliphatic hydrocarbon group which may have a substituent, an aromatic ring group which may have a substituent, or an aliphatic heterocyclic group which may have a substituent.

Examples of the substituent which may be included in the groups represented by R^{W5} to R^{W7} include the substituents exemplified as the substituent W.

The definition of the above-described aliphatic hydrocarbon group is as described above; and among these, an alkyl group is preferable, and a linear alkyl group is more preferable. The number of carbon atoms in the aliphatic hydrocarbon group is preferably 1 to 3.

The definition of the above-described aromatic ring group is as described above; and among these, an aryl group is preferable, and a phenyl group is more preferable.

The definition of the above-descried aliphatic heterocyclic group is as described above.

**In** Formula (A-1), C¹ represents a ring which contains two or more carbon atoms and may have a substituent.

The number of carbon atoms in the above-described ring is preferably 3 to 30, more preferably 3 to 20, and still more preferably 3 to 10. The above-described number of carbon atoms is a number including two carbon atoms specified in the formula.

The above-described ring may be aromatic or non-aromatic.

The above-described ring may be any of a monocyclic ring or a polycyclic ring, and is preferably a 5-membered ring, a 6-membered ring, or a fused ring including at least one of a 5-membered ring or a 6-membered ring. The number of rings forming the fused ring is preferably 1 to 4 and more preferably 1 to 3.

The above-described ring may have a heteroatom. Examples of the heteroatom include a nitrogen atom, a sulfur atom, an oxygen atom, a selenium atom, a tellurium atom, a phosphorus atom, a silicon atom, and a boron atom; and a sulfur atom, a nitrogen atom, or an oxygen atom is preferable.

The number of heteroatoms in the above-described ring is preferably 0 to 10 and more preferably 0 to 5.

Among the carbon atoms constituting the ring represented by C¹, a carbon atom other than the carbon atom at a bonding position to which * is attached in Formula (A-1) and the carbon atom bonded to W¹ may be replaced with a carbonyl carbon (>C=O) or a thiocarbonyl carbon (>C=S).

Examples of the substituent which may be included in the ring include the groups exemplified as the substituent W; and a halogen atom, an alkyl group, an aromatic ring group, or a silyl group is preferable, and a halogen atom or an alkyl group is more preferable.

The above-described alkyl group may be linear, branched, or cyclic, and is preferably linear.

The number of carbon atoms in the above-described alkyl group is preferably 1 to 10 and more preferably 1 to 3.

In Formula (A-1), as the above-described ring represented by C¹, a ring used as an acidic nucleus (for example, an acidic nucleus of a merocyanine coloring agent) is preferable; and examples thereof include the following nuclei.
(a) 1,3-dicarbonyl nucleus: for example, 1,3-indandione nucleus, 1,3-cyclohexanedione, 5,5-dimethyl-1,3-cyclohexanedione, 1,3-dioxane-4,6-dione, and the like
(b) pyrazolinone nucleus: for example, 1-phenyl-2-pyrazolin-5-one, 3-methyl-1-phenyl-2-pyrazolin-5-one, 1-(2-benzothiazolyl)-3-methyl-2-pyrazolin-5-one, and the like
(c) isoxazolinone nucleus: for example, 3-phenyl-2-isoxazolin-5-one, 3-methyl-2-isoxazolin-5-one, and the like
(d) oxindole nucleus: for example, 1-alkyl-2,3-dihydro-2-oxindole and the like
(e) 2,4,6-trioxohexahydropyrimidine nucleus: for example, barbituric acid, 2-thibarbituric acid and derivatives thereof, and the like; examples of the above-described derivatives include a 1-alkyl form such as 1-methyl and 1-ethyl, a 1,3-dialkyl form such as 1,3-dimethyl, 1,3-diethyl, and 1,3-dibutyl, a 1,3-diaryl form such as 1,3-diphenyl, 1,3-di(p-chlorophenyl), 1,3-di(p-ethoxycarbonylphenyl), a 1-alkyl-1-aryl form such as 1-ethyl-3-phenyl, and a 1,3-diheteroaryl form such as 1,3-di(2-pyridyl).
(f) 2-thio-2,4-thiazolidinedione nucleus: for example, rhodanine and derivatives thereof, and the like; examples of the above-described derivatives include a 3-alkylrhodanine such as 3-methylrhodanine, 3-ethylrhodanine, and 3-allylrhodanine, a 3-arylrhodanine such as 3-phenylrhodanine, and a 3-heteroaryl rhodanine such as 3-(2-pyridyl)rhodanine.
(g) 2-thio-2,4-oxazolidinedione (2-thio-2,4-(3H,5H)-oxazoledione) nucleus: for example, 3-ethyl-2-thio-2,4-oxazolidinedione and the like
(h) thianaphthenone nucleus: for example, 3(2H)-thianaphthenone-1,1-dioxide and the like
(i) 2-thio-2,5-thiazolidinedione nucleus: for example, 3-ethyl-2-thio-2,5-thiazolidinedione and the like
(j) 2,4-thiazolidinedione nucleus: for example, 2,4-thiazolidinedione, 3-ethyl-2,4-thiazolidinedione, 3-phenyl-2,4-thiazolidinedione, and the like
(k) thiazolin-4-one nucleus: for example, 4-thiazolinone, 2-ethyl-4-thiazolinone, and the like
(l) 2,4-imidazolidinedione (hydantoin) nucleus: for example, 2,4-imidazolidinedione, 3-ethyl-2,4-imidazolidinedione, and the like
(m) 2-thio-2,4-imidazolidinedione (2-thiohydantoine) nucleus: for example, 2-thio-2,4-imidazolidinedione, 3-ethyl-2-thio-2,4-imidazolidinedione, and the like
(n) imidazolin-5-one nucleus: for example, 2-propylmercapto-2-imidazolin-5-one and the like
(o) 3,5-pyrazolidinedione nucleus: for example, 1,2-diphenyl-3,5-pyrazolidinedione, 1,2-dimethyl-3,5-pyrazolidinedione, and the like
(p) benzothiophen-3(2H)-one nucleus: for example, benzothiophen-3(2H)-one, oxobenzothiophen-3(2H)-one, dioxobenzothiophen-3(2H)-one, and the like
(q) indanone nucleus: for example, 1-indanone, 3-phenyl-1-indanone, 3-methyl-1-indanone, 3,3-diphenyl-1-indanone, 3,3-dimethyl-1-indanone, and the like
(r) benzofuran-3-(2H)-one nucleus: for example, benzofuran-3-(2H)-one and the like
(s) 2,2-dihydrophenalene-1,3-dione nucleus and the like

From the viewpoint that the effect of the present invention is more excellent, the above-described group represented by Formula (A-1) is preferably a group represented by Formula (A-2).

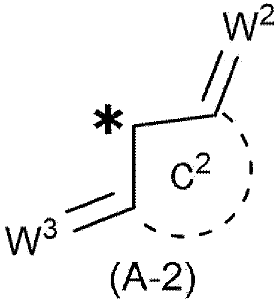

In Formula (A-2), * represents a bonding position.

W² and W³ each independently represent an oxygen atom or a sulfur atom.

It is preferable that both W² and W³ represent an oxygen atom.

In addition, in Formula (A-2), C² represents a ring which contains three or more carbon atoms and may have a substituent.

Three carbon atoms contained in C² are the three carbon atoms specified in Formula (A-2).

The number of carbon atoms in the above-described ring is preferably 3 to 30, more preferably 3 to 20, and still more preferably 3 to 10. The number of carbon atoms in the above-described ring is the number including the three carbon atoms specified in the formula.

The above-described ring may be an aromatic ring or a non-aromatic ring.

The above-described ring may be any of a monocyclic ring or a polycyclic ring, and is preferably a 5-membered ring, a 6-membered ring, or a fused ring including at least one of a 5-membered ring or a 6-membered ring. In a case where the ring is a polycyclic ring, the number of rings included is preferably 2 to 6 and more preferably 2 or 3.

The above-described ring may have a heteroatom. Examples of the heteroatom include a nitrogen atom, a sulfur atom, an oxygen atom, a selenium atom, a tellurium atom, a phosphorus atom, a silicon atom, and a boron atom; and a sulfur atom, a nitrogen atom, or an oxygen atom is preferable.

The number of heteroatoms in the above-described ring is preferably 0 to 10 and more preferably 0 to 5.

Among the carbon atoms constituting the ring represented by C², a carbon atom other than the carbon atom at a bonding position to which * is attached in Formula (A-2) and the carbon atom bonded to W² and W³ may be replaced with a carbonyl carbon (>C=O) or a thiocarbonyl carbon (>C=S).

A suitable aspect of the substituent which may be included in the ring is the same as that of the substituent which may be included in the ring C¹ described above.

In addition, it is preferable that the above-described group represented by Formula (A-2) is a group represented by Formula (C-1) or a group represented by Formula (C-2).

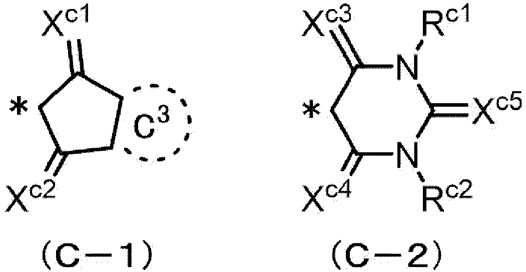

In Formula (C-1), X^{c1} and X^{c2} each independently represent a sulfur atom or an oxygen atom.

It is preferable that at least one of X^{c1} or x^{c2} is an oxygen atom, and it is more preferable that both X^{c1} and x^{c2} are oxygen atoms.

In Formula (C-1), C³ represents an aromatic ring which may have a substituent.

The number of carbon atoms in the above-described aromatic ring is preferably 4 to 30, more preferably 5 to 12, and still more preferably 6 to 8. The above-described number of carbon atoms is a number including two carbon atoms specified in the formula.

The above-described aromatic ring may be monocyclic or polycyclic.

In addition, the aromatic ring may be any of an aromatic hydrocarbon ring or an aromatic heterocyclic ring, and an aromatic hydrocarbon ring is preferable.

Examples of the aromatic ring represented by C³ include the rings exemplified in the description of the aromatic ring described above.

Among these, as the aromatic ring represented by C³, a benzene ring, a naphthalene ring, an anthracene ring, or a pyrene ring is preferable, and a benzene ring is more preferable.

Examples of the substituent which may be included in the aromatic ring include the groups exemplified as the substituent W.

In Formula (C-2), x^{c3} to x^{c5} represent a sulfur atom or an oxygen atom.

It is preferable that both x^{c3} and x^{c4} are oxygen atoms, and it is more preferable that all of x^{c3}, x^{c4}, and x^{c5} are oxygen atoms.

In addition, R^{c1} and R^{c2} each independently represent a hydrogen atom or a substituent. Examples of the substituent represented by R^{c1} and R^{c2} include the groups exemplified as the substituent W, and among these, an alkyl group or a phenyl group is preferable, and an alkyl group is more preferable.

The above-described phenyl group may further have a substituent, and examples thereof include the groups exemplified as the substituent W.

A molecular weight of the specific compound is preferably 400 to 1,200, more preferably 400 to 1,000, and still more preferably 500 to 800.

In a case where the molecular weight is in the above-described range, it is presumed that sublimation temperature of the specific compound is low, and thus the quantum efficiency is excellent also in a case where the photoelectric conversion film is formed at a high speed.

From the viewpoint of stability in a case of using the specific compound as a p-type organic semiconductor and matching of energy levels between the specific compound and an n-type organic semiconductor, an ionization potential of the specific compound in a single film is preferably -6.0 to -5.0 eV.

A maximal absorption wavelength of the specific compound is preferably in a wavelength range of 400 to 600 nm, and more preferably in a wavelength range of 400 to 500 nm.

The above-described maximal absorption wavelength is a value measured in a solution state (solvent: chloroform) by adjusting the absorption spectrum of the specific compound to a concentration such that the light absorbance is 0.5 to 1.0. However, in a case where the specific compound is not soluble in chloroform, a value measured by using the specific compound in which the specific compound is vapor-deposited and formed into a film state is defined as the maximal absorption wavelength of the specific compound.

The specific compound is particularly useful as a material of a photoelectric conversion film used for an imaging element, an optical sensor, or a photoelectric cell. The specific compound usually functions as a coloring agent in the photoelectric conversion film. In addition, the specific compound can also be used as a coloring material, a liquid crystal material, an organic semiconductor material, a charge transport material, a pharmaceutical material, and a fluorescent diagnostic material.

Hereinafter, specific examples of D (the group represented by any of Formula (2) to Formula (5)) in the compound represented by Formula (1) (specific compound) are shown below, but the present invention is not limited thereto.

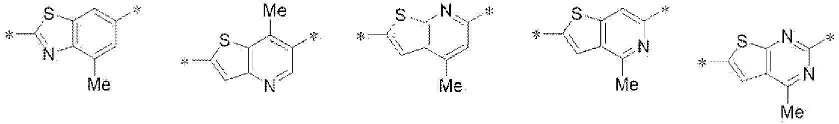

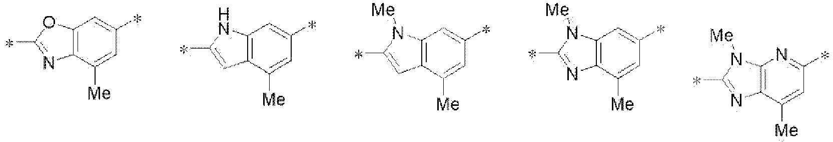

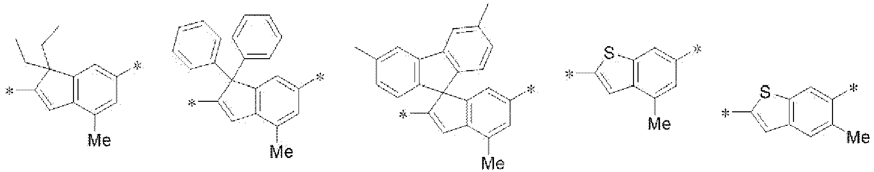

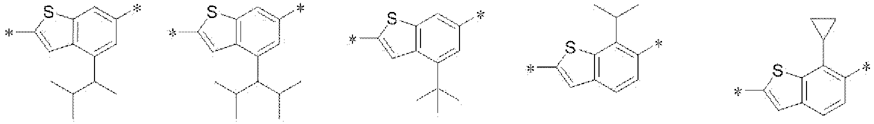

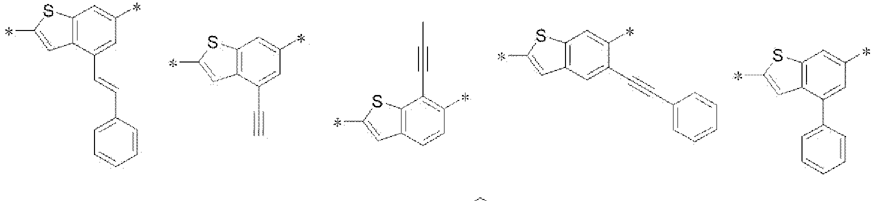

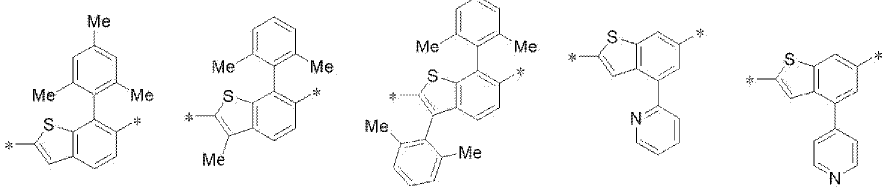

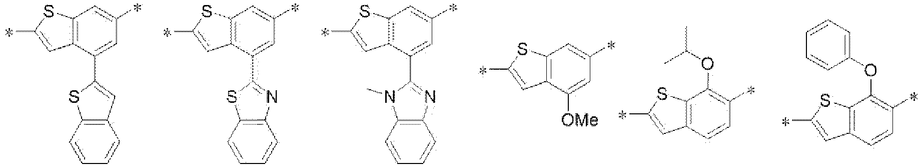

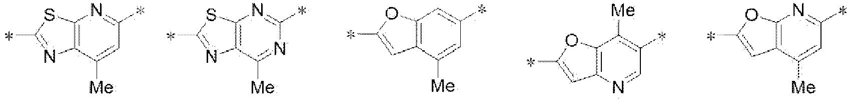

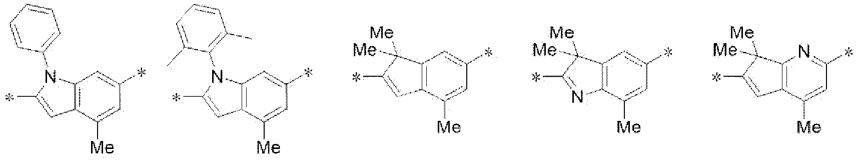

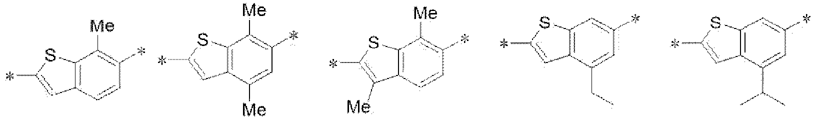

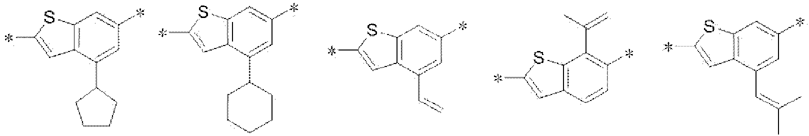

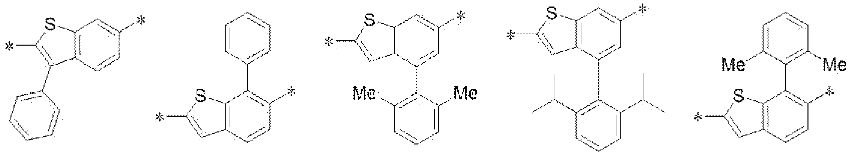

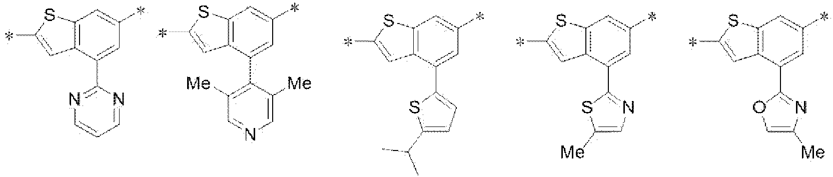

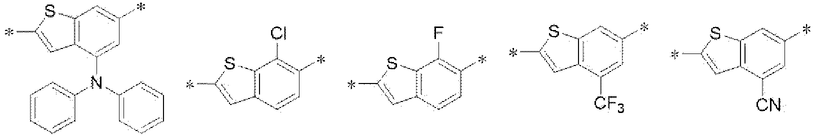

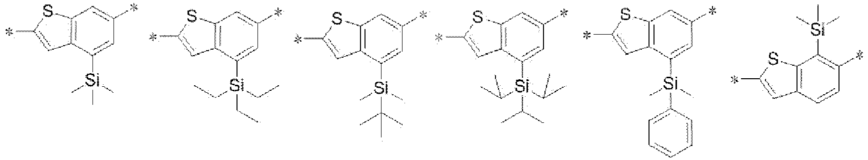

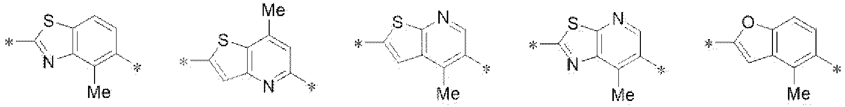

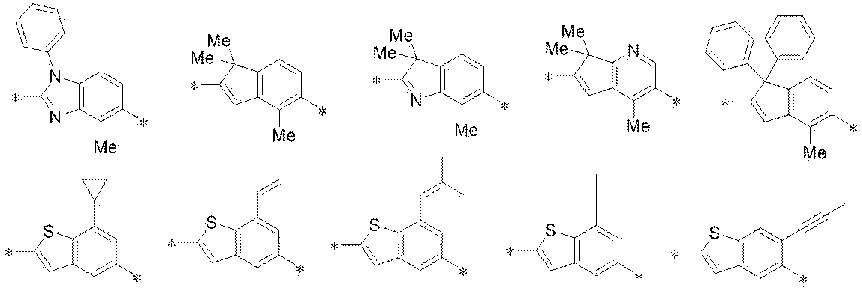

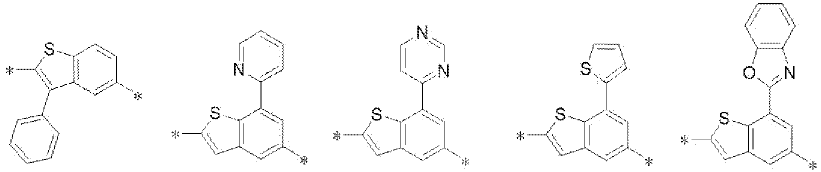

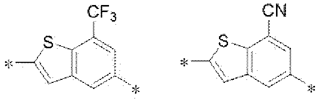

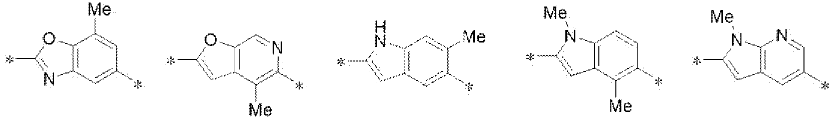

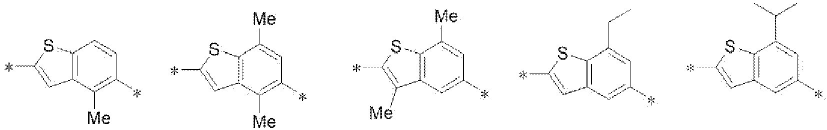

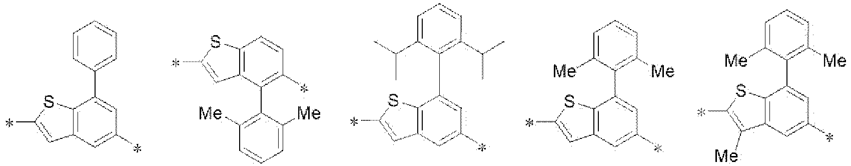

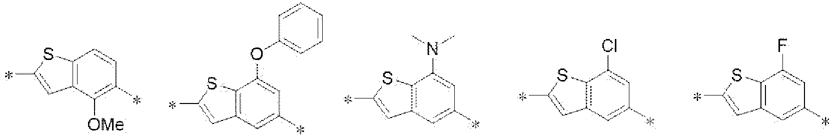

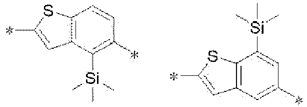

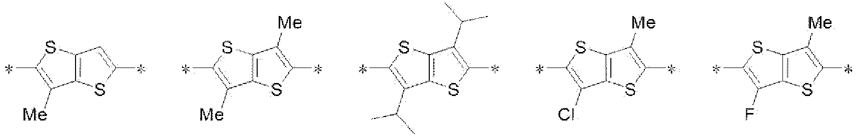

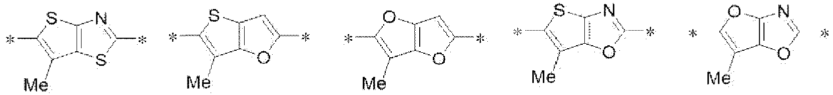

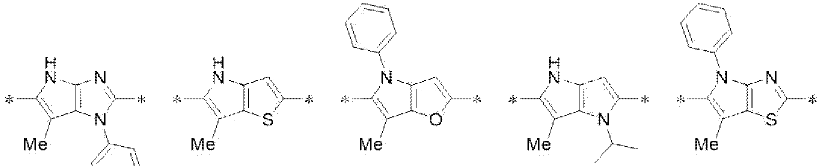

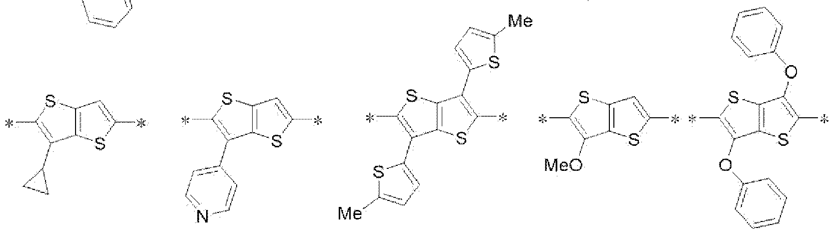

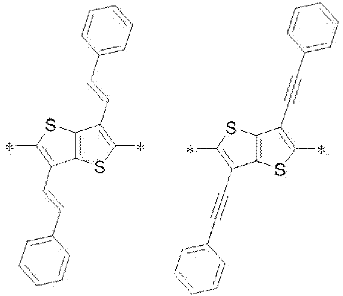

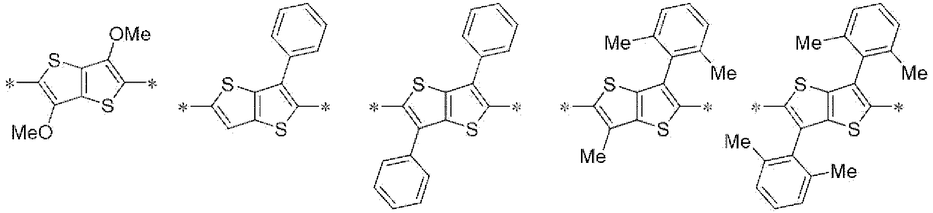

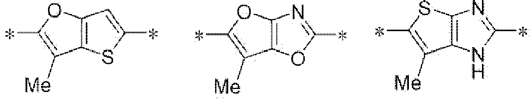

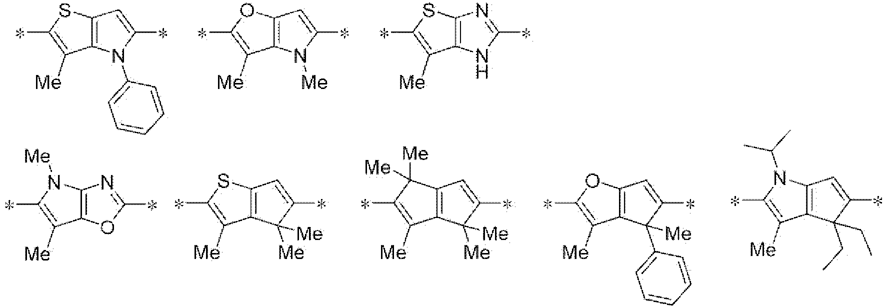

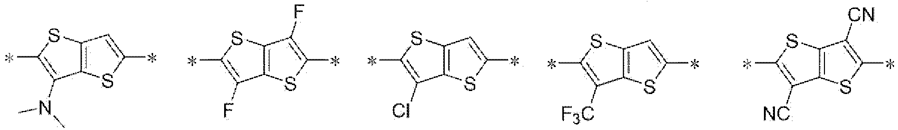

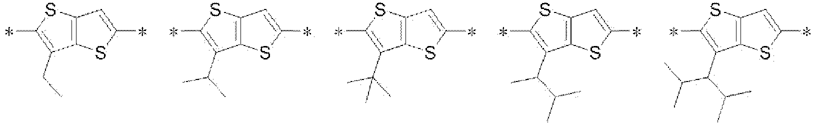

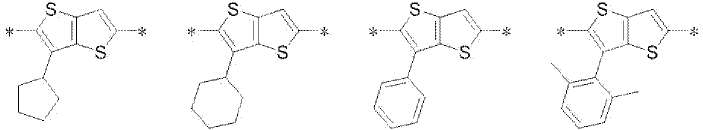

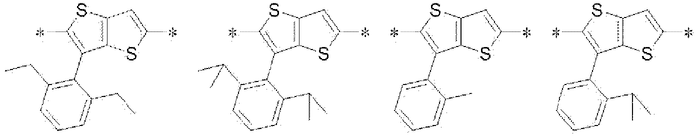

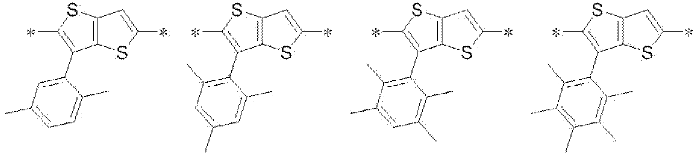

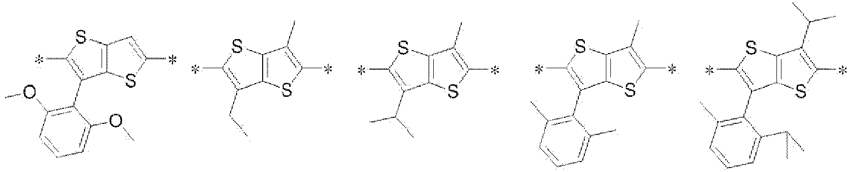

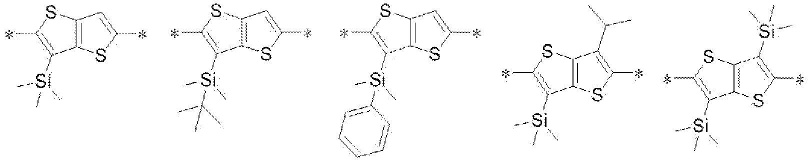

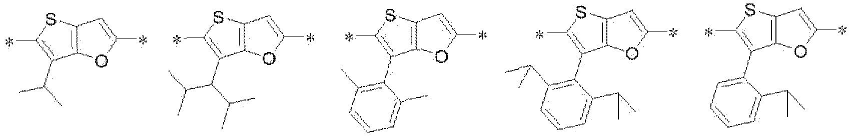

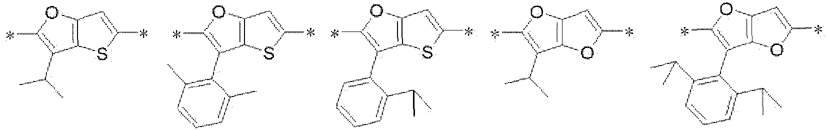

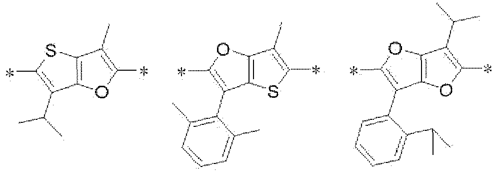

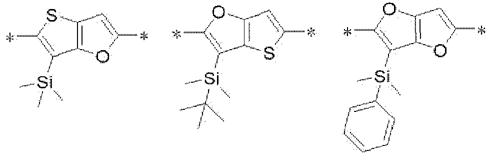

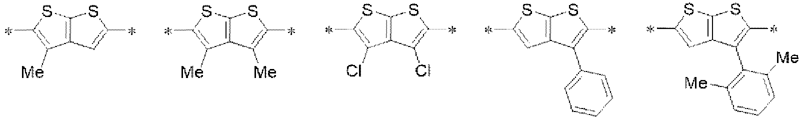

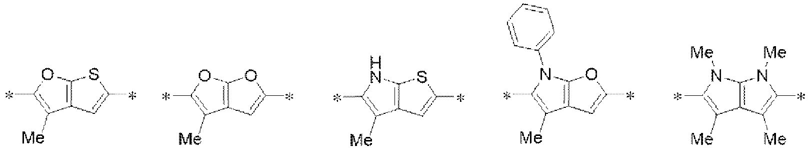

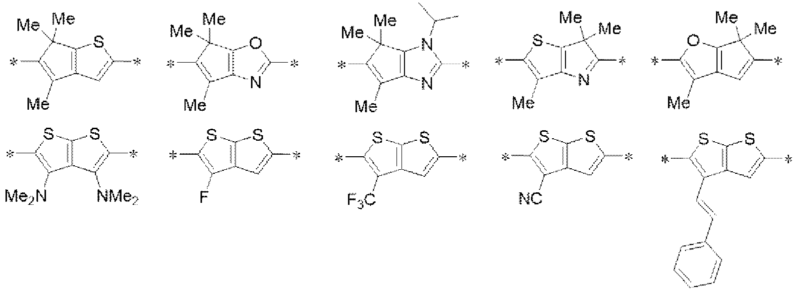

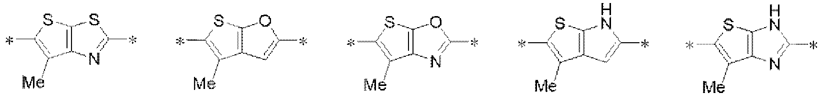

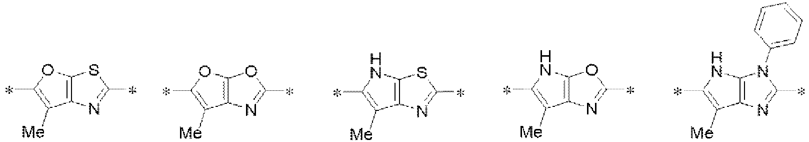

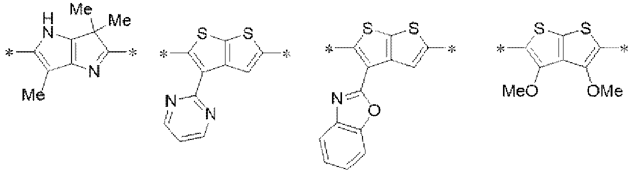

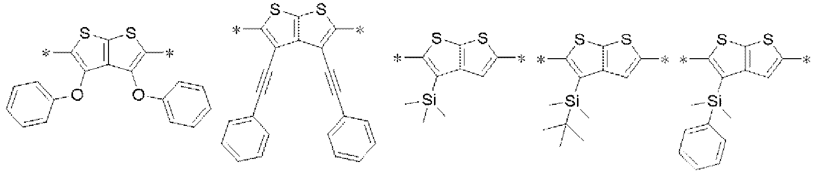

In addition, specific examples of A¹ and A² (group represented by Formula (A-1)) in the compound represented by Formula (1) (specific compound) are shown below, but the present invention is not limited thereto.

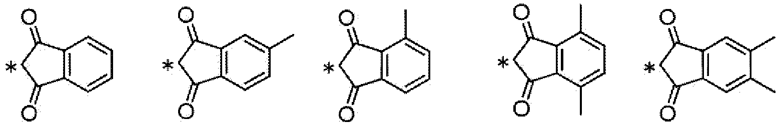

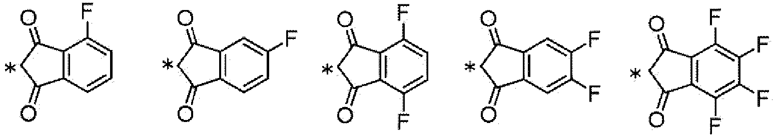

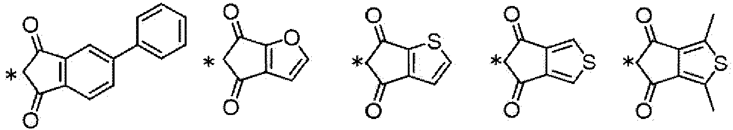

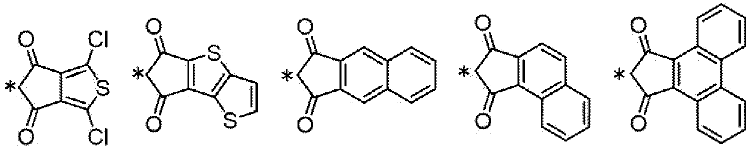

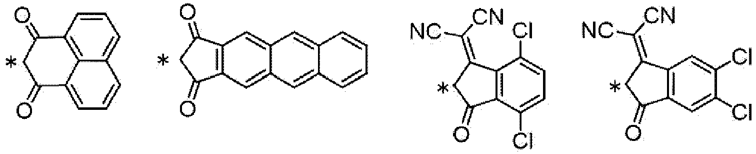

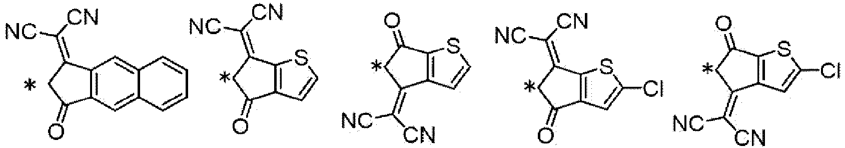

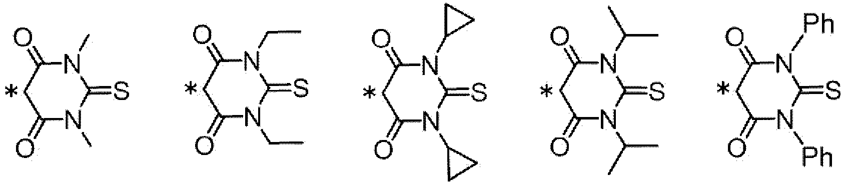

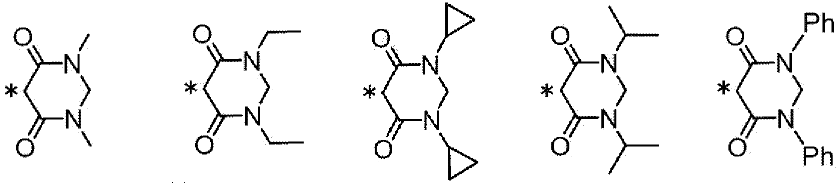

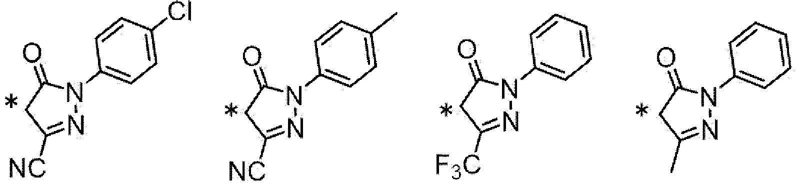

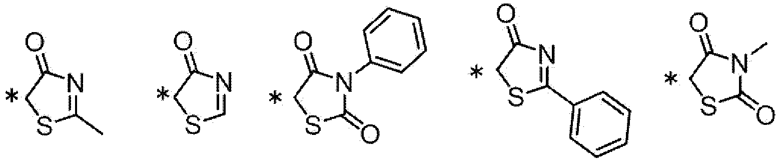

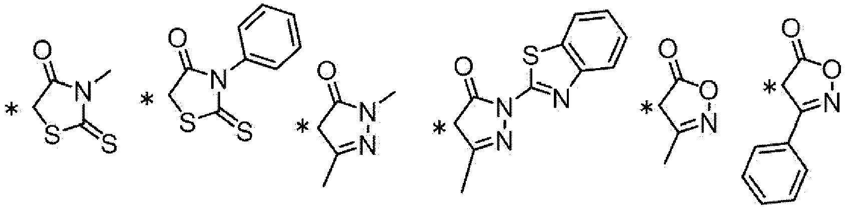

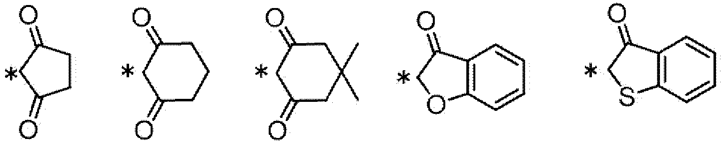

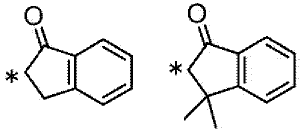

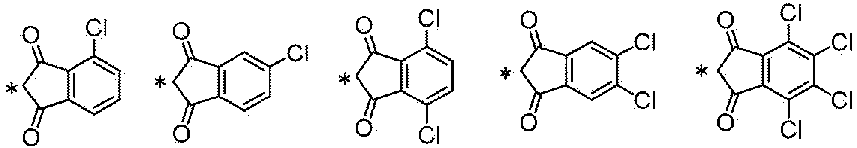

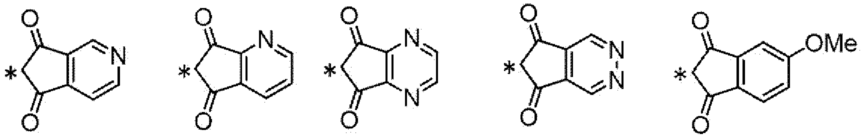

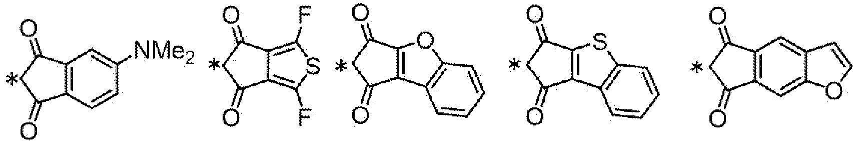

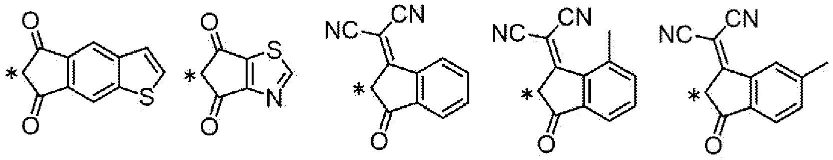

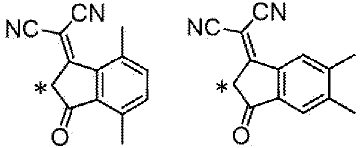

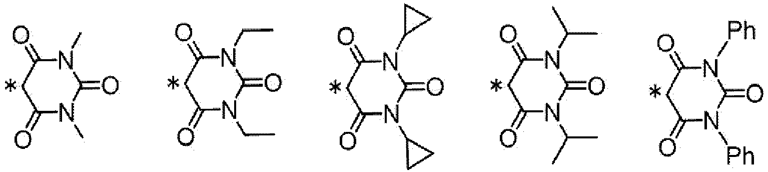

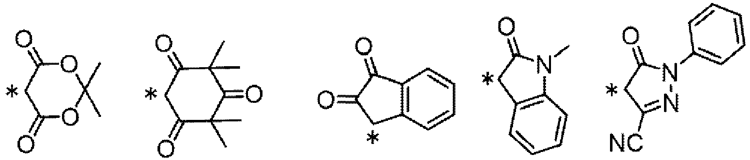

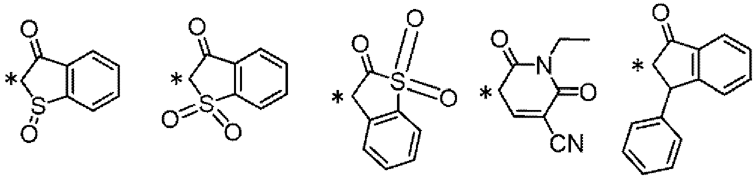

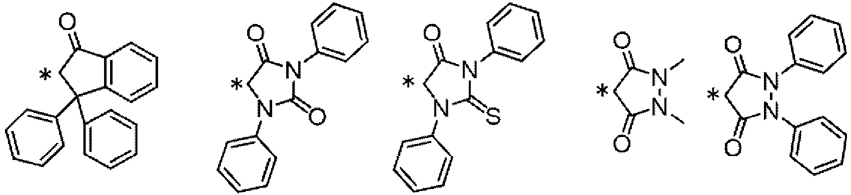

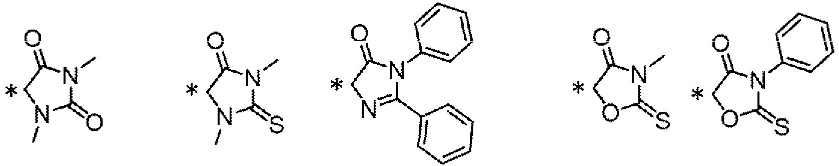

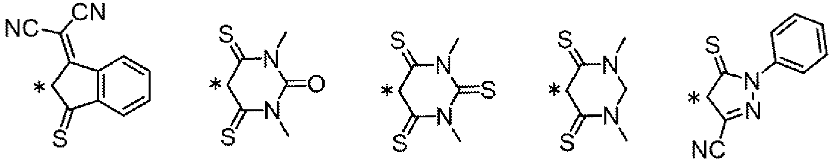

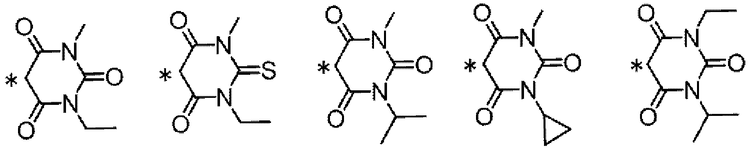

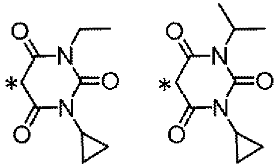

The specific compound may be purified as necessary.

Examples of a purification method of the specific compound include sublimation purification, purification using silica gel column chromatography, purification using gel permeation chromatography, re-slurry washing, re-purification by re-precipitation, purification using an adsorbent such as activated carbon, and recrystallization purification.

A content of the specific compound in the photoelectric conversion film (= Film thickness of specific compound in terms of single layer/Film thickness of photoelectric conversion film × 100) is not particularly limited, but is preferably 15% to 75% by volume, more preferably 20% to 60% by volume, and still more preferably 20% to 50% by volume.

The specific compound may be used alone or in combination of two or more types thereof. In a case where two or more types thereof are used, it is preferable that the total amount thereof is within the above-described range.

### <n-type organic semiconductor>

The photoelectric conversion film preferably contains an n-type organic semiconductor, in addition to the specific compound.

The n-type organic semiconductor is a compound different from the above-described specific compound.

The n-type organic semiconductor is an acceptor-type organic semiconductor material (compound), and refers to an organic compound having a property of easily accepting an electron. That is, the n-type organic semiconductor refers to an organic compound having a larger electron affinity in a case where two organic compounds used in contact with each other. That is, any organic compound having an electron accepting property can be used as the acceptor-type organic semiconductor.

Examples of the n-type organic semiconductor include fullerenes selected from the group consisting of a fullerene and derivatives thereof; fused aromatic carbocyclic compounds (for example, a naphthalene derivative, an anthracene derivative, a phenanthrene derivative, a tetracene derivative, a pyrene derivative, a perylene derivative, a fluoranthene derivative, and the like); heterocyclic compounds with a 5- to 7-membered ring having at least one selected from the group consisting of a nitrogen atom, an oxygen atom, and a sulfur atom (for example, pyridine, pyrazine, pyrimidine, pyridazine, triazine, quinoline, quinoxaline, quinazoline, phthalazine, cinnoline, isoquinoline, pteridine, acridine, phenazine, phenanthroline, tetrazole, pyrazole, imidazole, thiazole, and the like); polyarylene compounds; fluorene compounds; cyclopentadiene compounds; silyl compounds; 1,4,5,8-naphthalenetetracarboxylic acid anhydride; 1,4,5,8-naphthalenetetracarboxylic acid anhydride imide derivatives and oxadiazole derivatives; anthraquinodimethane derivatives; diphenylquinone derivatives; bathocuproine, bathophenanthroline, and derivatives thereof; triazole compounds; distyrylarylene derivatives; metal complexes having a nitrogen-containing heterocyclic compound as a ligand; silole compounds; and compounds described in paragraphs [0056] and [0057] of JP2006-100767A.

The n-type organic semiconductor (compound) is preferably fullerenes selected from the group consisting of a fullerene and derivatives thereof.

Examples of the fullerene include a fullerene C60, a fullerene C70, a fullerene C76, a fullerene C78, a fullerene C80, a fullerene C82, a fullerene C84, a fullerene C90, a fullerene C96, a fullerene C240, a fullerene C540, and a mixed fullerene.

Examples of the derivatives of fullerene include compounds in which a substituent is added to the above-described fullerenes. The above-described substituent is preferably an alkyl group, an aryl group, or a heterocyclic group. As the derivatives of fullerene, compounds described in JP2007-123707A are preferable.

The n-type organic semiconductor may be an organic coloring agent.

Examples of the organic coloring agent include a cyanine coloring agent, a styryl coloring agent, a hemicyanine coloring agent, a merocyanine coloring agent (including zeromethine merocyanine (simple merocyanine)), a rhodacyanine coloring agent, an allopolar coloring agent, an oxonol coloring agent, a hemioxonol coloring agent, a squarylium coloring agent, a croconium coloring agent, an azamethine coloring agent, a coumarin coloring agent, an arylidene coloring agent, an anthraquinone coloring agent, a triphenylmethane coloring agent, an azo coloring agent, an azomethine coloring agent, a metallocene coloring agent, a fluorenone coloring agent, a flugide coloring agent, a perylene coloring agent, a phenazine coloring agent, a phenothiazine coloring agent, a quinone coloring agent, a diphenylmethane coloring agent, a polyene coloring agent, an acridine coloring agent, an acridinone coloring agent, a diphenylamine coloring agent, a quinophthalone coloring agent, a phenoxazine coloring agent, a phthaloperylene coloring agent, a dioxane coloring agent, a porphyrin coloring agent, a chlorophyll coloring agent, a phthalocyanine coloring agent, a subphthalocyanine coloring agent, and a metal complex coloring agent.

A molecular weight of the n-type organic semiconductor is preferably 200 to 1,200 and more preferably 200 to 900.

A maximal absorption wavelength of the n-type organic semiconductor is preferably in a wavelength of 400 nm or less or in a wavelength range of 500 to 600 nm.

It is preferable that the photoelectric conversion film has a bulk hetero structure formed in a state in which the specific compound and the n-type organic semiconductor are mixed with each other. The bulk hetero structure refers to a layer in which the specific compound and the n-type organic semiconductor are mixed and dispersed in the photoelectric conversion film. The photoelectric conversion film having the bulk hetero structure can be formed by a wet method or a dry method. The bulk hetero structure is described in detail in paragraphs [0013] and [0014] of JP2005-303266A.

A difference in electron affinity between the specific compound and the n-type organic semiconductor is preferably 0.1 eV or more.

The n-type organic semiconductor may be used alone or in combination of two or more types thereof.

In a case where the photoelectric conversion film contains the n-type organic semiconductor, a content of the n-type organic semiconductor in the photoelectric conversion film (Film thickness of n-type organic semiconductor in terms of single layer/Film thickness of photoelectric conversion film × 100) is preferably 15% to 75% by volume, more preferably 20% to 60% by volume, and still more preferably 20% to 50% by volume.

In a case where the n-type organic semiconductor includes fullerenes, a content of the fullerenes to the total content of the n-type organic semiconductor (Film thickness of fullerenes in terms of single layer/Total film thickness of n-type organic semiconductors in terms of single layer × 100) is preferably 50% to 100% by volume, and more preferably 80% to 100% by volume. The fullerenes may be used alone or in combination of two or more types thereof.

From the viewpoint of response speed of the photoelectric conversion element, the content of the specific compound to the total content of the specific compound and the n-type organic semiconductor (Film thickness of specific compound in terms of single layer/(Film thickness of specific compound in terms of single layer + Film thickness of n-type organic semiconductor in terms of single layer) × 100) is preferably 20% to 80% by volume, and more preferably 40% to 80% by volume.

In a case where the photoelectric conversion film contains the n-type organic semiconductor and a p-type organic semiconductor, the content of the specific compound (Film thickness of specific compound in terms of single layer/(Film thickness of specific compound in terms of single layer + Film thickness of n-type organic semiconductor in terms of single layer + Film thickness of p-type organic semiconductor in terms of single layer) × 100) is preferably 15% to 75% by volume, and more preferably 30% to 75% by volume.

It is preferable that the photoelectric conversion film substantially contains the specific compound, the n-type organic semiconductor, and the p-type organic semiconductor contained as desired. The term "substantially" indicates that the total content of the specific compound, the n-type organic semiconductor, and the p-type organic semiconductor is 90% to 100% by volume, preferably 95% to 100% by volume, and more preferably 99% to 100% by volume with respect to the total mass of the photoelectric conversion film.

### <p-type organic semiconductor>

The photoelectric conversion film preferably contains a p-type organic semiconductor in addition to the above-described specific compound.

The p-type organic semiconductor is a compound different from the above-described specific compound.

The p-type organic semiconductor is a donor-type organic semiconductor material (compound), and refers to an organic compound having a property of easily donating an electron. That is, the p-type organic semiconductor refers to an organic compound having a smaller ionization potential in a case where two organic compounds are used in contact with each other.

The p-type organic semiconductor may be used alone or in combination of two or more types thereof.

Examples of the p-type organic semiconductor include triarylamine compounds (for example, N,N'-diphenyl-N,N'-bis(3-methylphenyl)-(1,1'-biphenyl)-4,4'-diamine (TPD), 4,4'-bis[N-(naphthyl)-N-Phenyl-amino] biphenyl (α-NPD), compounds described in paragraphs [0128] to [0148] of JP2011-228614A, compounds described in paragraphs [0052] to [0063] of JP2011-176259A, compounds described in paragraphs [0119] to [0158] of JP2011-225544A, compounds described in paragraphs [0044] to [0051] of JP2015-153910A, compounds described in paragraphs [0086] to [0090] of JP2012-094660A, and the like), pyrazoline compounds, styrylamine compounds, hydrazone compounds, polysilane compounds, thiophene compounds (for example, a thienothiophene derivative, a dibenzothiophene derivative, a benzodithiophene derivative, a dithienothiophene derivative, a [1]benzothieno[3,2-b]thiophene (BTBT) derivative, a thieno[3,2-f:4,5-f']bis[1]benzothiophene (TBBT) derivative, compounds described in paragraphs [0031] to [0036] of JP2018-014474A, compounds described in paragraphs [0043] to [0045] of WO2016/194630A, compounds described in paragraphs [0025] to [0037], and [0099] to [0109] of WO2017/159684A, compounds described in paragraphs [0029] to [0034] of JP2017-076766A, compounds described in paragraphs [0015] to [0025] of WO2018/207722A, compounds described in paragraphs [0045] to [0053] of JP2019-054228A, compounds described in paragraphs [0045] to [0055] of WO2019/058995A, compounds described in paragraphs [0063] to [0089] of WO2019/081416A, compounds described in paragraphs [0033] to [0036] of JP2019-080052A, compounds described in paragraphs [0044] to [0054] of WO2019/054125A, compounds described in paragraphs [0041] to [0046] of WO2019/093188A, compounds described in paragraphs [0034] to [0037] of JP2019-050398A, compounds described in paragraphs [0033] to [0036] of JP2018-206878A, compounds described n paragraph [0038] of JP2018-190755A, compounds described in paragraphs [0019] to [0021] of JP2018-026559A, compounds described in paragraphs [0031] to [0056] of JP2018-170487A, compounds described in paragraphs [0036] to [0041] of JP2018-078270A, compounds described in paragraphs [0055] to [0082] of JP2018-166200A, compounds described in paragraphs [0041] to [0050] of JP2018-113425A, compounds described in paragraphs [0044] to [0048] of JP2018-085430A, compounds described in paragraphs [0041] to [0045] of JP2018-056546A, compounds described in paragraphs [0042] to [0049] of JP2018-046267A, compounds described in paragraphs [0031] to [0036] of JP2018-014474A, compounds described in paragraphs [0036] to [0046] of WO2018/016465A, compounds described in paragraphs [0045] to [0048] of JP2020-010024A, and the like), a cyanine compound, an oxonol compound, a polyamine compound, an indole compound, a pyrrole compound, a pyrazole compound, a polyarylene compound, a fused aromatic carbocyclic compound (for example, a naphthalene derivative, an anthracene derivative, a phenanthrene derivative, a tetracene derivative, a pentacene derivative, a pyrene derivative, a perylene derivative, a fluoranthene derivative, and the like), a porphyrin compound, a phthalocyanine compound, a triazole compound, an oxadiazole compound, an imidazole compound, a polyarylalkane compound, a pyrazolone compound, an amino-substituted chalcone compound, an oxazole compound, a fluorenone compound, a silazane compound, and a metal complex having a nitrogen-containing heterocyclic compound as a ligand.

In addition, examples of the p-type organic semiconductor also include a benzoxazole compound (for example, compounds described in Figs. 3 to 7 of JP2022-123944A), a dicarbazole compound (for example, compounds described in Figs. 2 to 5 of JP2022-122839A), a benzoquinazoline compound (for example, compounds described in paragraphs [0053] to [0056] of JP2022-120323A), an azine compound (for example, compounds described in paragraphs [0041] and [0042] of JP2022-120273A), compounds described in Figs. 2 to 10 of JP2022-115832A, an indolotriphenylene compound (for example, compounds described in paragraphs [0065] to [0072] of JP2022-108268A), an indolocarbazole compound (for example, compounds described in paragraphs [0052] to [0073] of JP2023-005703A and paragraph [0028] of JP2022-100258A), a triscarbazolylphenyl compound (for example, compounds described in paragraphs [0038] to [0040] of JP2022-181226A), compounds described in paragraphs [0070] to [0082] of JP2022-027575A, and compounds described in paragraphs [0051] to [0064] of JP2021-163968A.

Examples of the p-type organic semiconductor also include compounds having an ionization potential smaller than that of the n-type organic semiconductor, and in a case where this condition is satisfied, the organic coloring agent exemplified as the n-type organic semiconductor can be used.

Compounds which can be used as the p-type organic semiconductor compound are exemplified below.

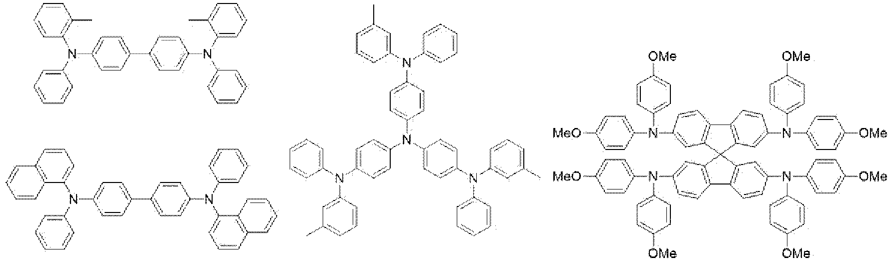

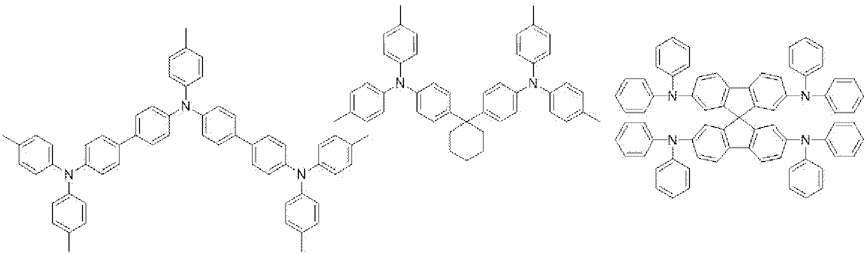

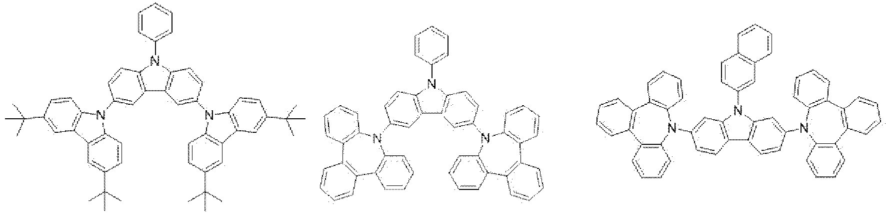

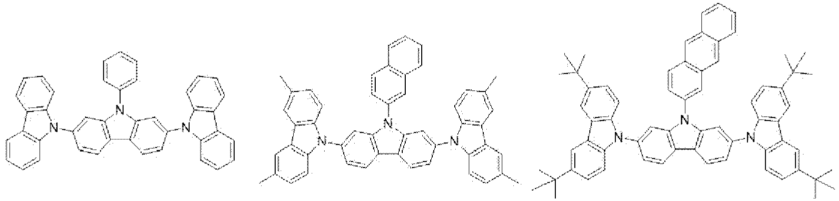

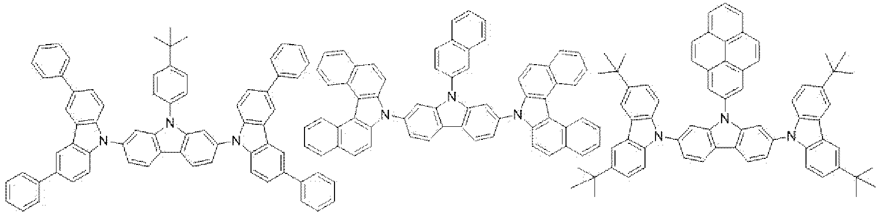

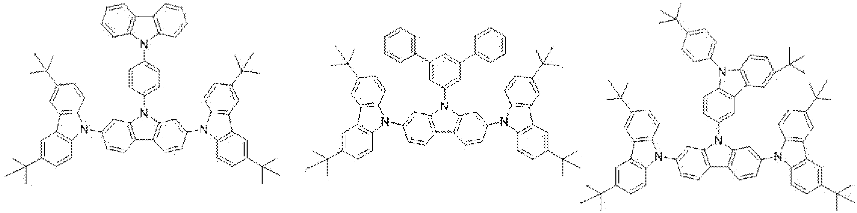

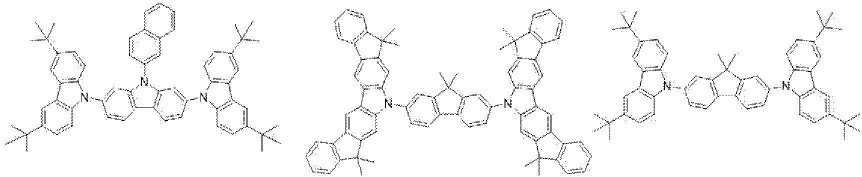

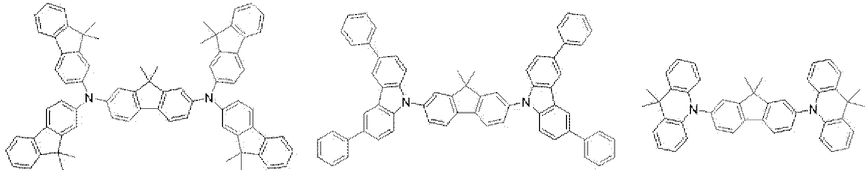

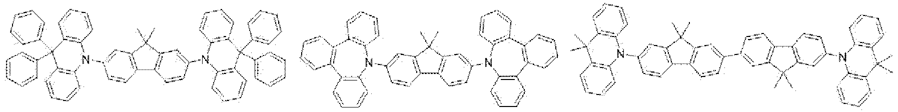

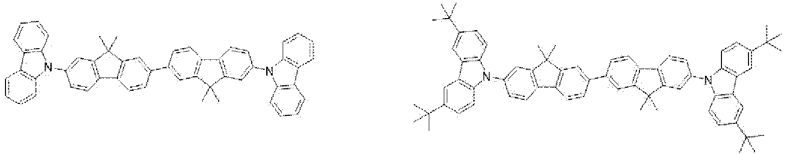

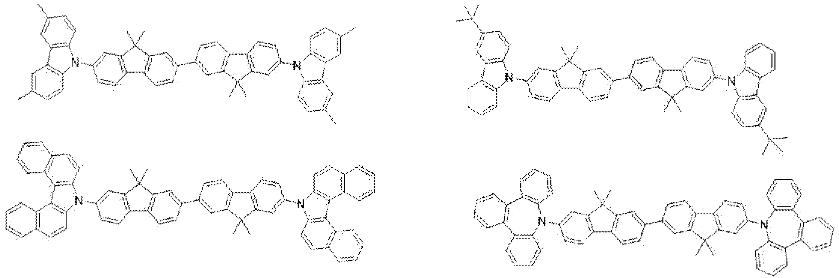

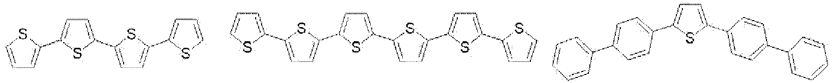

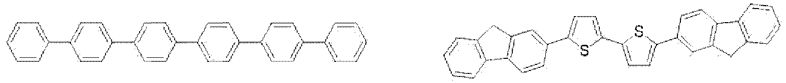

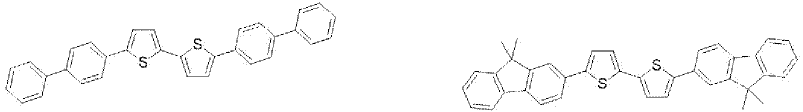

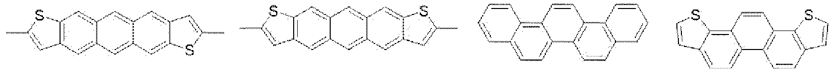

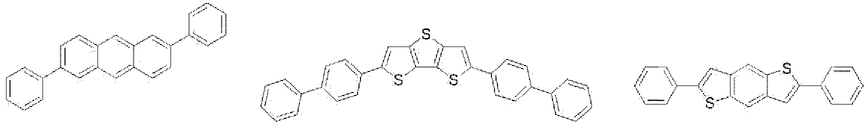

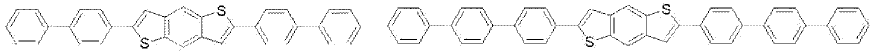

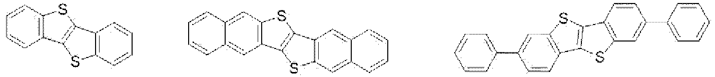

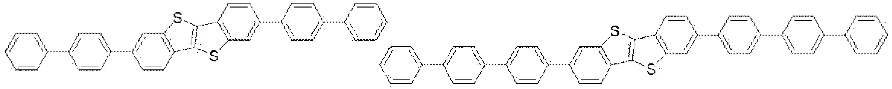

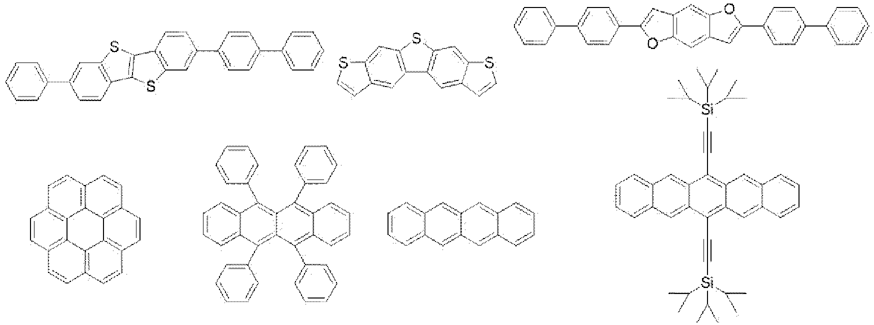

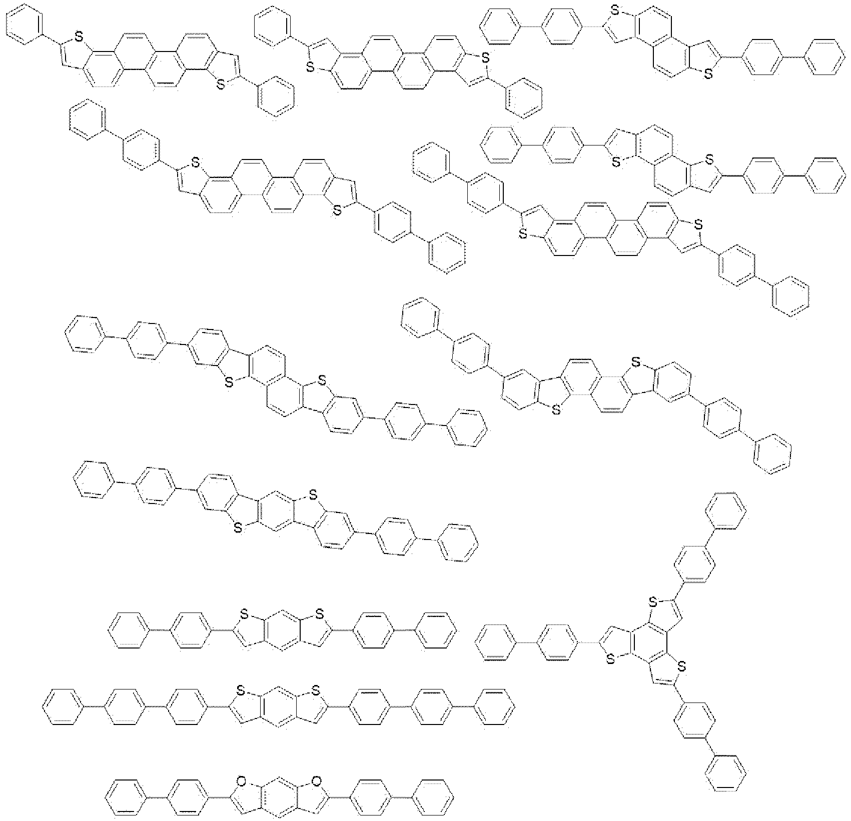

A difference in ionization potential between the specific compound and the p-type organic semiconductor is preferably 0.1 eV or more.

The p-type semiconductor material may be used alone or in combination of two or more types thereof.

In a case where the photoelectric conversion film contains the p-type organic semiconductor, a content of the p-type organic semiconductor in the photoelectric conversion film (Film thickness of p-type organic semiconductor in terms of single layer/Film thickness of photoelectric conversion film × 100) is preferably 15% to 75% by volume, more preferably 20% to 60% by volume, and still more preferably 25% to 50% by volume.

The photoelectric conversion film containing the specific compound is a non-luminescent film, and has a feature different from an organic light emitting diode (OLED). The non-luminescent film refers to a film having a light emission quantum efficiency of 1% or less, and the light emission quantum efficiency is preferably 0.5% or less and more preferably 0.1% or less. The lower limit thereof is often 0% or more.

### <Coloring agent>

The photoelectric conversion film may contain a coloring agent in addition to the above-described specific compound.

The coloring agent is a compound different from the above-described specific compound.

As the coloring agent, an organic coloring agent is preferable.

Examples of the organic coloring agent include a cyanine coloring agent, a styryl coloring agent, a hemicyanine coloring agent, a merocyanine coloring agent (including zeromethine merocyanine (simple merocyanine)), a rhodacyanine coloring agent, an allopolar coloring agent, an oxonol coloring agent, a hemioxonol coloring agent, a squarylium coloring agent, a croconium coloring agent, an azamethine coloring agent, a coumarin coloring agent, an arylidene coloring agent, an anthraquinone coloring agent, a triphenylmethane coloring agent, an azo coloring agent, an azomethine coloring agent, a metallocene coloring agent, a fluorenone coloring agent, a flugide coloring agent, a perylene coloring agent, a phenazine coloring agent, a phenothiazine coloring agent, a quinone coloring agent, a diphenylmethane coloring agent, a polyene coloring agent, an acridine coloring agent, an acridinone coloring agent, a diphenylamine coloring agent, a quinophthalone coloring agent, a phenoxazine coloring agent, a phthaloperylene coloring agent, a dioxane coloring agent, a porphyrin coloring agent, a chlorophyll coloring agent, a phthalocyanine coloring agent, a subphthalocyanine coloring agent, a metal complex coloring agent, an imidazoquinoxaline coloring agent described in WO2020/013246A, WO2022/168856A, JP2023-010305A, and JP2023-010299A, an acceptor-donor-acceptor type coloring agent in which two acidic nuclei are bonded to a donor, and a donor-acceptor-donor type coloring agent in which two donors are bonded to an acceptor.

Among these, the organic coloring agent is preferably a cyanine coloring agent, an imidazoquinoxaline coloring agent, or an acceptor-donor-acceptor type coloring agent; and more preferably an imidazoquinoxaline coloring agent or an acceptor-donor-acceptor type coloring agent.

A maximal absorption wavelength of the coloring agent is preferably in the visible light region, more preferably in a wavelength range of 400 to 650 nm, and still more preferably in a wavelength range of 450 to 650 nm.

The coloring agent may be used alone or in combination of two or more types thereof.

A content of the coloring agent with respect to the total content of the specific compound and the coloring agent in the photoelectric conversion film (= (Film thickness of coloring agent in terms of single layer/(Film thickness of specific compound in terms of single layer + Film thickness of coloring agent in terms of single layer) × 100)) is preferably 15% to 75% by volume, more preferably 20% to 60% by volume, and still more preferably 20% to 50% by volume.

### <Film formation method>

Examples of a film formation method of the above-described photoelectric conversion film include a dry film formation method.

Examples of the dry film formation method include a physical vapor deposition method such as a vapor deposition method (particularly, a vacuum vapor deposition method), a sputtering method, an ion plating method, and a molecular beam epitaxy (MBE) method, and a chemical vapor deposition (CVD) method such as plasma polymerization; and a vacuum vapor deposition method is preferable. In a case where the photoelectric conversion film is formed by the vacuum vapor deposition method, manufacturing conditions such as a degree of vacuum and a vapor deposition temperature can be set according to the conventional method.

A film thickness of the photoelectric conversion film is preferably 10 to 1,000 nm, more preferably 50 to 800 nm, and still more preferably 50 to 500 nm.

### [Electrode]

The photoelectric conversion element preferably includes an electrode.

The electrode (the upper electrode (transparent conductive film) 15 and the lower electrode (conductive film) 11) contains a conductive material. Examples of the conductive material include metals, alloys, metal oxides, electrically conductive compounds, and mixtures thereof.

Since light is incident through the upper electrode 15, the upper electrode 15 is preferably transparent to light to be detected. Examples of a material constituting the upper electrode 15 include conductive metal oxides such as tin oxide doped with antimony, fluorine, or the like (antimony tin oxide (ATO) and fluorine doped tin oxide (FTO)), tin oxide, zinc oxide, indium oxide, indium tin oxide (ITO), and indium zinc oxide (IZO); metal thin films such as gold, silver, chromium, and nickel; mixtures or laminates of these metals and the conductive metal oxides; and organic conductive materials such as polyaniline, polythiophene, and polypyrrole; and nano carbon materials such as carbon nanotubes and graphene. From the viewpoint of high conductivity and transparency, conductive metal oxides are preferable.

In general, in a case where the conductive film is thinner than a certain range, a resistance value rapidly increases in many cases. In a solid-state imaging element in which the photoelectric conversion element according to the present embodiment is incorporated, a sheet resistance may be 100 to 10,000 Q/o, and a degree of freedom of the film thickness range which can be reduced is large.

In addition, as the film thickness of the upper electrode (transparent conductive film) 15 is thinner, the amount of light which is absorbed in the upper electrode is smaller, and thus light transmittance usually increases. The increase in the light transmittance causes an increase in light absorbance in the photoelectric conversion film and an increase in the photoelectric conversion ability, which is preferable. Considering suppression of leakage current, increase in resistance value of the thin film, and increase in transmittance accompanied by the thinning, the thickness of the upper electrode 15 is preferably 5 to 100 nm, and more preferably 5 to 20 nm.

There is a case where the lower electrode 11 has transparency or an opposite case where the lower electrode does not have transparency and reflects light, depending on use. Examples of a material constituting the lower electrode 11 include conductive metal oxides such as tin oxide doped with antimony, fluorine, or the like (ATO and FTO), tin oxide, zinc oxide, indium oxide, indium tin oxide (ITO), and indium zinc oxide (IZO); metals such as gold, silver, chromium, nickel, titanium, tungsten, and aluminum; conductive compounds such as oxides or nitrides of these metals (for example, titanium nitride (TiN)); mixtures or laminates of these metals and conductive metal oxides; organic conductive materials such as polyaniline, polythiophene, and polypyrrole; and carbon materials such as carbon nanotubes and graphene.

A method of forming the electrode can be appropriately selected in accordance with the electrode material. Specific examples thereof include a wet method such as a printing method and a coating method; a physical method such as a vacuum vapor deposition method, a sputtering method, and an ion plating method; and a chemical method such as a CVD method and a plasma CVD method.

In a case where the electrode material is ITO, examples thereof include an electron beam method, a sputtering method, a resistance thermal vapor deposition method, a chemical reaction method (such as a sol-gel method), and a coating method with a dispersion of indium tin oxide.

### [Charge blocking film: electron blocking film and hole blocking film]

It is preferable that the photoelectric conversion element includes one or more interlayers between the conductive film and the transparent conductive film, in addition to the photoelectric conversion film.

Examples of the above-described interlayer include a charge blocking film. In a case where the photoelectric conversion element includes the film, the characteristics (such as the quantum efficiency and the response speed) of the photoelectric conversion element to be obtained are more excellent. Examples of the charge blocking film include an electron blocking film and a hole blocking film.

### [Electron blocking film]

The electron blocking film is a donor-type organic semiconductor material (compound), and the above-described p-type organic semiconductor can be used.

In addition, a polymer material can also be used in the electron blocking film.

Specific examples of the polymer material include a polymer such as phenylenevinylene, fluorene, carbazole, indole, pyrene, pyrrole, picoline, thiophene, acetylene, and diacetylene, and derivatives thereof.

The electron blocking film may be configured by a plurality of films.

The electron blocking film may be formed of an inorganic material. In general, since an inorganic material has a dielectric constant larger than that of an organic material, in a case where the inorganic material is used in the electron blocking film, a large voltage is applied to the photoelectric conversion film. Therefore, the quantum efficiency increases. Examples of the inorganic material which can be used in the electron blocking film include calcium oxide, chromium oxide, copper chromium oxide, manganese oxide, cobalt oxide, nickel oxide, copper oxide, copper gallium oxide, copper strontium oxide, niobium oxide, molybdenum oxide, copper indium oxide, silver indium oxide, and iridium oxide.

### [Hole blocking film]

The hole blocking film is an acceptor-type organic semiconductor material (compound), and the above-described n-type organic semiconductor described above can be used.

The hole blocking film may be configured by a plurality of films.

Examples of a method of manufacturing the charge blocking film include a dry film formation method and a wet film formation method. Examples of the dry film formation method include a vapor deposition method and a sputtering method. The vapor deposition method may be a physical vapor deposition (PVD) method or a chemical vapor deposition (CVD) method, and a physical vapor deposition method such as a vacuum vapor deposition method is preferable. Examples of the wet film formation method include an ink jet method, a spray method, a nozzle printing method, a spin coating method, a dip coating method, a casting method, a die coating method, a roll coating method, a bar coating method, and a gravure coating method; and an inkjet method is preferable from the viewpoint of high-precision patterning.

Each film thickness of the charge blocking films (the electron blocking film and the hole blocking film) is preferably 3 to 200 nm, more preferably 5 to 100 nm, and still more preferably 5 to 30 nm.

### [Substrate]

The photoelectric conversion element may further include a substrate.

Examples of the substrate include a semiconductor substrate, a glass substrate, and a plastic substrate.

As a position of the substrate, in general, the conductive film, the photoelectric conversion film, and the transparent conductive film are laminated on the substrate in this order.

### [Sealing layer]

The photoelectric conversion element may further include a sealing layer.

The performance of the photoelectric conversion material may deteriorate significantly due to the presence of deterioration factors such as water molecules. The deterioration can be prevented by coating and sealing the entirety of the photoelectric conversion film with a sealing layer such as diamond-like carbon (DLC) and ceramics such as metal oxide, metal nitride, or metal nitride oxide, which are dense and into which water molecules do not permeate.

Examples of the sealing layer include sealing layers described in paragraphs [0210] to [0215] of JP2011-082508A, the contents of which are incorporated herein by reference.

### [Manufacturing method of photoelectric conversion element]

Examples of a manufacturing method of the photoelectric conversion element include known manufacturing methods.

Specific examples thereof include a manufacturing method of the photoelectric conversion element, which includes a step of forming the conductive film on the substrate, a step of forming the photoelectric conversion film, and a step of forming the transparent conductive film.

The manufacturing method of the photoelectric conversion element may include a step other than the above-described steps (for example, a step of forming the charge blocking film and a step of forming the sealing layer).

The method of forming each layer is as described above.

### [Imaging element]

Examples of the application of the photoelectric conversion element include an imaging element.

The imaging element is an element which converts optical information of an image into the electric signal, and is usually an element in which a plurality of photoelectric conversion elements are arranged on a matrix in the same planar form, optical signals are converted into electric signals in each photoelectric conversion element (a pixel), and the electric signals can be sequentially output to the outside of the imaging elements for each pixel. Therefore, each pixel is formed of one or more photoelectric conversion elements and one or more transistors.

A manufacturing method of the imaging element is not particularly limited, and examples thereof include a method including a step of manufacturing the above-described photoelectric conversion element.

### [Optical sensor]

Examples of another application of the photoelectric conversion element include a photoelectric cell and a optical sensor, but it is preferable that the photoelectric conversion element according to the embodiment of the present invention is used as the optical sensor. The above-described photoelectric conversion element may be used alone as the optical sensor, or may be used as a line sensor in which the photoelectric conversion elements are linearly arranged or as a two-dimensional sensor in which the photoelectric conversion elements are arranged on a plane.

### [Compound]

The present invention also includes a compound. The compound according to the embodiment of the present invention is the above-described specific compound.

### Examples

Hereinbelow, the present invention will be described in more detail with reference to Examples.

The materials, the amounts of materials used, the proportions, the treatment details, the treatment procedure, and the like shown in Examples below may be modified as appropriate as long as the modifications do not depart from the spirit of the present invention. Therefore, the scope of the present invention should not be construed as being limited to Examples shown below.

### [Materials used for producing photoelectric conversion element]

Each material used for producing a photoelectric conversion element is shown below.

### [Synthesis of compound B-8 (Synthesis Example 1 of specific compound)]

The compound B-8, which is the specific compound in the present invention, was synthesized according to the following scheme.

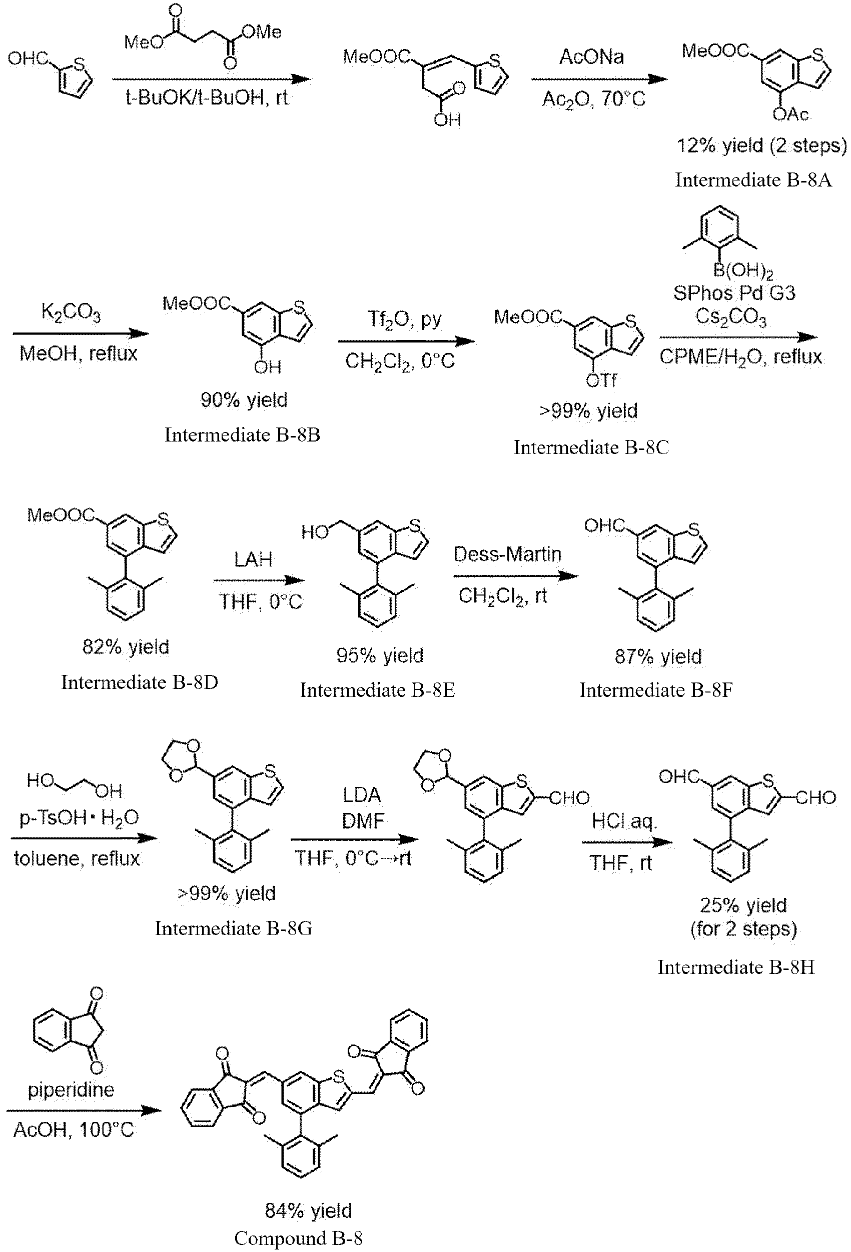

### <Synthesis of intermediate B-8A>

An intermediate B-8A was synthesized using thiophene-2-carboxyaldehyde and dimethyl succinate as starting materials, according to the method described in Asian J. Chem. 2004, 16, 823.

### <Synthesis of intermediate B-8B>

The intermediate B-8A (23.5 g) and dehydrated methanol (200 mL) were added to a 500 mL three-neck flask, potassium carbonate (26.1 g) was added thereto while stirring the mixture at room temperature in a nitrogen atmosphere, and the mixture was heated under reflux for 30 minutes.

300 mL of water, 24.6 g of ammonium chloride, and 200 mL of ethyl acetate were added to the obtained reaction solution, and liquid separation and extraction were carried out to recover the organic phase. The obtained organic phase was dried over magnesium sulfate, and the solvent was further removed. The obtained crude product was crystallized with ethyl acetate/hexane to obtain 17.5 g (yield: 90%) of an intermediate B-8B.

### <Synthesis of intermediate B-8C>

The intermediate B-8B (15.5 g) and dehydrated dichloromethane (300 mL) were added to a 1 L three-neck flask, dehydrated pyridine (7.2 mL) and trifluoromethanesulfonic acid anhydride (14.0 mL) were added thereto while stirring the mixture at 0°C in a nitrogen atmosphere, and the mixture was stirred for 30 minutes.

300 mL of saturated saline was added to the obtained reaction solution, and liquid separation and extraction were carried out to recover the organic phase. The obtained organic phase was dried over magnesium sulfate, and the solvent was further removed. The obtained crude product was purified by silica gel column chromatography (eluent: hexane/ethyl acetate = 85/15) to obtain 25.0 g (yield: 99%) of an intermediate B-8C.

### <Synthesis of intermediate B-8D>

The intermediate B-8C (12.3 g), 2,6-dimethylphenylboronic acid (16.3 g), cesium carbonate (35.3 g), methoxycyclopentane (CPME) (246 mL), and water (24.6 mL) were added to a 1 L three-neck flask, SPhos Pd G3 (1.45 g) was added thereto while stirring the mixture at room temperature in a nitrogen atmosphere, and the mixture was heated under reflux for 1.5 hours.

The obtained reaction solution was filtered through Celite, the obtained filtrate was separated and extracted, the obtained organic phase was dried over sodium sulfate, and the solvent was further removed. The obtained crude product was purified by silica gel column chromatography (eluent: hexane/ethyl acetate = 85/15) to obtain 8.8 g (yield: 82%) of an intermediate B-8D.

### <Synthesis of intermediate B-8E>

In a nitrogen atmosphere, the intermediate B-8D (8.8 g) and dehydrated tetrahydrofuran (180 mL) were added to a 500 mL three-neck flask, a tetrahydrofuran solution (1 mol/L, 30 mL) of lithium aluminum hydride was added dropwise thereto while stirring the mixture at 0°C, and the mixture was stirred at 0°C for 15 minutes.

Water (180 mL), a 15% by mass sodium hydroxide aqueous solution (1.14 mL), and water (3.42 mL) were added dropwise in this order to the obtained reaction solution, and the precipitated solid was removed by filtration. The obtained solution was dried over magnesium sulfate, and then the solvent was distilled off to obtain 7.6 g (yield: 95%) of an intermediate B-8E.

### <Synthesis of intermediate B-8F>

The intermediate B-8E (7.6 g) and dichloromethane (152 mL) were added to a 500 mL three-neck flask, a Dess-Martin reagent (12.0 g) was added thereto while stirring the mixture at room temperature, and the mixture was stirred at room temperature for 2 hours.

An aqueous solution obtained by dissolving 8.32 g of sodium thiosulfate in 180 mL of water was added to the obtained reaction solution, the mixture was liquid-separated, and the obtained organic phase was dried over sodium sulfate, and the solvent was further removed. The obtained crude product was purified by silica gel column chromatography (eluent: hexane/ethyl acetate = 80/20) to obtain 6.6 g (yield: 87%) of an intermediate B-8F.

### <Synthesis of intermediate B-8G>

The intermediate B-8F (6.6 g), toluene (100 mL), and ethylene glycol (12.4 mL) were added to a 300 mL three-neck flask, 0.94 g of p-toluenesulfonic acid monohydrate was added thereto while stirring the mixture at room temperature, and the mixture was heated under reflux for 2 hours.

2.07 mL of triethylamine, 100 mL of water, and 50 mL of ethyl acetate were added to the obtained reaction solution, and the organic phase was recovered by liquid separation. Saturated saline was added to the obtained organic phase, and the mixture was stirred and then subjected to liquid separation to recover the organic phase. The obtained organic phase was dried over sodium sulfate, and the solvent was further removed. The obtained crude product was purified by amino silica gel column chromatography (eluent: hexane/ethyl acetate = 90/10) to obtain 6.0 g (yield: 78%) of an intermediate B-8G.

### <Synthesis of intermediate B-8H>

In a nitrogen atmosphere, the intermediate B-8G (1.0 g) and dehydrated tetrahydrofuran (20 mL) were added to a 100 mL three-neck flask, a tetrahydrofuran solution (1.07 mol/L, 3.4 mL) of lithium diisopropylamide was added dropwise thereto while stirring the mixture at 0°C, and the mixture was stirred at 0°C for 20 minutes. 0.37 mL of dehydrated N,N-dimethylformamide was added to the reaction solution, the temperature was raised to room temperature, and the mixture was stirred for 1.5 hours. 20 mL of saturated saline and 20 mL of ethyl acetate were added thereto and stirred, the organic phase obtained by liquid separation was dried over sodium sulfate, and the solvent was further removed.

The obtained concentrate was dissolved in 16.5 mL of tetrahydrofuran, 3.3 mL of 30% by mass hydrochloric acid was added thereto while stirring the mixture at room temperature, and the mixture was stirred at room temperature for 15 minutes. 5 mL of triethylamine, 10 mL of water, and 10 mL of ethyl acetate were added to the reaction solution, the organic phase was recovered by liquid separation, dried over sodium sulfate, and the solvent was further removed.

The obtained crude product was purified by silica gel column chromatography (eluent: toluene/ethyl acetate = 90/10) to obtain 0.23 g (yield: 27%) of an intermediate B-8H.

### <Synthesis of compound B-8>

Piperidine (23 µL) was added to a 50 mL eggplant flask containing the intermediate B-8H (200 mg), 1,3-indandione (263 mg), and acetic acid (20 mL) while stirring at room temperature, and the mixture was reacted at 100°C for 4 hours.

After completion of the reaction, the precipitated solid was recovered by filtration, and the obtained crude product was re-crystallized from chloroform and methanol. The obtained solid was sublimated and purified to obtain 184 mg (yield: 49%) of a compound B-8.

¹H-nuclear magnetic resonance (NMR) data of the obtained compound B-8 are shown below.

¹H-NMR (CD₂Cl₂): δ = 9.26 (1H, s), 8.22 (1H, d), 8.10 to 8.00 (5H, m), 7.99 (1H, d), 7.92 to 7.86 (4H, m), 7.76 (1H, s), 7.36 (1H, dd), 7.28 (2H, d), 2.07 (6H, s)

### [Synthesis of compound B-47 (Synthesis Example 2 of specific compound)]

The compound B-47, which is the specific compound in the present invention, was synthesized according to the following scheme.

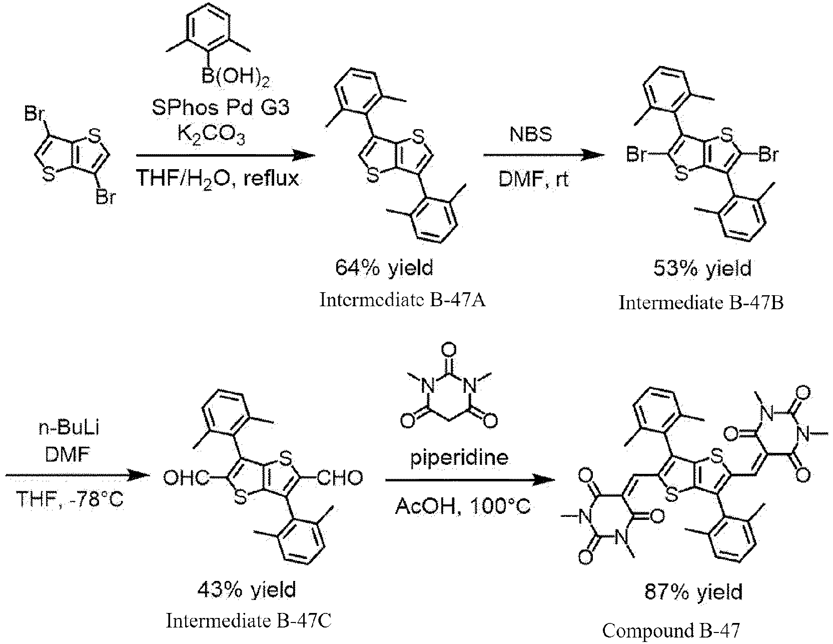

### <Synthesis of intermediate B-47A>

3,6-Dibromothieno[3,2-b]thiophene (10.0 g), 2,6-dimethylphenylboronic acid (15.2 g), potassium carbonate (29.0 g), tetrahydrofuran (250 mL), and water (76 mL) were added to a 1 L three-neck flask, SPhos Pd G3 (1.87 g) was added thereto while stirring the mixture at room temperature in a nitrogen atmosphere, and the reaction solution was heated under reflux all night.

The obtained reaction solution was allowed to stand, the aqueous phase was removed by liquid separation, 200 mL of saturated saline was added thereto, the mixture was stirred, and then the organic phase was recovered by liquid separation. 200 mL of dichloromethane was added to the organic phase, the organic phase was dried over magnesium sulfate, and the solvent was removed. The obtained crude product was subjected to dispersion washing with dichloromethane to obtain 7.5 g (yield: 64%) of an intermediate B-47A.

### <Synthesis of intermediate B-47B>

The intermediate B-47A (8.0 g) and N,N-dimethylformamide (400 mL) were added to a 1 L three-neck flask, N-bromosuccinimide (12.3 g) was added thereto while stirring the mixture at room temperature, and the mixture was stirred at 40°C for 4 hours.

The precipitate from the obtained reaction solution was collected by filtration to obtain a crude product. The crude product was completely dissolved in heated chloroform, and then cooled with ice to precipitate crystals. The precipitated crystals were collected by filtration to obtain 6.2 g (yield: 53%) of an intermediate B-47B.

### <Synthesis of intermediate B-47C>

In a nitrogen atmosphere, the intermediate B-47B (1.0 g) and dehydrated tetrahydrofuran (40 mL) were added to a 100 mL three-neck flask, an n-hexane solution (1.6 mol/L, 3.7 mL) of n-butyllithium was added dropwise thereto while stirring the mixture at -65°C, and the mixture was stirred at -65°C for 30 minutes. 0.69 mL of dehydrated N,N-dimethylformamide was added to the reaction solution, the temperature was raised to room temperature, and the mixture was stirred for 1.5 hours. 10 mL of a saturated ammonium chloride aqueous solution, 15 mL of 1 N hydrochloric acid, and 20 mL of ethyl acetate were added thereto, the mixture was stirred, the organic phase was recovered, and saturated saline was added thereto.

The organic phase was recovered by liquid separation, dried over sodium sulfate, and the solvent was further removed. The obtained crude product was crystallized with dichloromethane/ethyl acetate/methanol to obtain 0.35 g (yield: 43%) of an intermediate B-47C.

### <Synthesis of compound B-47>

Piperidine (24 µL) was added to the intermediate B-47C (250 mg), 1,3-dimethylbarbituric acid (251 mg), and acetic acid (25 mL) in a 100 mL eggplant flask while stirring at room temperature, and the mixture was reacted at 100°C for 1.5 hours.

After completion of the reaction, the precipitated solid was recovered by filtration, and the crude product was re-crystallized from methylene chloride and methanol. The obtained solid was sublimated and purified to obtain 218 mg (yield: 59%) of a compound B-47.

¹H-NMR data of the obtained compound B-47 are shown below.
¹H-NMR (CDCl₃): δ = 8.36 (2H, s), 7.38 (2H, dd), 7.25 (4H, d), 3.38 (6H, s), 3.32 (6H, s), 2.05 (12H, s)

Specific compounds and comparative compounds used in the photoelectric conversion film, other than the compound B-8 and the compound B-47, were synthesized with reference to [Synthesis of compound B-8] and [Synthesis of compound B-47] described above.

Each material used for the photoelectric conversion element is shown below. The compounds B-1 to B-73 correspond to the specific compounds used in Examples; and the compounds C1 to C3 correspond to the comparative compounds used in Comparative Examples.

### [Specific compound and comparative compound]

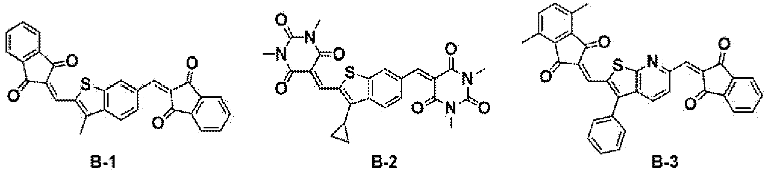

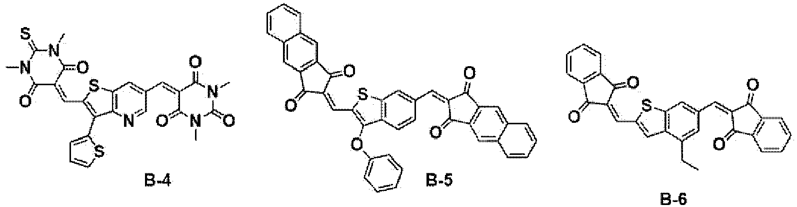

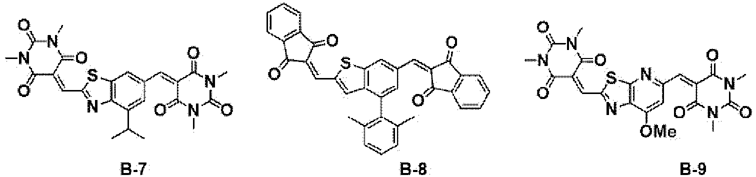

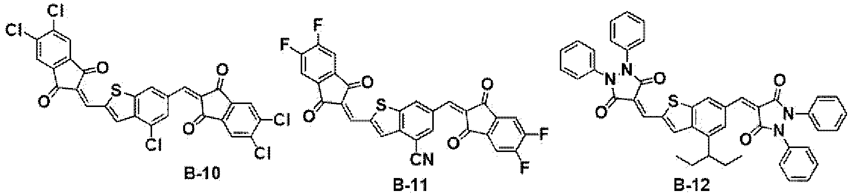

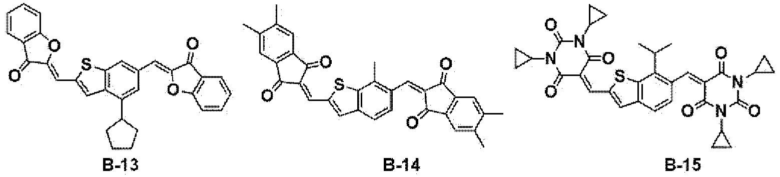

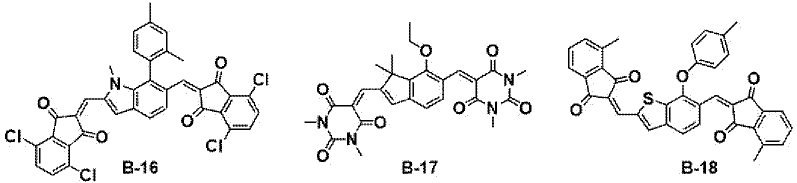

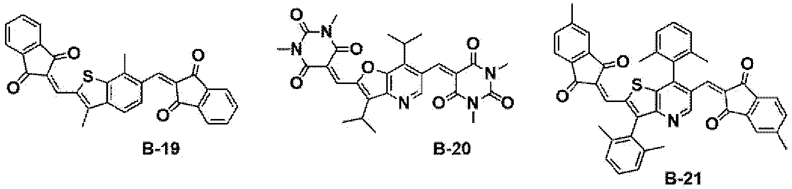

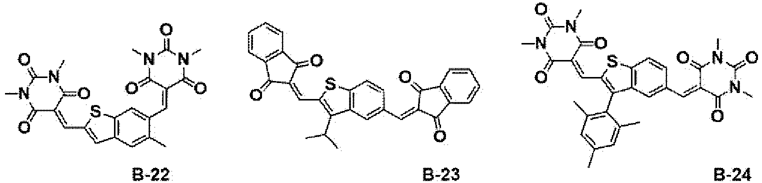

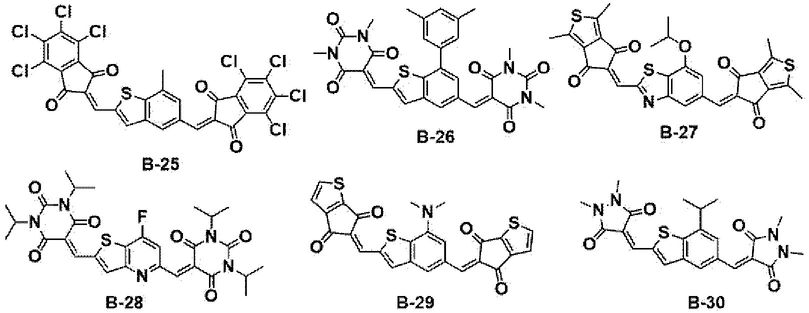

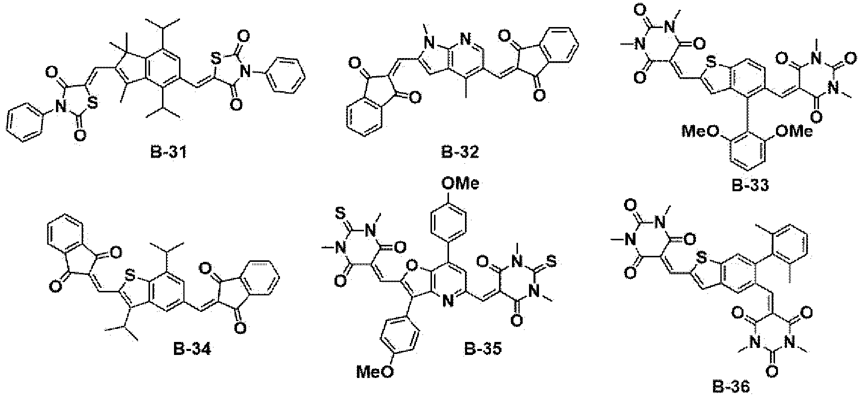

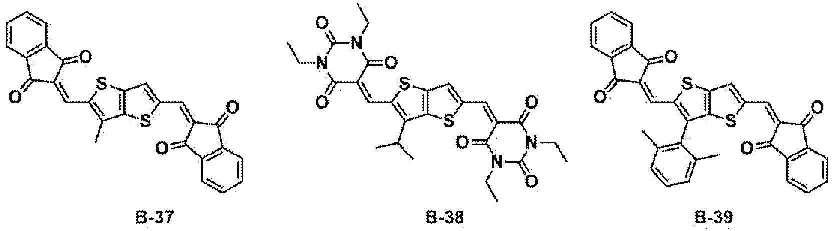

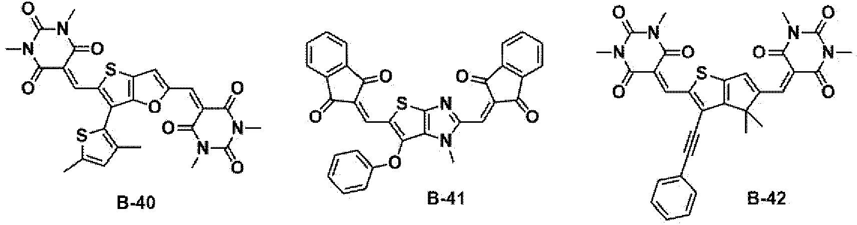

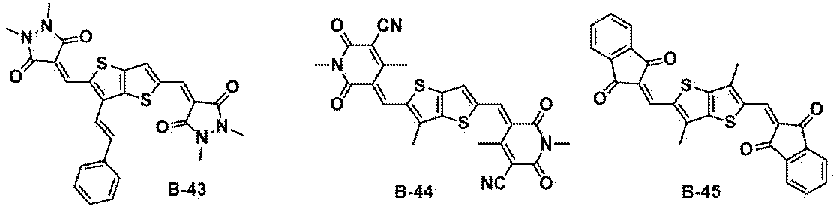

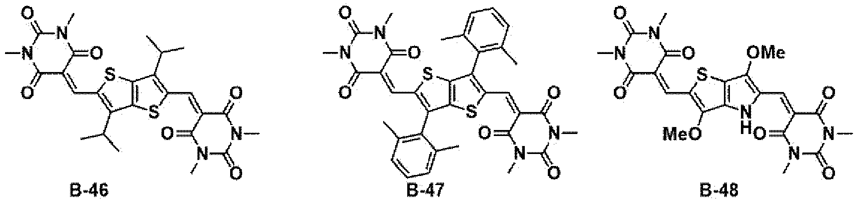

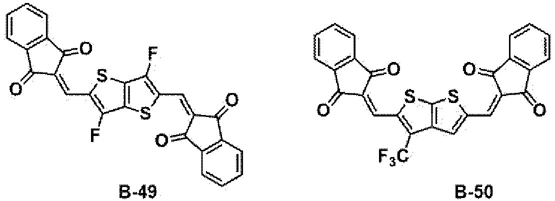

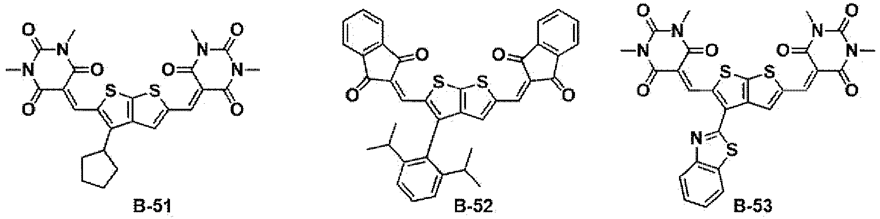

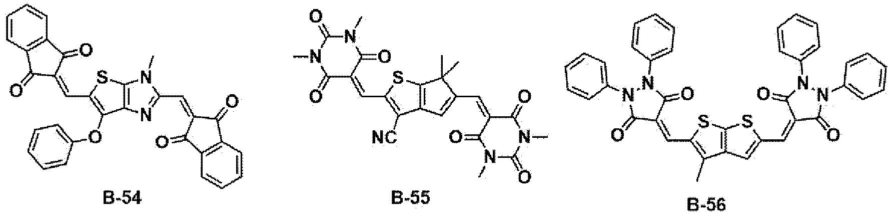

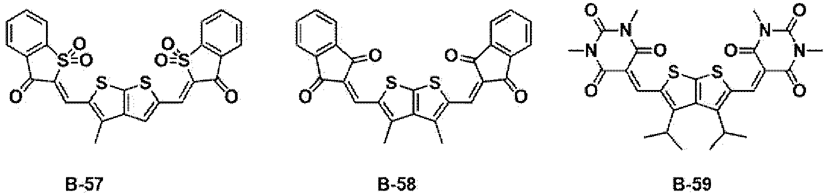

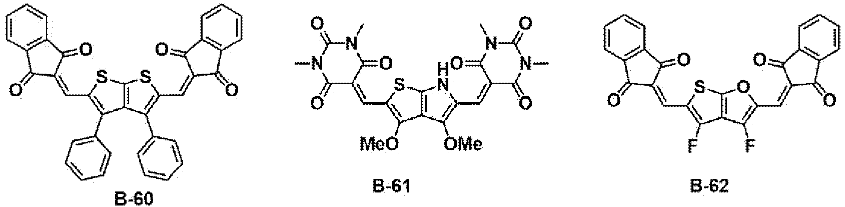

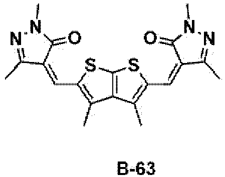

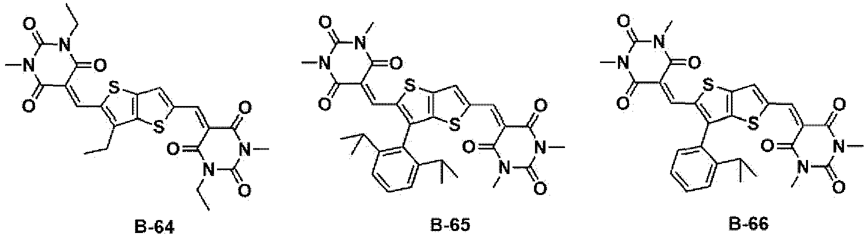

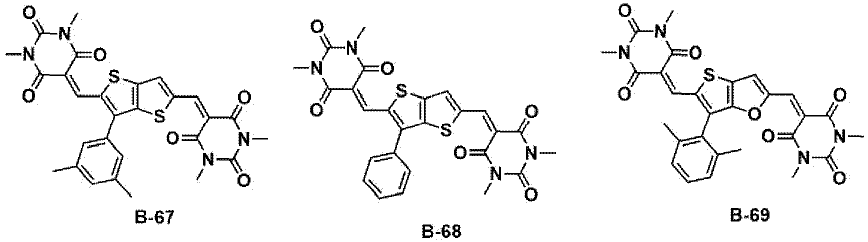

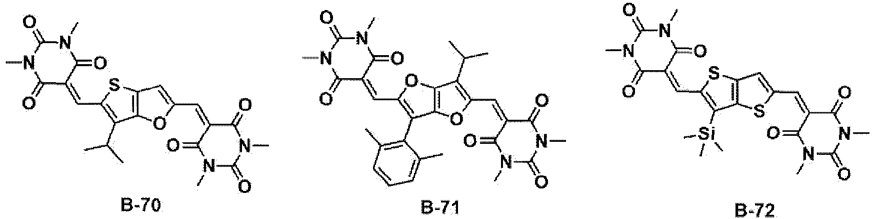

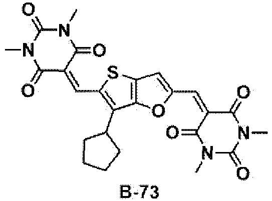

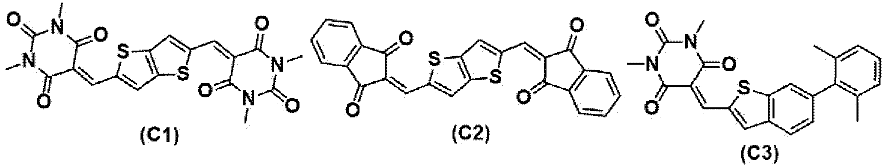

### [n-type organic semiconductor]

### · C60: fullerene (C₆₀)

### [p-type organic semiconductor]

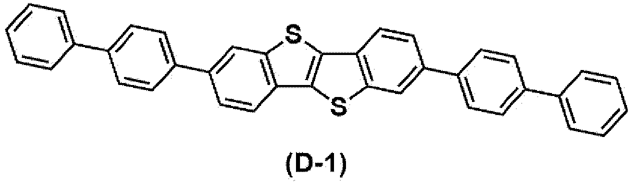

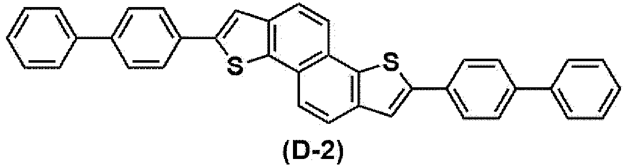

### [Evaluation]

A photoelectric conversion element was produced using each of the above-described materials, and the following evaluations were carried out.

### <Production of photoelectric conversion element>

A photoelectric conversion element having the form of Fig. 2 was produced using the various components shown above. Here, the photoelectric conversion element included a lower electrode 11, an electron blocking film 16A, a photoelectric conversion film 12, a hole blocking film 16B, and an upper electrode 15.

Specifically, an amorphous ITO was formed into a film on a glass substrate by a sputtering method to form the lower electrode 11 (thickness: 30 nm), and the electron blocking material (EB-1 or EB-3) shown in Tables 1 to 3 was further formed into a film on the lower electrode 11 by a vacuum thermal vapor deposition method to form the electron blocking film 16A (thickness: 30 nm).

Subsequently, the glass substrate was subjected to a vacuum vapor deposition method in a state of room temperature to form a film by co-vapor deposition of each specific compound or each comparative compound shown in Tables 1 to 3 on the electron blocking film 16A, and the n-type organic semiconductor (fullerene (C₆₀)) and the p-type organic semiconductor were each subjected to a co-vapor deposition method so that the thickness thereof was 80 nm in terms of single layer. As a result, the photoelectric conversion film 12 having a bulk hetero structure with 240 nm was formed. In this case, a film formation rate of the photoelectric conversion film 12 was set to 1.0 Å/sec.

Furthermore, the compound (EB-2) was vapor-deposited on the photoelectric conversion film 12 to form the hole blocking film 16B (thickness: 10 nm). Amorphous ITO was formed into a film on the hole blocking film 16B by a sputtering method to form the upper electrode 15 (transparent conductive film) (thickness: 10 nm). An SiO film was formed as a sealing layer on the upper electrode 15 by a vacuum vapor deposition method, an aluminum oxide (Al₂O₃) layer was formed thereon by an atomic layer chemical vapor deposition (ALCVD) method, and the obtained laminate was heated in a glove box at 150°C for 30 minutes to obtain a photoelectric conversion element of each of Examples and Comparative Examples.

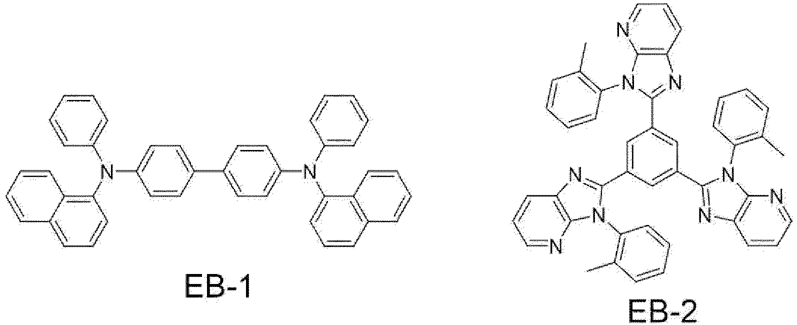

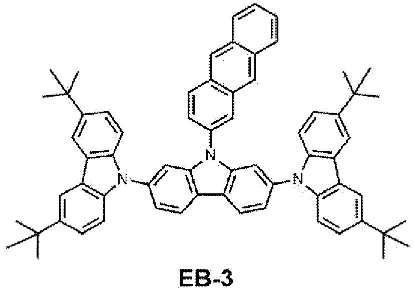

### <Dark current>

A dark current of each obtained photoelectric conversion element was measured by the following method.

A voltage was applied to the lower electrode and the upper electrode of each of the photoelectric conversion elements with an electric field strength of 2.5 × 10⁵ V/cm, and a current value (dark current) in a dark place was measured. As a result, it was confirmed that all of the photoelectric conversion elements had a dark current of 50 nA/cm² or less, which indicates that all of the photoelectric conversion elements had a sufficiently low dark current.

### <Quantum efficiency>

Quantum efficiency of each obtained photoelectric conversion element was measured by the following method.

A voltage was applied to each of the photoelectric conversion elements with an electric field strength of 2.0 × 10⁵ V/cm, and then light was emitted from the upper electrode (transparent conductive film) side to evaluate quantum efficiency (photoelectric conversion efficiency) at a wavelength of 460 nm. The quantum efficiency was obtained according to Expression (S1). Quantum efficiency (relative ratio) = (Quantum efficiency at wavelength of 460 nm in each of Examples or Comparative Examples)/(Quantum efficiency at wavelength of 460 nm in Example 1-47)

### (Evaluation standard)

A: quantum efficiency was 1.6 or more.
B: quantum efficiency was 1.2 or more and less than 1.6.
C: quantum efficiency was 0.8 or more and less than 1.2.
D: quantum efficiency was 0.4 or more and less than 0.8.
E: quantum efficiency was less than 0.4.

### <Electric field strength dependence of quantum efficiency>

Electric field strength dependence of the quantum efficiency of each photoelectric conversion element was evaluated by the following method.

Quantum efficiency (photoelectric conversion efficiency) at 7.5 × 10⁴ V/cm was measured by the same procedure as in the evaluation of [Quantum efficiency] described above, except that the voltage applied to each photoelectric conversion element was changed to 7.5 × 10⁴ V/cm.

The electric field strength dependence of the quantum efficiency was calculated according to Expression (S10), and the electric field strength dependence of the quantum efficiency was evaluated according to the following standard. In Expression (S10), the numerator and the denominator are values measured for the same photoelectric conversion element. It is preferable that the electric field strength dependence of the quantum efficiency was evaluated as C or more. Electric field strength dependence of quantum efficiency = (Quantum efficiency of each photoelectric conversion element at applied voltage of 7.5 × 104 V/cm)/(Quantum efficiency of each photoelectric conversion element at applied voltage of 2.0 × 105 V/cm)
A: electric field strength dependence of the quantum efficiency was 0.9 or more.
B: electric field strength dependence of the quantum efficiency was 0.8 or more and less than 0.9.
C: electric field strength dependence of the quantum efficiency was 0.7 or more and less than 0.8.
D: electric field strength dependence of the quantum efficiency was less than 0.7.

### <Response speed>

A response speed of each obtained photoelectric conversion element was evaluated by the following method.

A voltage was applied to the photoelectric conversion element with a strength of 2.0 × 10⁵ V/cm. Thereafter, a light emitting diode (LED) was turned on for an instant to emit light from the upper electrode (transparent conductive film) side, a photocurrent at this time at a wavelength of 460 nm was measured with an oscilloscope, a rise time until the signal intensity rose from 0% to 97% was measured, and the relative response speed was evaluated according to Expression (S2). Relative response speed = (Rise time at wavelength of 460 nm in each of Examples or Comparative Examples)/(Rise time at wavelength of 460 nm in Example 1-47)

### (Evaluation standard)

A: relative response speed was less than 0.5.
B: relative response speed was 0.5 or more and less than 1.0.
C: relative response speed was 1.0 or more and less than 1.5.
D: relative response speed was 1.5 or more and less than 2.0.
E: relative response speed was 2.0 or more.

### <Electric field strength dependence of response speed>

For each obtained photoelectric conversion element, electric field strength dependence of the response speed was evaluated by the following method.

A response speed at 7.5 × 10⁴ V/cm was measured by the same procedure as in the evaluation of the response speed, except that the voltage applied to each photoelectric conversion element was changed to 7.5 × 10⁴ V/cm.

The electric field strength dependence of the response speed was obtained and evaluated according to Expression (S3). Electric field strength dependence of response speed = (Rise time at 7.5 × 104 V/cm and wavelength of 460 nm in each of Examples or Comparative Examples)/(Rise time at 2.0 × 105 V/cm and wavelength of 460 nm in each of Examples or Comparative Examples)

In Expression (S3), each photoelectric conversion element used for the numerator and the denominator are the same.

For example, with regard to Example 1-1, the rise time of the photoelectric conversion efficiency at 7.5 × 10⁴ V/cm and a wavelength of 460 nm in Example 1-1 and the rise time of the photoelectric conversion efficiency at 2.0 × 10⁵ V/cm and a wavelength of 460 nm in Example 1-1 were compared.

### (Evaluation standard)

A: electric field strength dependence of the response speed was less than 2.0.
B: electric field strength dependence of the response speed was 2.0 or more and less than 3.0.
C: electric field strength dependence of the response speed was 3.0 or more and less than 4.0.
D: electric field strength dependence of the response speed was 4.0 or more and less than 5.0.
E: electric field strength dependence of the response speed was 5.0 or more.

### <Manufacturing suitability (vapor deposition heat resistance)>

A photoelectric conversion element (B) of each of Examples or Comparative Examples was produced according to the same procedure as that of the photoelectric conversion element (A), except that the film formation rate of the photoelectric conversion film 12 was set to 3.0 Å/sec. The obtained photoelectric conversion element (B) was evaluated by the same method as in <Quantum efficiency> described above.

The quantum efficiency of the photoelectric conversion element (B) was compared with the quantum efficiency of the photoelectric conversion element (A) having the same configuration in Examples or Comparative Examples, a relative ratio B/A of "Quantum efficiency of photoelectric conversion element (B)/Quantum efficiency of photoelectric conversion element (A)" was calculated, and manufacturing suitability of each photoelectric conversion element was evaluated by comparing the obtained value with the following standard. The fact that the present evaluation result was excellent indicates that the compound was a material in which the performance was less likely to be deteriorated during high-speed film formation, and thus indicates that the compound had excellent manufacturing suitability.

### (Evaluation standard)

A: relative ratio B/A was 0.90 or more.
B: relative ratio B/A was 0.85 or more and less than 0.90.
C: relative ratio B/A was 0.80 or more and less than 0.85.
D: relative ratio B/A was 0.75 or more and less than 0.80.
E: relative ratio B/A was less than 0.75.

Tables 1 to 3 show the above-described evaluation results.

The respective notations in Tables 1 to 3 indicate the following.

The column "D" indicates which of the groups represented by Formula (2) to Formula (5) corresponded to D in the compound.

The column of "Formulae (2-10) to (5-11)" indicates which of the groups represented by Formula (2-10), Formula (2-11), Formula (2-12), Formula (2-13), Formula (3-10), Formula (3-11), Formula (3-12), Formula (3-13), Formula (4-10), Formula (4-11), Formula (5-10), and Formula (5-11) corresponded to D in Formula (1); and in a case where D did not correspond to any of the formulae, it is indicated as "B".

In the column of "W¹ = O or S", a case where W¹ in Formula (A-1) was an oxygen atom or a sulfur atom is indicated as "A", and a case other than the above case is indicated as "B".

In the column of "Formula (A-1) = Formula (A-2)", a case where the group represented by Formula (A-1) was the group represented by Formula (A-2) is indicated as "A", and a case other than the above case is indicated as "B".

In the column of "Formula (A-1) = Formula (C-1) or Formula (C-2)", a case where the group represented by Formula (A-1) in Formula (1) was the group represented by Formula (C-1) or Formula (C-2) is indicated as "A", and a case other than the above case is indicated as "B".

In the column of "X = O", a case where both X^{c1} and X^{c2} in Formula (C-1) were oxygen atoms or a case where both X^{c3} and X^{c4} in Formula (C-2) were oxygen atoms is indicated as "A", and a case other than the above case is indicated as "B".

In the column of "Rs", a case where Rs in Formula (1) represented a linear alkyl group having 1 to 5 carbon atoms, which may have a substituent, a branched alkyl group having 3 to 7 carbon atoms, which may have a substituent, a cyclic alkyl group having 3 to 6 carbon atoms, which may have a substituent, an aryl group having 6 to 14 carbon atoms, which may have a substituent, a heteroaryl group having 2 to 14 carbon atoms, which may have a substituent, an alkoxy group having 1 to 5 carbon atoms, which may have a substituent, an aryloxy group having 6 to 14 carbon atoms, which may have a substituent, or a halogen atom is indicated as "A", and a case other than the above case is indicated as "B".

**[Table 1]**

| | Specific compound or comparative compound | | | | | | | | | | Evaluation | | | | |
|---|---|---|---|---|---|---|---|---|---|---|---|---|---|---|---|
| | Compound | p-Type organic semiconductor | Electron blocking material | D | Formulae (2-10) to (5-11) | W¹ = O or S | Formula (A-1) = Formula (A-2) | Formula (A-1) = Formula (C-1) or Formula (C-2) | X = O | Rs | Quantum efficiency | Electric field strength dependence of quantum efficiency | Response speed | Electric field strength dependence of response speed | Manufacturing suitability |
| Example 1-1 | B-1 | D-1 | EB-1 | (2) | (2-10) | A | A | A | A | A | A | C | B | B | A |
| Example 1-2 | B-2 | D-1 | EB-1 | (2) | (2-10) | A | A | A | A | A | A | C | B | B | A |
| Example 1-3 | B-3 | D-1 | EB-1 | (2) | (2-10) | A | A | A | A | A | A | C | B | B | A |
| Example 1-4 | B-4 | D-1 | EB-1 | (2) | (2-10) | A | A | A | A | A | A | C | B | B | A |
| Example 1-5 | B-5 | D-1 | EB-1 | (2) | (2-10) | A | A | A | A | A | A | C | B | B | A |
| Example 1-6 | B-6 | D-1 | EB-1 | (2) | (2-11) | A | A | A | A | A | A | C | A | A | A |
| Example 1-7 | B-7 | D-1 | EB-1 | (2) | (2-11) | A | A | A | A | A | A | C | A | A | A |
| Example 1-8 | B-8 | D-1 | EB-1 | (2) | (2-11) | A | A | A | A | A | A | C | A | A | A |
| Example 1-9 | B-9 | D-1 | EB-1 | (2) | (2-11) | A | A | A | A | A | A | C | A | A | A |
| Example 1-10 | B-10 | D-1 | EB-1 | (2) | (2-11) | A | A | A | A | A | A | C | A | A | A |
| Example 1-11 | B-11 | D-1 | EB-1 | (2) | (2-11) | A | A | A | A | B | A | C | A | B | B |
| Example 1-12 | B-12 | D-1 | EB-1 | (2) | (2-11) | A | A | B | B | A | A | C | A | C | B |
| Example 1-13 | B-13 | D-1 | EB-1 | (2) | (2-11) | A | B | B | B | A | A | C | A | C | C |
| Example 1-14 | B-14 | D-1 | EB-1 | (2) | (2-12) | A | A | A | A | A | A | C | A | B | A |
| Example 1-15 | B-15 | D-1 | EB-1 | (2) | (2-12) | A | A | A | A | A | A | C | A | B | A |
| Example 1-16 | B-16 | D-1 | EB-1 | (2) | (2-12) | A | A | A | A | A | A | C | A | B | A |
| Example 1-17 | B-17 | D-1 | EB-1 | (2) | (2-12) | A | A | A | A | A | A | C | A | B | A |
| Example 1-18 | B-18 | D-1 | EB-1 | (2) | (2-12) | A | A | A | A | A | A | C | A | B | A |
| Example 1-19 | B-19 | D-1 | EB-1 | (2) | (2-13) | A | A | A | A | A | A | C | B | B | A |
| Example 1-20 | B-20 | D-1 | EB-1 | (2) | (2-13) | A | A | A | A | A | A | C | B | B | A |
| Example 1-21 | B-21 | D-1 | EB-1 | (2) | (2-13) | A | A | A | A | A | A | C | B | B | A |
| Example 1-22 | B-22 | D-1 | EB-1 | (2) | B | A | A | A | A | A | A | C | C | C | C |
| Example 1-23 | B-23 | D-1 | EB-1 | (3) | (3-10) | A | A | A | A | A | B | C | B | B | A |
| Example 1-24 | B-24 | D-1 | EB-1 | (3) | (3-10) | A | A | A | A | A | B | C | B | B | A |
| Example 1-25 | B-25 | D-1 | EB-1 | (3) | (3-11) | A | A | A | A | A | B | C | A | A | A |
| Example 1-26 | B-26 | D-1 | EB-1 | (3) | (3-11) | A | A | A | A | A | B | C | A | A | A |
| Example 1-27 | B-27 | D-1 | EB-1 | (3) | (3-11) | A | A | A | A | A | B | C | A | A | A |
| Example 1-28 | B-28 | D-1 | EB-1 | (3) | (3-11) | A | A | A | A | A | B | C | A | A | A |
| Example 1-29 | B-29 | D-1 | EB-1 | (3) | (3-11) | A | A | A | A | B | B | C | B | B | B |
| Example 1-30 | B-30 | D-1 | EB-1 | (3) | (3-11) | A | A | B | B | A | B | C | B | C | B |
| Example 1-31 | B-31 | D-1 | EB-1 | (3) | (3-11) | A | B | B | B | A | B | C | B | C | C |
| Example 1-32 | B-32 | D-1 | EB-1 | (3) | (3-12) | A | A | A | A | A | B | C | B | B | A |
| Example 1-33 | B-33 | D-1 | EB-1 | (3) | (3-12) | A | A | A | A | A | B | C | B | B | A |
| Example 1-34 | B-34 | D-1 | EB-1 | (3) | (3-13) | A | A | A | A | A | B | C | A | B | A |
| Example 1-35 | B-35 | D-1 | EB-1 | (3) | (3-13) | A | A | A | A | A | B | C | A | B | A |
| Example 1-36 | B-36 | D-1 | EB-1 | (3) | B | A | A | A | A | A | B | C | C | C | C |

**[Table 2]**

| | Specific compound or comparative compound | | | | | | | | | | Evaluation | | | | |
|---|---|---|---|---|---|---|---|---|---|---|---|---|---|---|---|
| | Compound | p-Type organic semiconductor | Electron blocking material | D | Formulae (2-10) to (5-11) | W¹ = O or S | Formula (A-1) = Formula (A-2) | Formula (A-1) = Formula (C-1) or Formula (C-2) | X = O | Rs | Quantum efficiency | Electric field strength dependence of quantum efficiency | Response speed | Electric field strength dependence of response speed | Manufacturing suitability |
| Example 1-37 | B-37 | D-1 | EB-1 | (4) | (4-10) | A | A | A | A | A | C | A | B | B | A |
| Example 1-38 | B-38 | D-1 | EB-1 | (4) | (4-10) | A | A | A | A | A | C | A | B | B | A |
| Example 1-39 | B-39 | D-1 | EB-1 | (4) | (4-10) | A | A | A | A | A | C | A | B | B | A |
| Example 1-4C | B-40 | D-1 | EB-1 | (4) | (4-10) | A | A | A | A | A | C | B | B | B | A |
| Example 1-41 | B-41 | D-1 | EB-1 | (4) | (4-10) | A | A | A | A | A | C | B | B | B | A |
| Example 1-42 | B-42 | D-1 | EB-1 | (4) | (4-10) | A | A | A | A | B | C | B | B | B | B |
| Example 1-43 | B-43 | D-1 | EB-1 | (4) | (4-10) | A | A | B | B | B | C | B | B | C | B |
| Example 1-44 | B-44 | D-1 | EB-1 | (4) | (4-10) | A | B | B | B | A | C | A | B | C | c |
| Example 1-45 | B-45 | D-1 | EB-1 | (4) | (4-11) | A | A | A | A | A | C | A | C | B | A |
| Example 1-46 | B-46 | D-1 | EB-1 | (4) | (4-11) | A | A | A | A | A | C | A | C | B | A |
| Example 1-47 | B-47 | D-1 | EB-1 | (4) | (4-11) | A | A | A | A | A | C | A | C | B | A |
| Example 1-48 | B-48 | D-1 | EB-1 | (4) | (4-11) | A | A | A | A | A | C | B | C | B | A |
| Example 1-49 | B-49 | D-1 | EB-1 | (4) | (4-11) | A | A | A | A | A | C | B | C | B | A |
| Example 1-5C | B-50 | D-1 | EB-1 | (5) | (5-10) | A | A | A | A | A | B | C | B | B | A |
| Example 1-51 | B-51 | D-1 | EB-1 | (5) | (5-10) | A | A | A | A | A | B | C | B | B | A |
| Example 1-52 | B-52 | D-1 | EB-1 | (5) | (5-10) | A | A | A | A | A | B | C | B | B | A |
| Example 1-53 | B-53 | D-1 | EB-1 | (5) | (5-10) | A | A | A | A | A | B | C | B | B | A |
| Example 1-54 | B-54 | D-1 | EB-1 | (5) | (5-10) | A | A | A | A | A | B | C | B | B | A |
| Example 1-55 | B-55 | D-1 | EB-1 | (5) | (5-10) | A | A | A | A | B | B | C | B | B | B |
| Example 1-56 | B-56 | D-1 | EB-1 | (5) | (5-10) | A | A | B | B | A | B | C | B | C | B |
| Example 1-57 | B-57 | D-1 | EB-1 | (5) | (5-10) | A | B | B | B | A | B | C | B | C | c |
| Example 1-58 | B-58 | D-1 | EB-1 | (5) | (5-11) | A | A | A | A | A | B | C | C | B | A |
| Example 1-59 | B-59 | D-1 | EB-1 | (5) | (5-11) | A | A | A | A | A | B | C | C | B | A |
| Example 1-6C | B-60 | D-1 | EB-1 | (5) | (5-11) | A | A | A | A | A | B | C | C | B | A |
| Example 1-61 | B-61 | D-1 | EB-1 | (5) | (5-11) | A | A | A | A | A | B | C | C | B | A |
| Example 1-62 | B-62 | D-1 | EB-1 | (5) | (5-11) | A | A | A | A | A | B | C | C | B | A |
| Example 1-63 | B-63 | D-1 | EB-1 | (5) | (5-11) | A | B | B | B | A | B | C | C | C | c |
| Example 1-64 | B-47 | D-2 | EB-1 | (4) | (4-11) | A | A | A | A | A | C | A | C | B | A |
| Comparative Example 1-1 | C1 | D-1 | EB-1 | - | - | A | A | A | A | B | D | D | E | E | E |
| Comparative Example 1-2 | C2 | D-1 | EB-1 | - | - | A | A | A | A | B | E | D | D | E | E |
| Comparative Example 1-3 | C3 | D-1 | EB-1 | - | - | A | A | A | A | B | D | D | E | D | D |

**[Table 3]**

| | Specific compound or comparative compound | | | | | | | | | | Evaluation | | | | |
|---|---|---|---|---|---|---|---|---|---|---|---|---|---|---|---|
| | Compound | p-Type organic semiconductor | Electron blocking material | D | Formulae (2-10) to (5-11) | W¹ = O or S | Formula (A-1) = Formula (A-2) | Formula (A-1) = Formula (C-1) or Formula (C-2) | X = O | Rs | Quantum efficiency | Electric field strength dependence of quantum efficiency | Response speed | Electric field strength dependence of response speed | Manufacturing suitability |
| Example 1-65 | B-64 | D-1 | EB-1 | (4) | (4-10) | A | A | A | A | A | C | A | B | B | A |
| Example 1-66 | B-65 | D-1 | EB-1 | (4) | (4-10) | A | A | A | A | A | C | A | B | B | A |
| Example 1-67 | B-66 | D-1 | EB-1 | (4) | (4-10) | A | A | A | A | A | C | A | B | B | A |
| Example 1-68 | B-67 | D-1 | EB-1 | (4) | (4-10) | A | A | A | A | A | C | B | B | B | A |
| Example 1-69 | B-68 | D-1 | EB-1 | (4) | (4-10) | A | A | A | A | A | C | B | B | B | A |
| Example 1-70 | B-69 | D-1 | EB-1 | (4) | (4-10) | A | A | A | A | A | C | A | B | B | A |
| Example 1-71 | B-70 | D-1 | EB-1 | (4) | (4-10) | A | A | A | A | A | C | A | B | B | A |
| Example 1-72 | B-71 | D-1 | EB-1 | (4) | (4-11) | A | A | A | A | A | C | A | B | B | A |
| Example 1-73 | B-72 | D-1 | EB-1 | (4) | (4-10) | A | A | A | A | A | C | B | B | B | A |
| Example 1-74 | B-73 | D-1 | EB-1 | (4) | (4-10) | A | A | A | A | A | C | A | B | B | A |
| Example 1-75 | B-1 | D-1 | EB-3 | (2) | (2-10) | A | A | A | A | A | A | C | B | B | A |
| Example 1-76 | B-2 | D-1 | EB-3 | (2) | (2-10) | A | A | A | A | A | A | C | B | B | A |
| Example 1-77 | B-4 | D-1 | EB-3 | (2) | (2-10) | A | A | A | A | A | A | C | B | B | A |
| Example 1-78 | B-6 | D-1 | EB-3 | (2) | (2-11) | A | A | A | A | A | A | C | A | A | A |
| Example 1-79 | B-8 | D-1 | EB-3 | (2) | (2-11) | A | A | A | A | A | A | C | A | A | A |
| Example 1-80 | B-19 | D-1 | EB-3 | (2) | (2-13) | A | A | A | A | A | A | C | B | B | A |
| Example 1-81 | B-24 | D-1 | EB-3 | (3) | (3-10) | A | A | A | A | A | B | C | B | B | A |
| Example 1-82 | B-26 | D-1 | EB-3 | (3) | (3-11) | A | A | A | A | A | B | C | A | A | A |
| Example 1-83 | B-37 | D-1 | EB-3 | (4) | (4-10) | A | A | A | A | A | C | A | B | B | A |
| Example 1-84 | B-38 | D-1 | EB-3 | (4) | (4-10) | A | A | A | A | A | C | A | B | B | A |
| Example 1-85 | B-39 | D-1 | EB-3 | (4) | (4-10) | A | A | A | A | A | C | A | B | B | A |
| Example 1-86 | B-40 | D-1 | EB-3 | (4) | (4-10) | A | A | A | A | A | C | A | B | B | A |
| Example 1-87 | B-44 | D-1 | EB-3 | (4) | (4-10) | A | B | B | B | A | C | A | B | C | C |
| Example 1-88 | B-45 | D-1 | EB-3 | (4) | (4-11) | A | A | A | A | A | C | A | C | B | A |
| Example 1-89 | B-46 | D-1 | EB-3 | (4) | (4-11) | A | A | A | A | A | C | A | C | B | A |
| Example 1-90 | B-47 | D-1 | EB-3 | (4) | (4-11) | A | A | A | A | A | C | A | C | B | A |
| Example 1-91 | B-65 | D-1 | EB-3 | (4) | (4-10) | A | A | A | A | A | C | A | B | B | A |
| Example 1-92 | B-66 | D-1 | EB-3 | (4) | (4-10) | A | A | A | A | A | C | A | B | B | A |
| Example 1-93 | B-69 | D-1 | EB-3 | (4) | (4-10) | A | A | A | A | A | C | A | B | B | A |

As is clear from the results in the tables, it was found that the photoelectric conversion elements of Examples according to the present invention had excellent quantum efficiency. In addition, it was found that the photoelectric conversion element according to the embodiment of the present invention was also excellent in electric field strength dependence of the quantum efficiency, response speed, electric field strength dependence of the response speed, and manufacturing suitability.

On the other hand, the photoelectric conversion elements of Comparative Example, using the comparative compound not corresponding to the specific compound, had an insufficient quantum efficiency.

In addition, from the comparison of Examples 1-1 to 1-22 (D was the group represented by Formula (2)), Examples 1-23 to 1-36 (D was the group represented by Formula (3)), Examples 1-37 to 1-49 (D was the group represented by Formula (4)), and Examples 1-50 to 1-63 (D was the group represented by Formula (5)), it was found that, in the case of the group represented by Formula (2), Formula (3), or Formula (5), the quantum efficiency was more excellent, and in the case of the group represented by Formula (2), the quantum efficiency was further excellent.

It was found that, in a case where D represented the group represented by any of Formula (2-10), Formula (2-11), Formula (2-12), Formula (2-13), Formula (3-10), Formula (3-11), Formula (3-12), Formula (3-13), Formula (4-10), or Formula (5-10), the response speed was more excellent. In addition, it was found that, in a case where D represented the group represented by any of Formula (2-11), Formula (2-12), Formula (3-11), or Formula (3-13), the response speed was more excellent.

From the comparison of Examples 1-22 and 1-36 and other examples, it was found that, in a case where D in the specific compound represented the group represented by any of Formula (2-10), Formula (2-11), Formula (2-12), Formula (2-13), Formula (3-10), Formula (3-11), Formula (3-12), Formula (3-13), Formula (4-10), Formula (4-11), Formula (5-10), or Formula (5-11), at least one of the response speed, the electric field strength dependence of the response speed, or the manufacturing suitability was more excellent. In addition, from the comparison of Examples 1-1 to 1-10, the comparison of Examples 1-25 to 1-28 and Examples 1-32 to 1-35, and the like, it was found that, in a case where D represented the group represented by Formula (2-11) or Formula (3-11), the electric field strength dependence of the response speed was more excellent.

From the results of Examples 1-12 to 1-13, Examples 1-30 and 1-31, and Examples 1-43 and 1-44, Examples 1-56 and 1-57, and Example 1-63, it was found that, in a case where the group represented by Formula (A-1) in the specific compound is the group represented by Formula (C-1) or the group represented by Formula (C-2), the electric field strength dependence of the response speed was more excellent.

From the comparison of Examples 1-1 to 1-12 and Example 1-13, and the comparison of Examples 1-50 to 1-56 and Example 1-57, it was found that, in a case where the group represented by Formula (A-1) in the specific compound is the group represented by Formula (A-2), the manufacturing suitability was more excellent.

From the comparison of Examples 1-1 to 1-11 and Examples 1-50 to 1-55, it was found that, in a case where Rs in the specific compound represented a linear alkyl group having 1 to 5 carbon atoms, which may have a substituent, a branched alkyl group having 3 to 7 carbon atoms, which may have a substituent, a cyclic alkyl group having 3 to 6 carbon atoms, which may have a substituent, an aryl group having 6 to 14 carbon atoms, which may have a substituent, a heteroaryl group having 2 to 14 carbon atoms, which may have a substituent, an alkoxy group having 1 to 5 carbon atoms, which may have a substituent, an aryloxy group having 6 to 14 carbon atoms, which may have a substituent, or a halogen atom, the manufacturing suitability was further excellent.

In addition, from the comparison of Examples 1-37 to 1-64 and the like, it was found that, in a case where D in the specific compound represented the group represented by any of Formula (4-10) or Formula (4-11), the electric field strength dependence of the quantum efficiency was more excellent; and from the comparison of Examples 1-37 to 1-49 and the comparison of Examples 1-65 to 1-74, it was found that, in a case where D in the specific compound represented the group represented by any of Formula (4-10) or Formula (4-11) and Rs represented a linear alkyl group having 1 to 5 carbon atoms, which may have a substituent, a branched alkyl group having 3 to 7 carbon atoms, which may have a substituent, a cyclic alkyl group having 3 to 6 carbon atoms, which may have a substituent, or the group represented by Formula (S1), the electric field strength dependence of the quantum efficiency was further excellent.

### Explanation of References

10a, 10b: photoelectric conversion element
11: conductive film (lower electrode)
12: photoelectric conversion film
15: transparent conductive film (upper electrode)
16A: electron blocking film
16B: hole blocking film

## Claims

1. A photoelectric conversion element comprising, in the following order:
a conductive film;
a photoelectric conversion film; and
a transparent conductive film,
wherein the photoelectric conversion film contains a compound represented by Formula (1), 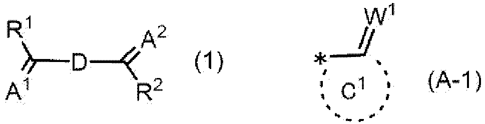 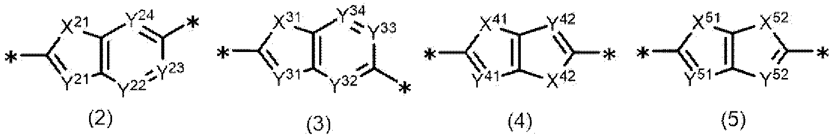
in Formula (1),
D represents a group represented by any of Formula (2) to Formula (5),
in Formula (2) to Formula (5), * represents a bonding position,
R¹ and R² each independently represent a hydrogen atom or a substituent,
A¹ and A² each independently represent a group represented by Formula (A-1), and in Formula (A-1), * represents a bonding position,
in Formula (2) to Formula (5),
X²¹, X³¹, X⁴¹, X⁴², X⁵¹, and X⁵² each independently represent a sulfur atom, an oxygen atom, NR^{X1}, or CR^{X2}R^{X3},
R^{X1} to R^{X3} each independently represent a hydrogen atom, an aliphatic hydrocarbon group which may have a substituent, or an aromatic ring group which may have a substituent,
R^{X2} and R^{X3} may be linked to each other through a single bond or a divalent linking group,
Y²¹, Y²², Y²³, Y²⁴, Y³¹, Y³², Y³³, Y³⁴, Y⁴¹, Y⁴², Y⁵¹, and Y⁵² each independently represent -CR^{Y1}= or a nitrogen atom, and R^{Y1} represents a hydrogen atom or a substituent,
provided that, in Formula (2), at least one of Y²¹, ..., or Y²⁴ represents -CRs=, in Formula (3), at least one of Y³¹, ..., or Y³⁴ represents -CRs=, in Formula (4), at least one of Y⁴¹ or Y⁴² represents -CRs=, and in Formula (5), at least one of Y⁵¹ or Y⁵² represents -CRs=,
where Rs represents an alkyl group which may have a substituent, an alkenyl group which may have a substituent, an alkynyl group which may have a substituent, an aryl group which may have a substituent, a heteroaryl group which may have a substituent, an alkoxy group which may have a substituent, an aryloxy group which may have a substituent, an amino group which may have a substituent, a silyl group which may have a substituent, a cyano group, or a halogen atom,
in Formula (A-1),
C¹ represents a ring which contains two or more carbon atoms and may have a substituent,
W¹ represents a sulfur atom, an oxygen atom, =NR^{W2}, or =CR^{W3}R^{W4},
R^{W2} represents a hydrogen atom or a substituent,
R^{W3} and R^{W4} each independently represent a cyano group, -SO₂R^{W5}, -COOR^{W6}, or -COR^{W7}, and
R^{W5} to R^{W7} each independently represent an aliphatic hydrocarbon group which may have a substituent, an aromatic ring group which may have a substituent, or an aliphatic heterocyclic group which may have a substituent.

2. The photoelectric conversion element according to claim 1,
wherein R^{Y1} represents a hydrogen atom, an alkyl group which may have a substituent, an alkenyl group which may have a substituent, an alkynyl group which may have a substituent, an aryl group which may have a substituent, a heteroaryl group which may have a substituent, an alkoxy group which may have a substituent, an aryloxy group which may have a substituent, an amino group which may have a substituent, a silyl group which may have a substituent, a cyano group, or a halogen atom.

3. The photoelectric conversion element according to claim 1,
wherein D represents a group represented by any of Formula (2-10), Formula (2-11), Formula (2-12), Formula (2-13), Formula (3-10), Formula (3-11), Formula (3-12), Formula (3-13), Formula (4-10), Formula (4-11), Formula (5-10), or Formula (5-11), 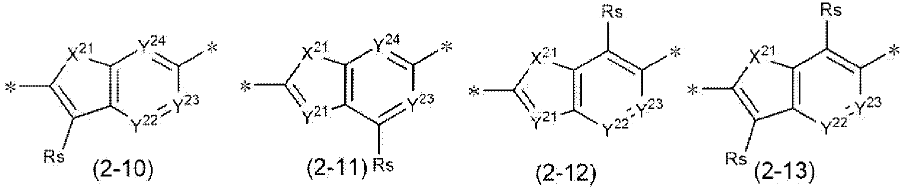 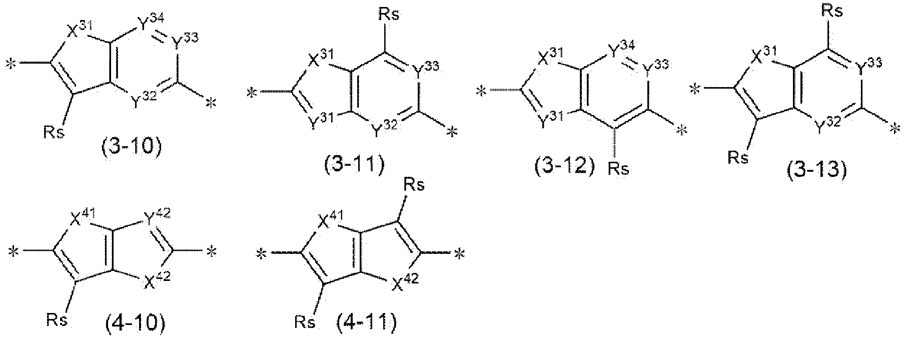 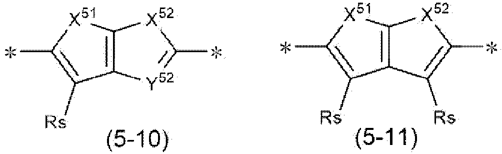
in Formula (2-10), Formula (2-11), Formula (2-12), Formula (2-13), Formula (3-10), Formula (3-11), Formula (3-12), Formula (3-13), Formula (4-10), Formula (4-11), Formula (5-10), and Formula (5-11), * represents a bonding position,
X²¹, X³¹, X⁴¹, X⁴², X⁵¹, and X⁵² each independently represent a sulfur atom, an oxygen atom, NR^{X1}, or CR^{X2}R^{X3},
R^{X1} to R^{X3} each independently represent a hydrogen atom, an aliphatic hydrocarbon group which may have a substituent, or an aromatic ring group which may have a substituent,
Y²¹, Y²², Y²³, Y²⁴, Y³¹, Y³², Y³³, Y³⁴, Y⁴², and Y⁵² each independently represent -CR^{Y1}= or a nitrogen atom, and R^{Y1} represents a hydrogen atom or a substituent,
where Rs represents an alkyl group which may have a substituent, an alkenyl group which may have a substituent, an alkynyl group which may have a substituent, an aryl group which may have a substituent, a heteroaryl group which may have a substituent, an alkoxy group which may have a substituent, an aryloxy group which may have a substituent, an amino group which may have a substituent, a silyl group which may have a substituent, a cyano group, or a halogen atom.

4. The photoelectric conversion element according to claim 3,
wherein Rs represents a linear alkyl group having 1 to 5 carbon atoms, which may have a substituent, a branched alkyl group having 3 to 7 carbon atoms, which may have a substituent, a cyclic alkyl group having 3 to 6 carbon atoms, which may have a substituent, an alkenyl group having 2 to 5 carbon atoms, which may have a substituent, an alkynyl group having 2 to 5 carbon atoms, which may have a substituent, an aryl group having 6 to 14 carbon atoms, which may have a substituent, a heteroaryl group having 2 to 14 carbon atoms, which may have a substituent, an alkoxy group having 1 to 5 carbon atoms, which may have a substituent, an aryloxy group having 6 to 14 carbon atoms, which may have a substituent, an amino group which may have a substituent, a silyl group which may have a substituent, a cyano group, or a halogen atom.

5. The photoelectric conversion element according to claim 4,
wherein D represents the group represented by any of Formula (2-10), Formula (2-11), Formula (2-12), Formula (2-13), Formula (3-10), Formula (3-11), Formula (3-12), Formula (3-13), Formula (5-10), or Formula (5-11).

6. The photoelectric conversion element according to claim 1,
wherein W¹ is an oxygen atom or a sulfur atom.

7. The photoelectric conversion element according to claim 6,
wherein the group represented by Formula (A-1) is a group represented by Formula (A-2), 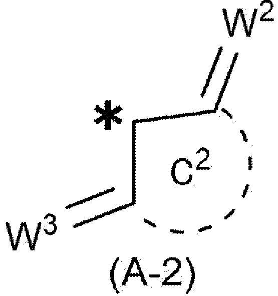
in Formula (A-2), * represents a bonding position,
C² represents a ring which contains three or more carbon atoms and may have a substituent, and
W² and W³ each independently represent an oxygen atom or a sulfur atom.

8. The photoelectric conversion element according to claim 7,
wherein the group represented by Formula (A-2) is a group represented by Formula (C-1) or a group represented by Formula (C-2), 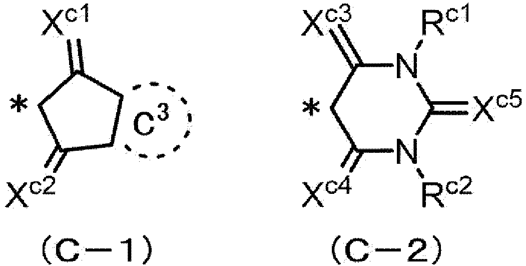
in Formula (C-1) and Formula (C-2),
* represents a bonding position,
in Formula (C-1),
C³ represents an aromatic ring which may have a substituent, and
X^{c1} and X^{c2} each independently represent a sulfur atom or an oxygen atom,
in Formula (C-2),
X^{c3} to X^{c5} each independently represent a sulfur atom or an oxygen atom, and
R^{c1} and R^{c2} each independently represent a hydrogen atom or a substituent.

9. The photoelectric conversion element according to claim 8,
wherein both X^{c1} and X^{c2} are oxygen atoms and both X^{c3} and X^{c4} are oxygen atoms.

10. The photoelectric conversion element according to any one of claims 1 to 9,
wherein Rs represents a linear alkyl group having 1 to 5 carbon atoms, which may have a substituent, a branched alkyl group having 3 to 7 carbon atoms, which may have a substituent, a cyclic alkyl group having 3 to 6 carbon atoms, which may have a substituent, an aryl group having 6 to 14 carbon atoms, which may have a substituent, a heteroaryl group having 2 to 14 carbon atoms, which may have a substituent, an alkoxy group having 1 to 5 carbon atoms, which may have a substituent, an aryloxy group having 6 to 14 carbon atoms, which may have a substituent, a silyl group which may have a substituent, or a halogen atom.

11. The photoelectric conversion element according to claim 4,
wherein D represents the group represented by Formula (4-10) or the group represented by Formula (4-11).

12. The photoelectric conversion element according to claim 11,
wherein Rs represents a linear alkyl group having 1 to 5 carbon atoms, which may have a substituent, a branched alkyl group having 3 to 7 carbon atoms, which may have a substituent, a cyclic alkyl group having 3 to 6 carbon atoms, which may have a substituent, or a group represented by Formula (S1), 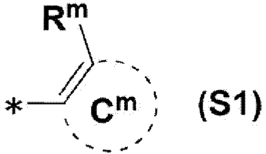
in Formula (S1),
C^{m} represents an aromatic hydrocarbon having 6 to 14 carbon atoms, which may have a substituent,
R^{m} represents a substituent, and
* represents a bonding position.

13. The photoelectric conversion element according to any one of claims 1 to 9, 11, and 12,
wherein the photoelectric conversion film further contains an n-type organic semiconductor, and
the photoelectric conversion film has a bulk hetero structure formed in a state in which the compound represented by Formula (1) and the n-type organic semiconductor are mixed with each other.

14. The photoelectric conversion element according to claim 13,
wherein the n-type organic semiconductor includes fullerenes selected from the group consisting of a fullerene and derivatives of the fullerene.

15. The photoelectric conversion element according to any one of claims 1 to 9, 11, and 12,
wherein the photoelectric conversion film further contains a p-type organic semiconductor.

16. The photoelectric conversion element according to any one of claims 1 to 9, 11, and 12, wherein the photoelectric conversion film further contains a coloring agent.

17. The photoelectric conversion element according to any one of claims 1 to 9, 11, and 12, further comprising:
one or more interlayers between the conductive film and the transparent conductive film, in addition to the photoelectric conversion film.

18. An imaging element comprising:
the photoelectric conversion element according to any one of claims 1 to 9, 11, and 12.

19. An optical sensor comprising:
the photoelectric conversion element according to any one of claims 1 to 9, 11, and 12.

20. A manufacturing method of an imaging element, comprising:
a step of manufacturing the photoelectric conversion element according to any one of claims 1 to 9, 11, and 12.

21. A compound represented by Formula (1), 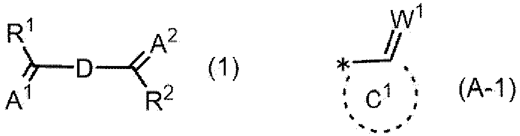 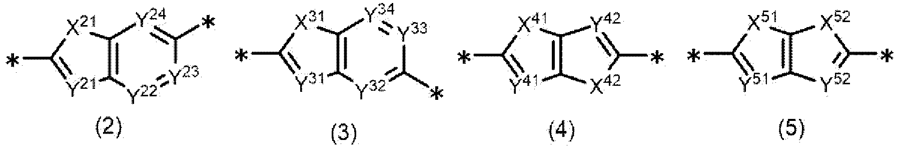 in Formula (1),
D represents a group represented by any of Formula (2) to Formula (5),
in Formula (2) to Formula (5), * represents a bonding position,
R¹ and R² each independently represent a hydrogen atom or a substituent,
A¹ and A² each independently represent a group represented by Formula (A-1), and in Formula (A-1), * represents a bonding position,
in Formula (2) to Formula (5),
X²¹, X³¹, X⁴¹, X⁴², X⁵¹, and X⁵² each independently represent a sulfur atom, an oxygen atom, NR^{X1}, or CR^{X2}R^{X3},
R^{X1} to R^{X3} each independently represent a hydrogen atom, an aliphatic hydrocarbon group which may have a substituent, or an aromatic ring group which may have a substituent,
R^{X2} and R^{X3} may be linked to each other through a single bond or a divalent linking group,
Y²¹, Y²², Y²³, Y²⁴, Y³¹, Y³², Y³³, Y³⁴, Y⁴¹, Y⁴², Y⁵¹, and Y⁵² each independently represent -CR^{Y1}= or a nitrogen atom, and R^{Y1} represents a hydrogen atom or a substituent,
provided that, in Formula (2), at least one of Y²¹, ..., or Y²⁴ represents -CRs=, in Formula (3), at least one of Y³¹, ..., or Y³⁴ represents -CRs=, in Formula (4), at least one of Y⁴¹ or Y⁴² represents -CRs=, and in Formula (5), at least one of Y⁵¹ or Y⁵² represents -CRs=,
where Rs represents an alkyl group which may have a substituent, an alkenyl group which may have a substituent, an alkynyl group which may have a substituent, an aryl group which may have a substituent, a heteroaryl group which may have a substituent, an alkoxy group which may have a substituent, an aryloxy group which may have a substituent, an amino group which may have a substituent, a silyl group which may have a substituent, a cyano group, or a halogen atom,
in Formula (A-1),
C¹ represents a ring which contains two or more carbon atoms and may have a substituent,
W¹ represents a sulfur atom, an oxygen atom, =NR^{W2}, or =CR^{W3}R^{W4},
R^{W2} represents a hydrogen atom or a substituent,
R^{W3} and R^{W4} each independently represent a cyano group, -SO₂R^{W5}, -COOR^{W6}, or -COR^{W7}, and
R^{W5} to R^{W7} each independently represent an aliphatic hydrocarbon group which may have a substituent, an aromatic ring group which may have a substituent, or an aliphatic heterocyclic group which may have a substituent.

22. The compound according to claim 21,
wherein R^{Y1} represents a hydrogen atom, an alkyl group which may have a substituent, an alkenyl group which may have a substituent, an alkynyl group which may have a substituent, an aryl group which may have a substituent, a heteroaryl group which may have a substituent, an alkoxy group which may have a substituent, an aryloxy group which may have a substituent, an amino group which may have a substituent, a silyl group which may have a substituent, a cyano group, or a halogen atom.

23. The compound according to claim 21,
wherein D represents a group represented by any of Formula (2-10), Formula (2-11), Formula (2-12), Formula (2-13), Formula (3-10), Formula (3-11), Formula (3-12), Formula (3-13), Formula (4-10), Formula (4-11), Formula (5-10), or Formula (5-11), 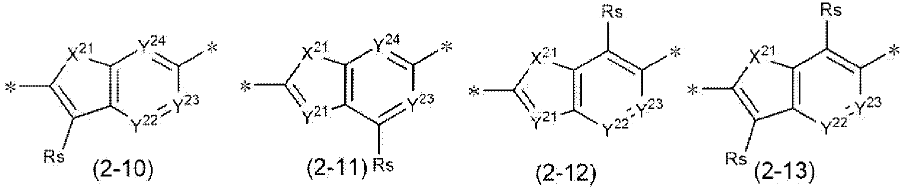 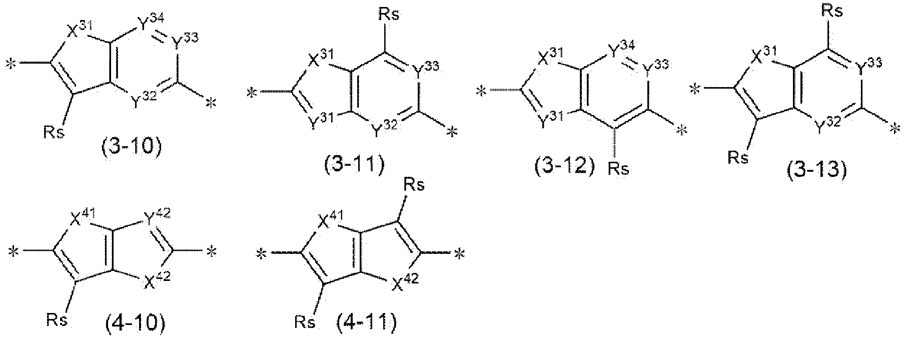 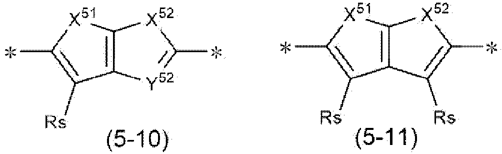
in Formula (2-10), Formula (2-11), Formula (2-12), Formula (2-13), Formula (3-10), Formula (3-11), Formula (3-12), Formula (3-13), Formula (4-10), Formula (4-11), Formula (5-10), and Formula (5-11), * represents a bonding position,
X²¹, X³¹, X⁴¹, X⁴², X⁵¹, and X⁵² each independently represent a sulfur atom, an oxygen atom, NR^{X1}, or CR^{X2}R^{X3},
R^{X1} to R^{X3} each independently represent a hydrogen atom, an aliphatic hydrocarbon group which may have a substituent, or an aromatic ring group which may have a substituent,
Y²¹, Y²², Y²³, Y²⁴, Y³¹, Y³², Y³³, Y³⁴, Y⁴², and Y⁵² each independently represent -CR^{Y1}= or a nitrogen atom, and R^{Y1} represents a hydrogen atom or a substituent,
where Rs represents an alkyl group which may have a substituent, an alkenyl group which may have a substituent, an alkynyl group which may have a substituent, an aryl group which may have a substituent, a heteroaryl group which may have a substituent, an alkoxy group which may have a substituent, an aryloxy group which may have a substituent, an amino group which may have a substituent, a silyl group which may have a substituent, a cyano group, or a halogen atom.

24. The compound according to claim 23,
wherein Rs represents a linear alkyl group having 1 to 5 carbon atoms, which may have a substituent, a branched alkyl group having 3 to 7 carbon atoms, which may have a substituent, a cyclic alkyl group having 3 to 6 carbon atoms, which may have a substituent, an alkenyl group having 2 to 5 carbon atoms, which may have a substituent, an alkynyl group having 2 to 5 carbon atoms, which may have a substituent, an aryl group having 6 to 14 carbon atoms, which may have a substituent, a heteroaryl group having 2 to 14 carbon atoms, which may have a substituent, an alkoxy group having 1 to 5 carbon atoms, which may have a substituent, an aryloxy group having 6 to 14 carbon atoms, which may have a substituent, an amino group which may have a substituent, a silyl group which may have a substituent, a cyano group, or a halogen atom.

25. The compound according to claim 24,
wherein D represents the group represented by any of Formula (2-10), Formula (2-11), Formula (2-12), Formula (2-13), Formula (3-10), Formula (3-11), Formula (3-12), Formula (3-13), Formula (5-10), or Formula (5-11).

26. The compound according to claim 21,
wherein W¹ is an oxygen atom or a sulfur atom.

27. The compound according to claim 26,
wherein the group represented by Formula (A-1) is a group represented by Formula (A-2), 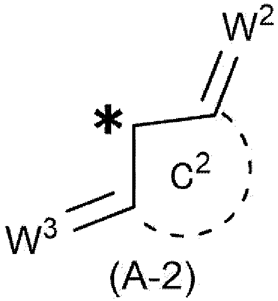
in Formula (A-2), * represents a bonding position,
C² represents a ring which contains three or more carbon atoms and may have a substituent, and
W² and W³ each independently represent an oxygen atom or a sulfur atom.

28. The compound according to claim 27,
wherein the group represented by Formula (A-2) is a group represented by Formula (C-1) or a group represented by Formula (C-2), 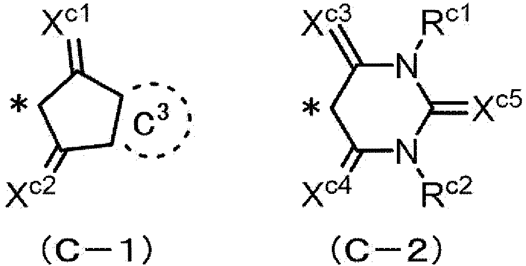
in Formula (C-1) and Formula (C-2),
* represents a bonding position,
in Formula (C-1),
C³ represents an aromatic ring which may have a substituent, and
X^{c1} and X^{c2} each independently represent a sulfur atom or an oxygen atom,
in Formula (C-2),
X^{c3} to X^{c5} each independently represent a sulfur atom or an oxygen atom, and
R^{c1} and R^{c2} each independently represent a hydrogen atom or a substituent.

29. The compound according to claim 28,
wherein both X^{c1} and X^{c2} are oxygen atoms and both X^{c3} and X^{c4} are oxygen atoms.

30. The compound any one of claims 21 to 29,
wherein Rs represents a linear alkyl group having 1 to 5 carbon atoms, which may have a substituent, a branched alkyl group having 3 to 7 carbon atoms, which may have a substituent, a cyclic alkyl group having 3 to 6 carbon atoms, which may have a substituent, an aryl group having 6 to 14 carbon atoms, which may have a substituent, a heteroaryl group having 2 to 14 carbon atoms, which may have a substituent, an alkoxy group having 1 to 5 carbon atoms, which may have a substituent, an aryloxy group having 6 to 14 carbon atoms, which may have a substituent, a silyl group which may have a substituent, or a halogen atom.

31. The compound according to claim 24,
wherein D represents the group represented by Formula (4-10) or the group represented by Formula (4-11).

32. The compound according to claim 31,
wherein Rs represents a linear alkyl group having 1 to 5 carbon atoms, which may have a substituent, a branched alkyl group having 3 to 7 carbon atoms, which may have a substituent, a cyclic alkyl group having 3 to 6 carbon atoms, which may have a substituent, or a group represented by Formula (S1), 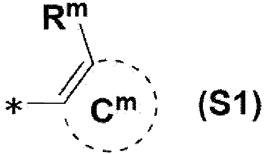
in Formula (S1),
C^{m} represents an aromatic hydrocarbon having 6 to 14 carbon atoms, which may have a substituent,
R^{m} represents a substituent, and
* represents a bonding position.
